# EUROPEAN PATENT APPLICATION

(11) **EP 1 640 360 A1**
(43) Date of publication of application: **29.03.2006**
(21) Application number: 04736459.1
(22) Date of filing: 09.06.2004
(51) Int. Cl.: C07C 255/44, A01N 37/34

(54) **AMIDES AND METHOD FOR PLANT DISEASE CONTROL WITH THE SAME**

(30) Priority: 27.06.2003 JP 2003184281; 24.12.2003 JP 2003426611
(71) Applicant: Sumitomo Chemical Company, Limited, Chuo-ku Osaka 541-8550 (JP)
(72) Inventor: SAKAGUCHI, Hiroshi, Toyonaka-shi, Osaka 561-0802 (JP)
(74) Representative: Henkel, Feiler & Hänzel
(86) International application number: PCT/JP2004/008397
(87) International publication number: WO 2005/000796

(57) **Abstract**

N-(α-cyanobenzyl)amide compounds represented by the formula (1): 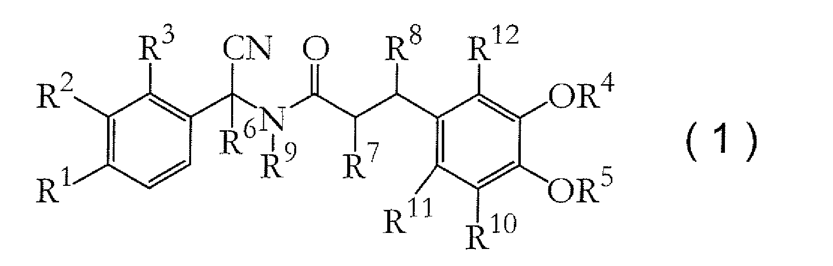 wherein R¹ represents a hydrogen atom; a halogen atom; a C1-C6 alkyl group optionally substituted with a halogen atom or the like; or the like, R² represents a hydrogen atom, a halogen atom, a C1-C6 alkyl group or the like, R³ represents a hydrogen atom or the like, R⁴ represents a C1-C4 alkyl group, a C3-C4 alkenyl group or the like, R⁵ represents a C1-C4 alkyl group, a C3-C4 alkenyl group, or the like, R⁶ represents a hydrogen atom or the like, R⁷ represents a hydrogen atom or the like, R⁸ represents a hydrogen atom or the like, R⁹ represents a hydrogen atom or the like, R¹⁰ represents a hydrogen atom or the like, R¹¹ represents a hydrogen atom or the like, and R¹² represents a hydrogen atom or the like, have excellent control activities against plant diseases.

## Description

### Technical Field

The present invention relates to amide compounds, and a method for controlling plant diseases comprising applying them to plants or soil where plants are grown.

### Background Art

Plant diseases-controlling compositions have been developed and many compounds having a controlling activity against plant diseases have been found out. However, their controlling activities are not enough in some cases. Therefore intensive researches into new compounds are carried out.

### Disclosure of Invention

An object of the present invention is to provide compounds having excellent controlling activity against plant diseases.

Namely, the present invention provides an N-(α-cyanobenzyl)amide compound represented by the formula (1): 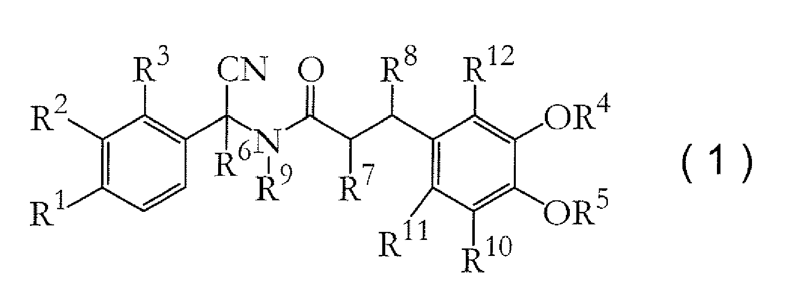 wherein
R¹ represents a hydrogen atom; a halogen atom; a nitro group; a cyano group; a C1-C6 alkyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C2-C6 alkenyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C2-C6 alkynyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C3-C6 cycloalkyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C1-C6 alkoxy group optionally substituted with a substituent selected from the group consisting of a halogen atom, a C1-C6 alkoxy group and a C1-C6 alkylthio group; a C3-C6 cycloalkoxy group optionally substituted with a subsitutent selected from the group consisting of a halogen atom, a C1-C6 alkoxy group and a C1-C6 alkylthio group; a C1-C6 alkylthio group optionally substituted with a substituent selected from the group consisting of a halogen atom, a C1-C6 alkoxy group and a C1-C6 alkylthio group; a C3-C6 cycloalkylthio group optionally substituted with a substituent selected from the group consisting of a halogen atom, a C1-C6 alkoxy group and a C1-C6 alkylthio group; a di(C1-C6 alkyl)amino group optionally substituted with a substituent selected from the group consisting of a halogen atom, a C1-C6 alkoxy group and a C1-C6 alkylthio group; a phenyl group optionally substituted with a substituent selected from the group consisting of a halogen atom, a C1-C6 alkyl group, a C1-C6 haloalkyl group and a C1-C6 alkoxy group; a phenoxy group optionally substituted with a substituent selected from the group consisting of a halogen atom, a C1-C6 alkyl group, a C1-C6 haloalkyl group and a C1-C6 alkoxy group; a phenylthio group optionally substituted with a substituent selected from the group consisting of a halogen atom, a C1-C6 alkyl group, a C1-C6 haloalkyl group and a C1-C6 alkoxy group; a benzoyl group optionally substituted with a substituent selected from the group consisting of a halogen atom, a C1-C6 alkyl group, a C1-C6 haloalkyl group and a C1-C6 alkoxy group; a benzoyloxy group optionally substituted with a substituent selected from the group consisting of a halogen atom, a C1-C6 alkyl group, a C1-C6 haloalkyl group and a C1-C6 alkoxy group; a formyl group; a (C1-C6 alkyl)carbonyl group; a (C2-C6 alkenyl)carbonyl group; a (C2-C6 alkynyl)carbonyl group; a (C3-C6 cycloalkyl)carbonyl group; a (C1-C6 haloalkyl)carbonyl group; a (C1-C6 alkoxy)carbonyl group; or a tri(C1-C6 alkyl)silyl group,
R² represents a hydrogen atom, a halogen atom, a C1-C6 alkyl group, a C3-C6 cycloalkyl group, a C2-C6 alkenyl group, a C2-C6 alkynyl group, a C1-C6 haloalkyl group, a C1-C6 alkoxy group, a C1-C6 haloalkoxy group, a C1-C6 alkylthio group, a C1-C6 haloalkylthio group, a di(C1-C6 alkyl)amino group, a nitro group or a cyano group, or
R¹ and R² are taken together to represent a C3-C6 polymethylene group optionally substituted with a substituent selected from the group consisting of a halogen atom, a C1-C3 alkyl group and a C1-C3 haloalkyl group; or a 1,3-butadiene-1,4-diyl group optionally substituted with a substituent selected from the group consisting of a halogen atom, a C1-C3 alkyl group and a C1-C3 haloalkyl group,
R³ represents a hydrogen atom, a halogen atom or a C1-C3 alkyl group,
R⁴ represents a C1-C4 alkyl group, a C1-C4 haloalkyl group, a C3-C4 alkenyl group or a C3-C4 alkynyl group,
R⁵ represents a C1-C4 alkyl group, a C3-C4 alkenyl group, a C3-C6 alkynyl group, a C1-C4 haloalkyl group, a cyano C1-C6 alkyl group or a (C1-C3 alkyl)carbonyl group,
R⁶ represents a hydrogen atom, a C1-C4 alkyl group, a C3-C4 alkenyl group or a C3-C4 alkynyl group,
R⁷ represents a hydrogen atom, a halogen atom or a C1-C3 alkyl group,
R⁸ represents a hydrogen atom, a halogen atom or a C1-C3 alkyl group,
R⁹ represents a hydrogen atom or a C1-C3 alkyl group,
R¹⁰ represents a hydrogen atom, a halogen atom or a C1-C3 alkyl group,
R¹¹ represents a hydrogen atom, a halogen atom or a C1-C3 alkyl group,
R¹² represents a hydrogen atom, a halogen atom or a C1-C3 alkyl group (referred as "the compound of the present invention" hereinafter), a plant disease-controlling composition comprising the compound of the present invention as an active ingredient, and a method for controlling a plant disease comprising treating a plant or soil where a plant is cultivated with an effective amount of the compound of the present invention.

Respective substituents represented by R¹, R², R³, R⁴, R⁵, R⁶, R⁷, R⁸, R⁹, R¹⁰, R¹¹ and R¹² in the compound of the present invention will be exemplified below.

Examples of a halogen atom represented by R¹ include a fluorine atom, a chlorine atom, a bromine atom, and an iodine atom.

Examples of a C1-C6 alkyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group represented by R¹ include a C1-C6 alkyl group such as a methyl group, an ethyl group, a propyl group, an isopropyl group, a butyl group, an isobutyl group, a sec-butyl group, a tert-butyl group, a pentyl group, a 2-methylpentyl group, a 3-methylpentyl group, a 4-methylpentyl group, a hexyl group and the like; a C1-C6 haloalkyl group such as a fluoromethyl group, a difluoromethyl group, a trifluoromethyl group, a chloromethyl group, a dichloromethyl group, a trichloromethyl group and the like; a cyano C1-C6 alkyl group such as a cyanomethyl group, a 2-cyanoethyl group, a 1-cyanoethyl group and the like; a C1-C6 hydroxyalkyl group such as a hydroxymethyl group, a 1-hydroxyethyl group, a 2-hydroxyethyl group, a 3-hydroxypropyl group and the like; a C3-C6 cycloalkyl C1-C6 alkyl group such as a cyclopropylmethyl group, a cyclobutylmethyl group, a cyclopentylmethyl group, a cyclohexylmethyl group and the like; a C1-C6 alkoxy C1-C6 alkyl group such as a 2-methoxyethoxy group, a 2-ethoxyethoxy group and the like; a (C1-C3 alkoxy)cyano C1-C6 alkyl group such as an ethoxycyanomethyl group, a methoxycyanomethyl group and the like; a C1-C6 cycloalkoxy C1-C6 alkyl group such as a cyclopropyloxymethyl group, a cyclobutyloxymethyl group, a cyclopentyloxymethyl group, a cyclohexyloxymethyl group and the like; a C1-C6 alkylthio C1-C6 alkyl group such as a 2-methylthioethyl group and the like; a C1-C6 cycloalkylthio C1-C6 alkyl group such as a cyclopentylthiomethyl group and the like; and a tri(C1-C3 alkyl)silyl C1-C6 alkyl group.

Examples of a C2-C6 alkenyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group represented by R¹ include a C2-C6 alkenyl group such as a vinyl group, a 1-methylvinyl group, a 1-propenyl group, a 2-propenyl group, a 1-methyl-2-propenyl group, a 2-methyl-2-propenyl group, a 2-butenyl group, a 3-butenyl group, a 1-pentenyl group, a 4-pentenyl group, a 1-hexenyl group, a 5-hexenyl group and the like; a C2-C6 haloalkenyl group such as a 2-fluorovinyl group, a 2,2-difluorovinyl group, a 2-chlorovinyl group, a 2,2-dichlorovinyl group, a 2-bromovinyl group, a 2,2-dibromovinyl group, a 2-chloro-2-propenyl group, a 3,3-dichloro-2-propenyl group and the like; a cyano C2-C6 alkenyl group; a C2-C6 hydroxyalkenyl group; a C3-C6 cycloalkyl C2-C6 alkenyl group; a C1-C6 alkoxy C2-C6 alkenyl group; a C1-C6 cycloalkoxy C2-C6 alkenyl group; a C1-C6 alkylthio C2-C6 alkenyl group; a C1-C6 cycloalkylthio C2-C6 alkenyl group; and a tri(C1-C3 alkyl)silyl C2-C6 alkenyl group.

Examples of a C2-C6 alkynyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group represented by R¹ include a C2-C6 alkynyl group such as an ethynyl group, a 1-propynyl group, a 2-propynyl group, a 1-methyl-2-propynyl group, a 1-butynyl group, a 2-butynyl group, a 3-butynyl group, a 3,3-dimethyl-1-butynyl group, a 1-pentynyl group, a 4-pentynyl group, a 1-hexynyl group, a 5-hexynyl group and the like; a C2-C6 haloalkynyl group such as a 2-fluoroethynyl group, a 2-chloroethynyl group, a 2-bromoethynyl group, a 2-iodoethynyl group and the like; a cyano C2-C6 alkynyl group; a C2-C6 hydroxyalkynyl group; a C3-C6 cycloalkyl C2-C6 alkynyl group; a C1-C6 alkoxy C2-C6 alkynyl group; a C1-C6 cycloalkoxy C2-C6 alkynyl group; a C1-C6 alkylthio C2-C6 alkynyl group; a C1-C6 cycloalkylthio C2-C6 alkynyl group; and a tri(C1-C3 alkyl)silyl C2-C6 alkynyl group such as a trimethylsilylethynyl group, a triethylsilylethynyl group and the like.

Examples of a C3-C6 cycloalkyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group represented by R¹ include a C3-C6 cycloalkyl group such as a cyclopropyl group, a cyclobutyl group, a cyclopentyl group, a cyclohexyl group and the like; a C3-C6 halocycloalkyl group; a cyano C3-C6 cycloalkyl group; a C3-C6 hydroxycycloalkyl group; a C3-C6 cycloalkyl C3-C6 cycloalkyl group; a C1-C6 alkoxy C3-C6 cycloalkyl group; a C1-C6 cycloalkoxy C3-C6 cycloalkyl group; a C1-C6 alkylthio C3-C6 cycloalkyl group; a C1-C6 cycloalkylthio C3-C6 cycloalkyl group; and a tri(C1-C3 alkyl)silyl C3-C6 cycloalkyl group.

Examples of a C1-C6 alkoxy group optionally substituted with a substituent selected from a group consisting of a halogen atom, a C1-C6 alkoxy group and a C1-C6 alkylthio group represented by R¹ include a C1-C6 alkoxy group such as a methoxy group, an ethoxy group, a propoxy group, an isopropoxy group, a butoxy group, an isobutoxy group, a sec-butoxy group, a pentyloxy group, a hexyloxy group and the like; a C1-C6 haloalkoxy group such as a fluoromethoxy group, a difluoromethoxy group, a trifluoromethoxy group, a 2,2,2-trifluoroethoxy group, a 1,1,2,2-tetrafluoroethoxy group, a 2-fluoroethoxy group and the like; a C1-C6 alkoxy C1-C6 alkoxy group such as a 2-methoxyethoxy group and the like; and a C1-C6 alkylthio C1-C6 alkoxy such as a 2-methylthioethoxy group and the like.

Examples of a C3-C6 cycloalkoxy group optionally substituted with a substituent selected from the group consisting of a halogen atom, a C1-C6 alkoxy group and a C1-C6 alkylthio group represented by R¹ include a C3-C6 cycloalkoxy group such as a cyclopropyloxy group, a cyclobutyloxy group, a cyclopentyloxy group, a cyclohexyl group, a 2,2-dimethylpropyl group and the like; a C3-C6 halocycloalkoxy group; a C1-C6 alkoxy C3-C6 cycloalkoxy group; and a C1-C6 alkylthio C3-C6 cycloalkoxy group.

Examples of a C1-C6 alkylthio group optionally substituted with a substituent selected from the group consisting of a halogen atom, a C1-C6 alkoxy group and a C1-C6 alkylthio group represented by R¹ include a C1-C6 alkylthio group such as a methylthio group, an ethylthio group, a propylthio group, an isopropylthio group, a butylthio group, an isobutylthio group, a sec-butylthio group, a tert-butylthio group, a pentylthio group, a hexylthio group and the like; a C1-C6 haloalkylthio group such as a fluoromethylthio group, a difluoromethylthio group, a trifluoromethylthio group, a 2,2,2-trifluoroethylthio group, a 1,1,2,2-tetrafluoroethylthio group, a 2-fluoroethylthio group and the like; a C1-C6 alkoxy C1-C6 alkylthio group such as a 2-methoxyethylthio group and the like; and a C1-C6 alkylthio C1-C6 alkylthio group.

Examples of a C3-C6 cycloalkylthio group optionally substituted with a substituent selected from the group consisting of a halogen atom, a C1-C6 alkoxy group and a C1-C6 alkylthio group represented by R¹ include a C3-C6 cycloalkylthio group such as a cyclopropylthio group, a cyclobutylthio group, a cyclopentylthio group and the like; a C3-C6 halocycloalkylthio group; a C1-C6 alkoxy C3-C6 cycloalkylthio group; and a C1-C6 alkylthio C3-C6 cycloalkylthio group.

Examples of a di(C3-C6 alkyl)amino group optionally substituted with a substituent selected from the group consisting of a halogen atom, a C1-C6 alkoxy group and a C1-C6 alkylthio group represented by R¹ include a di(C3-C6 alkyl)amino group such as a dimethylamino group, a diethylamino group, a dipropylamino group, a dipentylamino group, a dihexylamino group and the like; a (C3-C6 alkyl) (C1-C6 alkoxy C3-C6 alkyl)amino group such as a methyl(2-methoxyethyl)amino group and the like; and a (C3-C6 alkyl)(C1-C6 alkylthio C3-C6 alkyl)amino group.

Examples of a phenyl group optionally substituted with a substituent selected from the group consisting of a halogen atom, a C1-C6 alkyl group, a C1-C6 haloalkyl group and a C1-C6 alkoxy group represented by R¹ include a phenyl group, a 2-fluorophenyl group, a 3-fluorophenyl group, a 4-fluorophenyl group, a 3,4-difluorophenyl group, a 2-chlorophenyl group, a 3-chlorophenyl group, a 4-chlorophenyl group, a 3,4-dichlorophenyl group, a 2-bromophenyl group, a 3-bromophenyl group, a 4-bromophenyl group, a 2-methylphenyl group, a 3-methylphenyl group, a 4-methylphenyl group, a 2-ethylphenyl group, a 3-ethylphenyl group, a 4-ethylphenyl group, a 2-trifluoromethylphenyl group, a 3-trifluoromethylphenyl group, a 4-trifluoromethylphenyl group, a 2-methoxyphenyl group, a 3-methoxyphenyl group, and a 4-methoxyphenyl group.

Examples of a phenoxy group optionally substituted with a substituent selected from the group consisting of a halogen atom, a C1-C6 alkyl group, a C1-C6 haloalkyl group and a C1-C6 alkoxy group represented by R¹ include a phenoxy group, a 2-fluorophenoxy group, a 3-fluorophenoxy group, a 4-fluorophenoxy group, a 3,4-difluorophenoxy group, a 2-chlorophenoxy group, a 3-chlorophenoxy group, a 4-chlorophenoxy group, a 3,4-dichlorophenoxy group, a 2-bromophenoxy group, a 3-bromophenoxy group, a 4-bromophenoxy group, a 2-methylphenoxy group, a 3-methylphenoxy group, a 4-methylphenoxy group, a 2-ethylphenoxy group, a 3-ethylphenoxy group, a 4-ethylphenoxy group, a 2-trifluoromethylphenoxy group, a 3-trifluoromethylphenoxy group, a 4-trifluoromethylphenoxy group, a 2-methoxyphenoxy group, a 3-methoxyphenoxy group, and a 4-methoxyphenoxy group.

Examples of a phenylthio group optionally substituted with a substituent selected from the group consisting of a halogen atom, a C1-C6 alkyl group, a C1-C6 haloalkyl group and a C1-C6 alkoxy group represented by R¹ include a phenylthio group, a 2-fluorophenylthio group, a 3-fluorophenylthio group, a 4-fluorophenylthio group, a 3,4-difluorophenylthio group, a 2-chlorophenylthio group, a 3-chlorophenylthio group, a 4-chlorophenylthio group, a 3,4-dichlorophenylthio group, a 2-bromophenylthio group, a 3-bromophenylthio group, a 4-bromophenylthio group, a 2-methylphenylthio group, a 3-methylphenylthio group, a 4-methylphenylthio group, a 2-ethylphenylthio group, a 3-ethylphenylthio group, a 4-ethylphenylthio group, a 2-trifluoromethylphenylthio group, a 3-trifluoromethylphenylthio group, a 4-trifluoromethylphenylthio group, a 2-methoxyphenylthio group, a 3-methoxyphenylthio group, and a 4-methoxyphenylthio group.

Examples of a benzoyl group optionally substituted with a substituent selected from the group consisting of a halogen atom, a C1-C6 alkyl group, a C1-C6 haloalkyl group and a C1-C6 alkoxy group represented by R¹ include a benzoyl group, a 2-fluorobenzoyl group, a 3-fluorobenzoyl group, a 4-fluorobenzoyl group, a 3,4-difluorobenzoyl group, a 2-chlorobenzoyl group, a 3-chlorobenzoyl group, a 4-chlorobenzoyl group, 3,4-dichlorobenzoyl group, a 2-bromobenzoyl group, a 3-bromobenzoyl group, a 4-bromobenzoyl group, a 2-methylbenzoyl group, a 3-methylbenzoyl group, a 4-methylbenzoyl group, a 2-ethylbenzoyl group, a 3-ethylbenzoyl group, a 4-ethylbenzoyl group, a 2-trifluoromethylbenzoyl group, a 3-trifluoromethylbenzoyl group, a 4-trifluoromethylbenzoyl group, a 2-methoxybenzoyl group, a 3-methoxybenzoyl group, and a 4-methoxybenzoyl group.

Examples of a benzoyloxy group optionally substituted with a substituent selected from the group consisting of a halogen atom, a C1-C6 alkyl group, a C1-C6 haloalkyl group and a C1-C6 alkoxy group represented by R¹ include a benzoyloxy group, a 2-fluorobenzoyloxy group, a 3-fluorobenzoyloxy group, a 4-fluorobenzoyloxy group, a 3,4-difluorobenzoyloxy group, a 2-chlorobenzoyloxy group, a 3-chlorobenzoyloxy group, a 4-chlorobenzoyloxy group, a 3,4-dichlorobenzoyloxy group, a 2-bromobenzoyloxy group, a 3-bromobenzoyloxy group, a 4-bromobenzoyloxy group, a 2-methylbenzoyloxy group, a 3-methylbenzoyloxy group, a 4-methylbenzoyloxy group, a 2-ethylbenzoyloxy group, a 3-ethylbenzoyloxy group, a 4-ethylbenzoyloxy group, a 2-trifluoromethylbenzoyloxy group, a 3-trifluoromethylbenzoyloxy group, a 4-trifluoromethylbenzoyloxy group, a 2-methoxybenzoyloxy group, a 3-methoxybenzoyloxy group, and a 4-methoxybenzoyloxy group.

Examples of a (C1-C6 alkyl)carbonyl group represented by R¹ include an acetyl group, a propionyl group, and a butyryl group.

Examples of a (C2-C6 alkenyl)carbonyl group represented by R¹ include an acryloyl group, a methacryloyl group, a crotonoyl group, and an isocrotonoyl group.

Examples of a (C2-C6 alkynyl)carbonyl group represented by R¹ include a propioloyl group.

Examples of a (C3-C6 cycloalkyl)carbonyl group represented by R¹ include a cyclopropylcarbonyl group, a cyclobutylcarbonyl group, a cyclopentylcarbonyl group, and a cyclohexylcarbonyl group.

Examples of a (C1-C6 haloalkyl)carbonyl group represented by R¹ include a chloroacetyl group, and a trifluoroacetyl group.

Examples of a (C1-C6 alkoxy)carbonyl group represented by R¹ include an acetyloxy group, a propionyloxy group, and a butyryloxy group.

Examples of a tri(C1-C6 alkyl)silyl group represented by R¹ include a trimethylsilyl group, a triethylsilyl group, a triisopropylsilyl group, and a tert-butyldimethylsilyl group.

Examples of a halogen atom represented by R² include a fluorine atom, a chlorine atom, a bromine atom, and an iodine atom.

Examples of a C1-C6 alkyl group represented by R² include a methyl group, an ethyl group, a propyl group, an isopropyl group, a butyl group, an isobutyl group, a sec-butyl group, and a tert-butyl group.

Examples of a C3-C6 cycloalkyl group represented by R² include a cyclopropyl group, a cyclobutyl group, a cyclopentyl group, and a cyclohexyl group.

Examples of a C2-C6 alkenyl group represented by R² include a vinyl group, a 1-methylvinyl group, a 1-propenyl group, a 2-propenyl group, a 1-methyl-2-propenyl group, a 2-methyl-2-propenyl group, a 2-butenyl group, a 3-butenyl group, a 1-pentenyl group, a 4-pentenyl group, a 1-hexenyl group, and a 5-hexenyl group.

Examples of a C2-C6 alkynyl group represented by R² include an ethynyl group, a 1-propynyl group, a 2-propynyl group, a 1-methyl-2-propynyl group, a 1-butynyl group, a 2-butynyl group, a 3-butynyl group, a 3,3-dimethyl-1-butynyl group, a 1-pentynyl group, a 4-pentynyl group, a 1-hexynyl group, and a 5-hexynyl group.

Examples of a C1-C6 haloalkyl group represented by R² include a fluoromethyl group, a difluoromethyl group and a trifluoromethyl group.

Examples of a C1-C6 alkoxy group represented by R² include a methoxy group, an ethoxy group, a propoxy group, an isopropoxy group, a butoxy group, an isobutoxy group, a sec-butoxy group, a pentyloxy group and a hexyloxy group.

Examples of a C1-C6 haloalkoxy group represented by R² include a fluoromethoxy group, a difluoromethoxy group, a trifluoromethoxy group, a 2,2,2-trifluoroethoxy group, a 1,1,2,2-tetrafluoroethoxy group, and a 2-fluoroethoxy group.

Examples of a C1-C6 alkylthio group represented by R² include a methylthio group, an ethylthio group, a propylthio group, an isopropylthio group, a butylthio group, an isobutylthio group, a sec-butylthio group, a tert-butylthio group, a pentylthio group, and a hexylthio group.

Examples of a C1-C6 haloalkylthio group represented by R² include a fluoromethylthio group, a difluoromethylthio group, a trifluoromethylthio group, a 2,2,2-trifluoroethylthio group, a 1,1,2,2-tetrafluoroethylthio group, and a 2-fluoroethylthio group.

Examples of a di(C1-C6 alkyl)amino group represented by R² include a dimethylamino group, a diethylamino group, a dipropylamino group, a dipentylamino group, and a dihexylamino group.

Examples of a C3-C6 polymethylene group optionally substituted with a substituent selected from the group consisting of a halogen atom, a C1-C3 alkyl group and a C1-C3 haloalkyl group represented by R¹ and R² together include a 1,3-propanediyl group, a 1,3-butanediyl group, a 1,4-butanediyl group, a 1,5-pentanediyl group, a 1,4-pentanediyl group and a 1,6-hexanediyl group.

Examples of a 1,3-butadiene-1,4-diyl group optionally substituted with a substituent selected from the group consisting of a halogen atom, a C1-C3 alkyl group and a C1-C3 haloalkyl group represented by R¹ and R² together include a 1,3-butadiene-1,4-diyl group, a 1-methyl-1,3-butadiene-1,4-diyl group, a 2-methyl-1,3-butadiene-1,4-diyl group, a 3-methyl-1,3-butadiene-1,4-diyl group, a 4-methyl-1,3-butadiene-1,4-diyl group, a 2,3-dimethyl-1,3-butadiene-1,4-diyl group, a 1-ethyl-1,3-butadiene-1,4-diyl group, a 2-ethyl-1,3-butadiene-1,4-diyl group, a 3-ethyl-1,3-butadiene-1,4-diyl group, a 4-ethyl-1,3-butadiene-1,4-diyl group, a 1-fluoro-butadiene-1,4-diyl group, a 2-fluoro-1,3-butadiene-1,4-diyl group, a 3-fluoro-1,3-butadiene-1,4-diyl group, a 4-fluoro-1,3-butadiene-1,4-diyl group, a 2,3-difluoro-1,3-butadiene-1,4-diyl group, a 1-chlorobutadiene-1,4-diyl group, a 2-chloro-1,3-butadiene-1,4-diyl group, a 3-chloro-1,3-butadiene-1,4-diyl group, a 4-chloro-1,3-butadiene-1,4-diyl group, a 2,3-dichloro-1,3-butadiene-1,4-diyl group, a 1-bromo-butadiene-1,4-diyl group, a 2-bromo-1,3-butadiene-1,4-diyl group, a 3-bromo-1,3-butadiene-1,4-diyl group, a 4-bromo-1,3-butadiene-1,4-diyl group, and a 2,3-dibromo-1,3-butadiene-1,4-diyl group. In the 1,3-butadiene-1,4-diyl group substituted with a substituent selected from the group consisting of a halogen atom, a C1-C3 alkyl group and a C1-C3 haloalkyl group, the carbon atom at the 1-position binds to the position where R² is attached.

Examples of a halogen atom represented by R³ include a fluorine atom, a chlorine atom, a bromine atom, and an iodine atom.

Examples of a C1-C3 alkyl group represented by R³ include a methyl group, an ethyl group, a propyl group, and an isopropyl group.

Examples of a C1-C4 alkyl group represented by R⁴ include a methyl group, an ethyl group, a propyl group, an isopropyl group, a butyl group, an isobutyl group, a sec-butyl group, and a tert-butyl group.

Examples of a C1-C4 haloalkyl group represented by R⁴ include a fluoromethyl group, a difluoromethyl group, a trifluoromethyl group, a 2-fluoroethyl group, and a 1,1,2,2-tetrafluoroethyl group.

Examples of a C3-C4 alkenyl group represented by R⁴ include a 2-propenyl group, a 1-methyl-2-propenyl group, a 2-methyl-2-propenyl group, a 2-butenyl group, and a 3-butenyl group.

Examples of a C3-C4 alkynyl group represented by R⁴ include a 2-propynyl group, a 1-methyl-2-propynyl group, a 2-butynyl, and a 3-butynyl group.

Examples of a C1-C4 alkyl group represented by R⁵ include a methyl group, an ethyl group, a propyl group, an isopropyl group, a butyl group, an isobutyl group, a sec-butyl group, and a tert-butyl group.

Examples of a C3-C4 alkenyl group represented by R⁵ include a 2-propenyl group, a 1-methyl-2-propenyl group, a 2-methyl-2-propenyl group, a 2-butenyl group, and a 3-butenyl group.

Examples of a C3-C6 alkynyl group represented by R⁵ include a 2-propynyl group, a 1-methyl-2-propynyl group, a 1,1-dimethyl-2-propynyl group, a 2-butynyl group, a 3-butynyl group, a 1-methyl-2-butynyl group, a 2-pentynyl group, a 1-methyl-2-pentynyl group, a 4-methyl-2-pentynyl group, a 3-pentynyl group, a 4-pentynyl group, a 2-hexynyl group, and a 3-hexynyl group.

Examples of a C1-C4 haloalkyl group represented by R⁵ include a fluoromethyl group, a difluoromethyl group, a trifluoromethyl group, a 2-fluoroethyl group, and a 2,2,2-trifluoroethyl group.

Examples of a cyano C1-C6 alkyl group represented by R⁵ include a cyanomethyl group, a 1-cyanoethyl group, a 2-cyanoethyl group, and a 1-cyanopropyl group.

Examples of a (C1-C3 alkyl)carbonyl group represented by R⁵ include an acetyl group, a propionyl group, a butyryl group, and an isobutyryl group.

Examples of a C1-C4 alkyl group represented by R⁶ include a methyl group, an ethyl group, a propyl group, and a butyl group.

Examples of a C3-C4 alkenyl group represented by R⁶ include a 2-propenyl group, a 1-methyl-2-propenyl group, a 2-methyl-2-propenyl group, a 2-butenyl group, and a 3-butenyl group.

Examples of a C3-C4 alkynyl group represented by R⁶ include a 2-propynyl group, a 1-methyl-2-propynyl group, a 2-butynyl group, and a 3-butynyl group.

Examples of a halogen atom represented by R⁷ include a fluorine atom, a chlorine atom, a bromine atom, and an iodine atom.

Examples of a C1-C3 alkyl group represented by R⁷ include a methyl group, an ethyl group, a propyl group, and an isopropyl group.

Examples of a halogen atom represented by R⁸ include a fluorine atom, a chlorine atom, a bromine atom, and an iodine atom.

Examples of a C1-C3 alkyl group represented by R⁸ include a methyl group, an ethyl group, a propyl group, and an isopropyl group.

Examples of a C1-C3 alkyl group represented by R⁹ include a methyl group, an ethyl group, a propyl group, and an isopropyl group.

Examples of a halogen atom represented by R¹⁰ include a fluorine atom, a chlorine atom, a bromine atom, and an iodine atom.

Examples of a C1-C3 alkyl group represented by R¹⁰ include a methyl group, an ethyl group, a propyl group, and an isopropyl group.

Examples of a halogen atom represented by R¹¹ include a fluorine atom, a chlorine atom, a bromine atom, and an iodine atom.

Examples of a C1-C3 alkyl group represented by R¹¹ include a methyl group, an ethyl group, a propyl group, and an isopropyl group.

Examples of a halogen atom represented by R¹² include a fluorine atom, a chlorine atom, a bromine atom, and an iodine atom.

Examples of a C1-C3 alkyl group represented by R¹² include a methyl group, an ethyl group, a propyl group, and an isopropyl group.

The compound of the present invention has optical isomers of (R) form and (S) form based on the carbon atom to which the nitrogen atom of amide binds, and respective optical isomers and a mixture thereof are included in the present invention.

The compound of the present invention includes, for example, the following compounds:
an N-(α-cyanobenzyl)amide compound represented by the formula (1) wherein R³ is a hydrogen atom,
an N-(α-cyanobenzyl)amide compound represented by the formula (1) wherein R⁴ is a C1-C4 alkyl group, a C3-C4 alkenyl group or a C3-C4 alkynyl group,
an N-(α-cyanobenzyl)amide compound represented by the formula (1) wherein R⁵ is a C1-C4 alkyl group, a C3-C4 alkenyl group or a C3-C4 alkynyl group,
an N-(α-cyanobenzyl)amide compound represented by the formula (1) wherein R⁶ is a hydrogen atom,
an N-(α-cyanobenzyl)amide compound represented by the formula (1) wherein R⁷ is a hydrogen atom,
an N-(α-cyanobenzyl)amide compound represented by the formula (1) wherein R⁸ is a hydrogen atom,
an N-(α-cyanobenzyl)amide compound represented by the formula (1) wherein R⁹ is a hydrogen atom,
an N-(α-cyanobenzyl)amide compound represented by the formula (1) wherein R⁶ and R⁹ are hydrogen atoms,
an N-(α-cyanobenzyl)amide compound represented by the formula (1) wherein R⁷ and R⁸ are hydrogen atoms,
an N-(α-cyanobenzyl)amide compound represented by the formula (1) wherein R¹⁰, R¹¹ and R¹² are hydrogen atoms,
an N-(a-cyanobenzyl)amide compound represented by the formula (1) wherein R³, R⁷ and R⁸ are hydrogen atoms,
an N-(α-cyanobenzyl)amide compound represented by the formula (1) wherein R³, R⁷, R⁸, R¹⁰ , R¹¹ and R¹² are hydrogen atoms,
an N-(α-cyanobenzyl)amide compound represented by the formula (1) wherein R³, R⁷, R⁸ and R⁹ are hydrogen atoms,
an N-(α-cyanobenzyl)amide compound represented by the formula (1) wherein R³, R⁷, R⁸, R⁹, R¹⁰, R¹¹ and R¹² are hydrogen atoms,
an N-(α-cyanobenzyl)amide compound represented by the formula (1) wherein R⁴ and R⁵ are each a C1-C4 alkyl group, a C3-C4 alkenyl group or a C3-C4 alkynyl group,
an N-(α-cyanobenzyl)amide compound represented by the formula (1) wherein R⁶ is a hydrogen atom or a C3-C4 alkynyl group and R³, R⁷ and R⁸ are hydrogen atoms,
an N-(α-cyanobenzyl)amide compound represented by the formula (1) wherein R⁶ is a hydrogen atom or a C3-C4 alkynyl group and R³, R⁷, R⁸, R¹⁰, R¹¹ and R¹² are hydrogen atoms,
an N-(α-cyanobenzyl)amide compound represented by the formula (1) wherein R⁴ is a C1-C4 alkyl group, a C3-C4 alkenyl group or a C3-C4 alkynyl group, R⁵ is a C1-C4 alkyl group, a C3-C4 alkenyl group or a C3-C4 alkynyl group, R⁶ is a hydrogen atom or a C3-C4 alkynyl group, and R³, R⁷ and R⁸ are hydrogen atoms,
an N-(α-cyanobenzyl)amide compound represented by the formula (1) wherein R⁴ is a C1-C4 alkyl group, a C3-C4 alkenyl group or a C3-C4 alkynyl group, R⁵ is a C1-C4 alkyl group, a C3-C4 alkenyl group or a C3-C4 alkynyl group, R⁶ is a hydrogen atom or a C3-C4 alkynyl group, and R³, R⁷, R⁸, R¹⁰, R¹¹ and R¹² are hydrogen atoms,
an N-(α-cyanobenzyl)amide compound represented by the formula (1) wherein R¹ is a hydrogen atom, a halogen atom, a C1-C6 alkyl group, a C1-C6 alkoxy group or a C1-C6 haloalkoxy group, R² is a hydrogen atom, a halogen atom, a C1-C6 alkyl group, a C1-C6 alkoxy group or a C1-C6 haloalkoxy group, or R¹ and R² are taken together to form a C3-C5 alkylene group or CH=CH-CH=CH, R⁴ is a C1-C4 alkyl group, a C3-C4 alkenyl group or a C3-C4 alkynyl group, R⁵ is a C1-C4 alkyl group, a C3-C4 alkenyl group or a C3-C4 alkynyl group, R⁶ is a hydrogen atom or a C3-C4 alkynyl group, and R³, R⁷, R⁸ and R⁹ are hydrogen atoms,
an N-(α-cyanobenzyl)amide compound represented by the formula (1) wherein R¹ is a hydrogen atom, a halogen atom, a C1-C6 alkyl group, a C1-C6 alkoxy group or a C1-C6 haloalkoxy group, R² is a hydrogen atom, a halogen atom, a C1-C6 alkyl group, a C1-C6 alkoxy group or a C1-C6 haloalkoxy group, or R¹ and R² are taken together to form a C3-C5 alkylene group or CH=CH-CH=CH, R⁴ is a C1-C4 alkyl group, a C3-C4 alkenyl group or a C3-C4 alkynyl group, R⁵ is a C1-C4 alkyl group, a C3-C4 alkenyl group or a C3-C4 alkynyl group, R⁶ is a hydrogen atom or a C3-C4 alkynyl group, and R³, R⁷, R⁸, R⁹, R¹⁰, R¹¹ and R¹² are hydrogen atoms, an N-(α-cyanobenzyl)amide compound represented by the formula (1) wherein R¹ is a halogen atom; a nitro group; a cyano group; a C1-C6 alkyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C2-C6 alkenyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C2-C6 alkynyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C3-C6 cycloalkyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C1-C6 alkoxy group optionally substituted with a substituent selected from the group consisting of a halogen atom, a C1-C6 alkoxy group and a C1-C6 alkylthio group; a C3-C6 cycloalkoxy group optionally substituted with a substituent selected from the group consisting of a halogen atom, a C1-C6 alkoxy group and a C1-C6 alkylthio group; a C1-C6 alkylthio group optionally substituted with a substituent selected from the group consisting of a halogen atom, a C1-C6 alkoxy group and a C1-C6 alkylthio group; a C3-C6 cycloalkylthio group optionally substituted with a substituent selected from the group consisting of a halogen atom, a C1-C6 alkoxy group and a C1-C6 alkylthio group; a di(C1-C6 alkyl)amino group optionally substituted with a substituent selected from the group consisting of a halogen atom, a C1-C6 alkoxy group and a C1-C6 alkylthio group; a phenyl group optionally substituted with a substituent selected from the group consisting of a halogen atom, a C1-C6 alkyl group, a C1-C6 haloalkyl group and a C1-C6 alkoxy group; a phenoxy group optionally substituted with a substituent selected from the group consisting of a halogen atom, a C1-C6 alkyl group, a C1-C6 haloalkyl group and a C1-C6 alkoxy group; a phenylthio group optionally substituted with a substituent selected from the group consisting of a halogen atom, a C1-C6 alkyl group, a C1-C6 haloalkyl group and a C1-C6 alkoxy group; a benzoyl group optionally substituted with a substituent selected from the group consisting of a halogen atom, a C1-C6 alkyl group, a C1-C6 haloalkyl group and a C1-C6 alkoxy group; a benzoyloxy group optionally substituted wit a substituent selected from the group consisting of a halogen atom, a C1-C6 alkyl group, a C1-C6 haloalkyl group and a C1-C6 alkoxy group; a formyl group; a (C1-C6 alkyl)carbonyl group; a (C2-C6 alkenyl)carbonyl group; a (C2-C6 alkynyl)carbonyl group; a (C3-C6 cycloalkyl)carbonyl group; a (C1-C6 haloalkyl)carbonyl group; a (C1-C6 alkoxy)carbonyl group; or a tri(C1-C6 alkyl)silyl group, a R² is a hydrogen atom, a halogen atom, a C1-C6 alkyl group, a C3-C6 cycloalkyl group, a C2-C6 alkenyl group, a C2-C6 alkynyl group, a C1-C6 haloalkyl group, a C1-C6 alkoxy group, a C1-C6 haloalkoxy group, a C1-C6 alkylthio group, a C1-C6 haloalkylthio group, a di(C1-C6 alkyl)amino group, a nitro group or a cyano group, or R¹ and R² are taken together to form a C3-C5 polymethylene group optionally substituted with a substituent selected from the group consisting of a halogen atom, a C1-C3 alkyl group and a C1-C3 haloalkyl group; or a 1,3-butadiene-1,4-diyl group optionally substituted with a substituent selected from the group consisting of a halogen atom, a C1-C3 alkyl group and a C1-C3 haloalkyl group,
an N-(α-cyanobenzyl)amide compound represented by the formula (1) wherein R¹ is a halogen atom; a nitro group; a cyano group; a C1-C6 alkyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C2-C6 alkenyl group optionally substituted with a substituent selected from the group consisting of a halogen atom, a cyano group, a hydroxyl group, a C3-C6 cyclolalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C2-C6 alkynyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C3-C6 cycloalkyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C1-C6 alkoxy group optionally substituted with a substituent selected from the group consisting of a halogen atom, a C1-C6 alkoxy group and a C1-C6 alkylthio group; a C3-C6 cycloalkoxy group optionally substituted with a substituent selected from the group consisting of a halogen atom, a C1-C6 alkoxy group and a C1-C6 alkylthio group; a C1-C6 alkylthio group optionally substituted with a substituent selected from the group consisting of a halogen atom, a C1-C6 alkoxy group and a C1-C6 alkylthio group; a C3-C6 cycloalkylthio group optionally substituted with a substituent selected from the group consisting of a halogen atom, a C1-C6 alkoxy group and a C1-C6 alkylthio group; a di(C1-C6 alkyl)amino group optionally substituted with a substituent selected from the group consisting of a halogen atom, a C1-C6 alkoxy group and a C1-C6 alkylthio group; a phenyl group optionally substituted with a substituent selected from the group consisting of a halogen atom, a C1-C6 alkyl group, a C1-C6 haloalkyl group and a C1-C6 alkoxy group; a phenoxy group optionally substituted with a substituent selected from the group consisting of a halogen atom, a C1-C6 alkyl group, a C1-C6 haloalkyl group and a C1-C6 alkoxy group; a phenylthio group optionally substituted with a substituent selected from the group consisting of a halogen atom, a C1-C6 alkyl group, a C1-C6 haloalkyl group and a C1-C6 alkoxy group; a benzoyl group optionally substituted with a substituent selected from the group consisting of a halogen atom, a C1-C6 alkyl group, a C1-C6 haloalkyl group and a C1-C6 alkoxy group; a benzoyloxy group optionally substituted with a substituent selected from the group consisting of a halogen atom, a C1-C6 alkyl group, a C1-C6 haloalkyl group and a C1-C6 alkoxy group; a formyl group; a (C1-C6 alkyl)carbonyl group; a (C2-C6 alkenyl)carbonyl group; a (C2-C6 alkynyl)carbonyl group; a (C3-C6 cycloalkyl)carbonyl group; a (C1-C6 haloalkyl)carbonyl group; a (C1-C6 alkoxy)carbonyl group; or a tri(C1-C6 alkyl)silyl group, R² is a hydrogen atom, a halogen atom, a C1-C6 alkyl group, a C3-C6 cycloalkyl group, a C2-C6 alkenyl group, a C2-C6 alkynyl group or a C1-C6 haloalkyl group, or R¹ and R² are taken together to form a C3-C5 polymethylene group optionally substituted with a substituent selected from the group consisting of a halogen atom, a C1-C3 alkyl group and a C1-C3 haloalkyl group; or a 1,3-butadiene-1,4-diyl group optionally substituted with a substituent selected from the group consisting of a halogen atom, a C1-C3 alkyl group and a C1-C3 haloalkyl group,
an N-(α-cyanobenzyl)amide compound represented by the formula (1) wherein R¹ is a halogen atom; a nitro group; a cyano group; a C1-C6 alkyl group optionally substituted with a substituent selected from a group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C2-C6 alkenyl group; a C2-C6 alkynyl group; a C3-C6 cycloalkyl group; a C1-C6 alkoxy group optionally substituted with a halogen atom; a C3-C6 cycloalkoxy group optionally substituted with a halogen atom; a C1-C6 alkylthio group optionally substituted with a halogen atom; a C3-C6 cycloalkylthio group optionally substituted with a halogen atom; a di(C1-C6 alkyl)amino group; a phenyl group optionally substituted with a substituent selected from the group consisting of a halogen atom, a C1-C6 alkyl group, a C1-C6 haloalkyl group and a C1-C6 alkoxy group; a phenoxy group optionally substituted with a substituent selected from the group consisting of a halogen atom, a C1-C6 alkyl group, a C1-C6 haloalkyl group and a C1-C6 alkoxy group; a formyl group; a (C1-C6 alkyl)carbonyl group; a (C1-C6 alkoxy)Carbonyl group; or a tri(C1-C6 alkyl)silyl group, R² is a hydrogen atom, a halogen atom, a C1-C6 alkyl group, a C3-C6 cycloalkyl group, a C2-C6 alkenyl group, a C2-C6 alkynyl group or a C1-C6 haloalkyl group, or R¹ and R² are taken together to form a C3-C5 polymethylene group optionally substituted with a substituent selected from the group consisting of a halogen atom, a C1-C3 alkyl group and a C1-C3 haloalkyl group; or a 1,3-butadiene-1,4-diyl group optionally substituted with a substituent selected from the group consisting of a halogen atom, a C1-C3 alkyl group and a C1-C3 haloalkyl group,
an N-(α-cyanobenzyl)amide compound represented by the formula (1) wherein R¹ is a hydrogen atom, a halogen atom, a C1-C6 alkyl group, a C2-C6 alkenyl group, a C2-C6 alkynyl group, a C1-C6 haloalkyl group, a C1-C6 alkoxy group, a C1-C6 haloalkoxy group, a C1-C6 alkylthio group, a C1-C6 haloalkylthio group, a di(C1-C6 alkyl)amino group, a phenyl group, a phenoxy group or a cyano group, R² is a hydrogen atom, a halogen atom, a C1-C6 alkyl group, a C1-C6 alkoxy group or a C1-C6 haloalkyl group, or R¹ and R² are taken together to form a C3-C5 alkylene group or a CH=CH-CH=CH group, R³ is a hydrogen atom, a halogen atom or a C1-C3 alkyl group, R⁴ is a C1-C4 alkyl group, a C3-C4 alkenyl group or a C3-C4 alkynyl group, R⁵ is a C1-C4 alkyl group, a C3-C4 alkenyl group, a C3-C6 alkynyl group, a C1-C4 haloalkyl group or a (C1-C3)alkylcarbonyl group, R⁶ is a hydrogen atom or a C1-C4 alkyl group, a C3-C4 alkenyl group, or a C3-C4 alkynyl group, R⁷ is a hydrogen atom, a halogen atom or a C1-C3 alkyl group, R⁸ is a hydrogen atom, a halogen atom or a C1-C3 alkyl group, R⁹ is a hydrogen atom or a C1-C3 alkyl group, and R¹⁰, R¹¹ and R¹² are hydrogen atoms; that is, an N-(α-cyanobenzyl)amide compound represented by the formula (60): 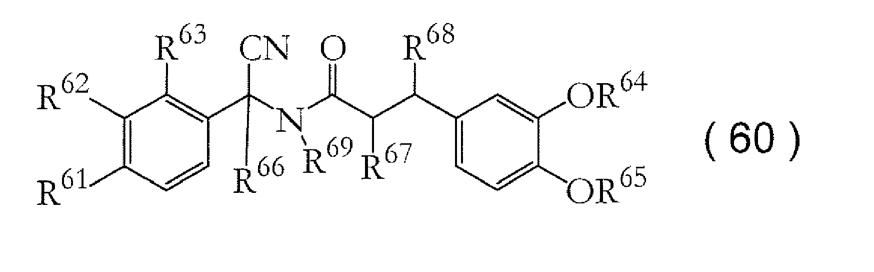 wherein R⁶¹ represents a hydrogen atom, a halogen atom, a C1-C6 alkyl group, a C2-C6 alkenyl group, a C2-C6 alkynyl group, a C1-C6 haloalkyl group, a C1-C6 alkoxy group, a C1-C6 haloalkoxy group, a C1-C6 alkylthio group, a C1-C6 haloalkylthio group, a di (C1-C6 alkyl)amino group, a phenyl group, a phenoxy group or a cyano group, R⁶² represents a hydrogen atom, a halogen atom, a C1-C6 alkyl group, a C1-C6 alkoxy group or a C1-C6 haloalkyl group, or R⁶¹ and R⁶² are taken together to represent a C3-C5 alkylene group or CH=CH-CH=CH, R⁶³ represents a hydrogen atom, a halogen atom or a C1-C3 alkyl group, a R⁶⁴ represents a C1-C4 alkyl group, a C3-C4 alkenyl group or a C3-C4 alkynyl group, R⁶⁵ represents a C1-C4 alkyl group, a C3-C4 alkenyl group, a C3-C6 alkynyl group, a C1-C4 haloalkyl group or a (C1-C3 alkyl)carbonyl group, R⁶⁶ represents a hydrogen atom or a C1-C4 alkyl group, a C3-C4 alkenyl group, or a C3-C4 alkynyl group, R⁶⁷ and R⁶⁸ independently represent a hydrogen atom, a halogen atom, or a C1-C3 alkyl group, and R⁶⁹ represents a hydrogen atom or a C1-C3 alkyl group, an N-(α-cyanobenzyl)amide compound represented by the formula (60) wherein R⁶³ is a hydrogen atom, an N-(α-cyanobenzyl)amide compound represented by the formula (60) wherein R⁶⁴ is a C1-C4 alkyl group, a C3-C4 alkenyl group or a C3-C4 alkynyl group,
an N-(α-cyanobenzyl)amide compound represented by the formula (60) wherein R⁶⁵ is a C1-C4 alkyl group, a C3-C4 alkenyl group or a C3-C4 alkynyl group,
an N-(α-cyanobenzyl)amide compound represented by the formula (60) wherein R⁶⁶ is a hydrogen atom,
an N-(α-cyanobenzyl)amide compound represented by the formula (60) wherein R⁶⁷ is a hydrogen atom,
an N-(α-cyanobenzyl)amide compound represented by the formula (60) wherein R⁶⁸ is a hydrogen atom,
an N-(α-cyanobenzyl)amide compound represented by the formula (60) wherein R⁶⁹ is a hydrogen atom,
an N-(α-cyanobenzyl)amide compound represented by the formula (60) wherein R⁶⁶ and R⁶⁹ are hydrogen atoms,
an N-(α-cyanobenzyl)amide compound represented by the formula (60) wherein R⁶⁷ and R⁶⁸ are hydrogen atoms,
an N-(α-cyanobenzyl)amide compound represented by the formula (60) wherein R⁶³, R⁶⁷ and R⁶⁸ are hydrogen atoms,
an N-(α-cyanobenzyl)amide compound represented by the formula (60) wherein R⁶³, R⁶⁷, R⁶⁸ and R⁶⁹ are hydrogen atoms, an N-(α-cyanobenzyl)amide compound represented by the formula (60) wherein R⁶⁴ and R⁶⁵ are each a C1-C4 alkyl group, a C3-C4 alkenyl group or a C3-C4 alkynyl group,
an N-(α-cyanobenzyl)amide compound represented by the formula (60) wherein R⁶⁶ is a hydrogen atom or a C3-C4 alkynyl group, and R⁶³, R⁶⁷ and R⁶⁸ are hydrogen atoms,
an N-(α-cyanobenzyl)amide compound represented by the formula (60) wherein R⁶⁴ is a C1-C4 alkyl group, a C3-C4 alkenyl group or a C3-C4 alkynyl group, R⁶⁵ is a C1-C4 alkyl group, a C3-C4 alkenyl group or a C3-C4 alkynyl group, R⁶⁶ is a hydrogen atom or a C3-C4 alkynyl group, and R⁶³, R⁶⁷ and R⁶⁸ are hydrogen atoms,
an N-(α-cyanobenzyl)amide compound represented by the formula (60) wherein R⁶¹ is a halogen atom, a C1-C6 alkyl group, a C2-C6 alkenyl group, a C2-C6 alkynyl group, a C1-C6 haloalkyl group, a C1-C6 alkoxy group, a C1-C6 haloalkoxy group, a C1-C6 alkylthio group, a C1-C6 haloalkylthio group, a di(C1-C6 alkyl)amino group, a phenyl group, a phenoxy group or a cyano group, R⁶² is a hydrogen atom, a halogen atom, a C1-C6 alkyl group, a C1-C6 alkoxy group or a C1-C6 haloalkyl group, or R⁶¹ and R⁶² are taken together to form a C3-C5 alkylene group or CH=CH-CH=CH,
an N-(α-cyanobenzyl)amide compound represented by the formula (60) wherein R⁶¹ is a halogen atom, a C1-C6 alkyl group, a C2-C6 alkenyl group, a C2-C6 alkynyl group, a C1-C6 haloalkyl group, a C1-C6 alkoxy group, a C1-C6 haloalkoxy group, a C1-C6 alkylthio group, a C1-C6 haloalkylthio group, a di(C1-C6 alkyl)amino group, a phenyl group, a phenoxy group or a cyano group, R⁶² represents a hydrogen atom, a halogen atom, a C1-C6 alkyl group or a C1-C6 haloalkyl group, or R⁶¹ and R⁶² are taken together to form a C3-C5 alkylene group or CH=CH-CH=CH,
an N-(α-cyanobenzyl)amide compound represented by the formula (60) wherein R⁶¹ and R⁶² are a hydrogen atom, a halogen atom, a C1-C6 alkyl group, a C1-C6 alkoxy group or a C1-C6 haloalkoxy group, or R⁶¹ and R⁶² are taken together to form a C3-C5 alkylene group or a CH=CH-CH=CH group, R⁶⁴ is a C1-C4 alkyl group, a C3-C4 alkenyl group or a C3-C4 alkynyl group, R⁶⁵ is a C1-C4 alkyl group, a C3-C4 alkenyl group or a C3-C4 alkynyl group, R⁶⁶ is a hydrogen atom or a C3-C4 alkynyl group, and R⁶³, R⁶⁷, R⁶⁸ and R⁶⁹ are hydrogen atoms,
an N-(α-cyanobenzyl)amide compound represented by the formula (1) wherein R¹ is a hydrogen atom, a halogen atom, a C1-C4 alkyl group, a C1-C4 alkoxy group or a C1-C4 haloalkyl group, R² is a hydrogen atom, a halogen atom, a C1-C4 alkyl group, a C1-C4 alkoxy group or a C1-C4 haloalkyl group, or R¹ and R² are taken together to form a C3-C5 alkylene group or a CH=CH-CH=CH group, R⁴ is a C1-C4 alkyl group, a C3-C4 alkenyl group or a C3-C4 alkynyl group, R⁵ is a C1-C4 alkyl group, a C3-C4 alkenyl group or a C3-C4 alkynyl group, R⁶ is a hydrogen atom or a C3-C4 alkyl group, and R³, R⁷, R⁸, R⁹, R¹⁰, R¹¹ and R¹² are hydrogen atoms, that is, an N-(α-cyanobenzyl)amide compound represented by the formula (50): 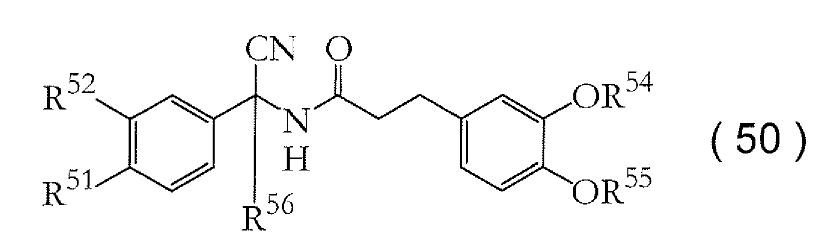 wherein R⁵¹ and R⁵² are the same or different, and each represent a hydrogen atom, a halogen atom, a C1-C4 alkyl group, a C1-C4 alkoxy group or a C1-C4 haloalkyl group, or R⁵¹ and R⁵² are taken together to represent a C3-C5 alkylene group or a CH=CH-CH=CH group, R⁵⁴ represents a C1-C4 alkyl group, a C3-C4 alkenyl group or a C3-C4 alkynyl group, R⁵⁵ represents a C1-C4 alkyl group, a C3-C4 alkenyl group or a C3-C4 alkynyl group, and R⁵⁶ represents a hydrogen atom or a C3-C4 alkynyl group,
an N-(α-cyanobenzyl)amide compound represented by the formula (50) wherein R⁵¹ is a halogen atom,
an N-(α-cyanobenzyl)amide compound represented by the formula (50) wherein R⁵¹ is a C1-C4 alkyl group,
an N-(α-cyanobenzyl)amide compound represented by the formula (50) wherein R⁵¹ is a C1-C4 alkoxy group,
an N-(α-cyanobenzyl)amide compound represented by the formula (50) wherein R⁵¹ is a C1-C4 haloalkyl group,
an N-(α-cyanobenzyl)amide compound represented by the formula (50) wherein R⁵¹ is a C1-C2 alkyl group,
an N-(α-cyanobenzyl)amide compound represented by the formula (50) wherein R⁵¹ is a fluorine atom,
an N-(α-cyanobenzyl)amide compound represented by the formula (50) wherein R⁵¹ is a hydrogen atom,
an N-(α-cyanobenzyl)amide compound represented by the formula (50) wherein R⁵¹ is a chlorine atom,
an N-(α-cyanobenzyl)amide compound represented by the formula (50) wherein R⁵¹ is a bromine atom,
an N-(α-cyanobenzyl)amide compound represented by the formula (50) wherein R⁵¹ is a methyl group,
an N-(α-cyanobenzyl)amide compound represented by the formula (50) wherein R⁵¹ is a methoxy group,
an N-(α-cyanobenzyl)amide compound represented by the formula (50) wherein R⁵¹ is a trifluoromethyl group, an N-(α-cyanobenzyl)amide compound represented by the formula (50) wherein R⁵² is a hydrogen atom,
an N-(α-cyanobenzyl)amide compound represented by the formula (50) wherein R⁵² is a halogen atom,
an N-(α-cyanobenzyl)amide compound represented by the formula (50) wherein R⁵² is a chlorine atom,
an N-(α-cyanobenzyl)amide compound represented by the formula (50) wherein R⁵⁴ is a C1-C4 alkyl group,
an N-(α-cyanobenzyl)amide compound represented by the formula (50) wherein R⁵⁴ is a C1-C2 alkyl group,
an N-(α-cyanobenzyl)amide compound represented by the formula (50) wherein R⁵⁴ is a C3-C4 alkynyl group,
an N-(α-cyanobenzyl)amide compound represented by the formula (50) wherein R⁵⁴ is a 2-propynyl group,
an N-(α-cyanobenzyl)amide compound represented by the formula (50) wherein R⁵⁵ is a C1-C4 alkyl group,
an N-(α-cyanobenzyl)amide compound represented by the formula (50) wherein R⁵⁵ is a C3-C4 alkynyl group,
an N-(α-oyanobenzyl)amide compound represented by the formula (50) wherein R⁵⁵ is a C1-C2 alkyl group,
an N-(α-cyanobenzyl)amide compound represented by the formula (50) wherein R⁵⁵ is a 2-propynyl group,
an N-(α-cyanobenzyl)amide compound represented by the formula (50) wherein R⁵⁵ is a 2-propenyl group,
an N-(α-cyanobenzyl)amide compound represented by the formula (50) wherein R⁵⁶ is a hydrogen atom,
an N-(α-cyanobenzyl)amide compound represented by the formula (50) wherein R⁵¹ is a halogen atom and R⁵² is a hydrogen atom,
an N-(α-cyanobenzyl)amide compound represented by the formula (50) wherein R⁵¹ is a halogen atom and R⁵² is a halogen atom,
an N-(α-cyanobenzyl)amide compound represented by the formula (50) wherein R⁵¹ is a C1-C4 alkyl group and R⁵² is a hydrogen atom,
an N-(α-cyanobenzyl)amide compound represented by the formula (50) wherein R⁵¹ is a C1-C4 haloalkyl group and R⁵² is a hydrogen atom,
an N-(α-cyanobenzyl)amide compound represented by the formula (50) wherein R⁵¹ is a chlorine atom and R⁵² is a hydrogen atom,
an N-(α-cyanobenzyl)amide compound represented by the formula (50) wherein R⁵¹ is a chlorine atom and R⁵² is a halogen atom,
an N-(α-cyanobenzyl)amide compound represented by the formula (50) wherein R⁵¹ is a C1-C2 alkyl group and R⁵² is a hydrogen atom,
an N-(α-cyanobenzyl) amide compound represented by the formula (50) wherein R¹ is a C1-C4 alkoxy group and R⁵² is a hydrogen atom,
an N-(α-cyanobenzyl)amide compound represented by the formula (50) wherein R⁵¹ is a trifluoromethyl group and R⁵² is a hydrogen atom,
an N-(α-cyanobenzyl) amide compound represented by the formula (50) wherein R⁵¹ is a halogen atom, R⁵² is a hydrogen atom, and R⁵⁴ is a C1-C4 alkyl group,
an N-(α-cyanobenzyl)amide compound represented by the formula (50) wherein R⁵¹ is a halogen atom, R⁵² is a hydrogen atom, and R⁵⁴ is a C1-C2 alkyl group,
an N-(α-cyanobenzyl)amide compound represented by the formula (50) wherein R⁵¹ is a halogen atom, R⁵² is a hydrogen atom, and R⁵⁵ is a C1-C4 alkyl group,
an N-(α-cyanobenzyl)amide compound represented by the formula (50) wherein R⁵¹ is a halogen atom, R⁵² is a hydrogen atom, and R⁵⁵ is a C3-C4 alkynyl group,
an N-(α-cyanobenzyl)amide compound represented by the formula (50) wherein R⁵¹ is a halogen atom, R⁵² is a hydrogen atom, and R⁵⁵ is a C1-C2 alkyl group,
an N-(α-cyanobenzyl)amide compound represented by the formula (50) wherein R⁵¹ is a C1-C4 alkyl group, R⁵² is a hydrogen atom, and R⁵⁴ is a C1-C4 alkyl group,
an N-(α-cyanobenzyl)amide compound represented by the formula (50) wherein R⁵¹ is a C1-C4 alkyl group, R⁵² is a hydrogen atom, and R⁵⁴ is a C1-C2 alkyl group,
an N-(α-cyanobenzyl)amide compound represented by the formula (50) wherein R⁵¹ is a C1-C4 alkyl group, R⁵² is a hydrogen atom, and R⁵⁵ is a C1-C4 alkyl group,
an N-(α-cyanobenzyl)amide compound represented by the formula (50) wherein R⁵¹ is a C1-C4 alkyl group, R⁵² is a hydrogen atom, and R⁵⁵ is a C3-C4 alkynyl group,
an N-(α-cyanobenzyl)amide compound represented by the formula (50) wherein R⁵¹ is a C1-C4 alkyl group, R⁵² is a hydrogen atom, and R⁵⁵ is a 2-propynyl group,
an N-(α-cyanobenzyl)amide compound represented by the formula (50) wherein R⁵¹ is a C1-C4 alkoxy group, R⁵² is a hydrogen atom, and R⁵⁴ is a C1-C4 alkyl group,
an N-(α-cyanobenzyl)amide compound represented by the formula (50) wherein R⁵¹ is a C1-C4 alkoxy group, R⁵² is a hydrogen atom, and R⁵⁴ is a C1-C2 alkyl group,
an N-(α-cyanobenzyl)amide compound represented by the formula (50) wherein R⁵¹ is a C1-C4 alkoxy group, R⁵² is a hydrogen atom, and R⁵⁵ is a C1-C4 alkyl group,
an N-(α-cyanobenzyl)amide compound represented by the formula (50) wherein R⁵¹ is a C1-C4 alkoxy group, R⁵² is a hydrogen atom, and R⁵⁵ is a C3-C4 alkynyl group,
an N-(α-cyanobenzyl)amide compound represented by the formula (50) wherein R⁵¹ is a C1-C4 alkoxy group, R⁵² is a hydrogen atom, and R⁵⁵ is a 2-propynyl group,
an N-(α-cyanobenzyl)amide compound represented by the formula (50) wherein R⁵¹ is a C1-C4 haloalkyl group, R⁵² is a hydrogen atom, and R⁵⁴ is a C1-C4 alkyl group,
an N-(α-cyanobenzyl)amide compound represented by the formula (50) wherein R⁵¹ is a C1-C4 haloalkyl group, R⁵² is a hydrogen atom, and R⁵⁴ is a C1-C2 alkyl group,
an N-(α-cyanobenzyl)amide compound represented by the formula (50) wherein R⁵¹ is a C1-C4 haloalkyl group, R⁵² is a hydrogen atom, and R⁵⁵ is a C3-C4 alkynyl group,
an N-(α-cyanobenzyl)amide compound represented by the formula (50) wherein R⁵¹ is a C1-C4 haloalkyl group, R⁵² is a hydrogen atom, and R⁵⁵ is a 2-propynyl group,
an N-(α-cyanobenzyl)amide compound represented by the formula (50) wherein R⁵² is a hydrogen atom and R⁵⁴ is a C1-C4 alkyl group,
an N-(α-cyanobenzyl)amide compound represented by the formula (50) wherein R⁵² is a hydrogen atom and R⁵⁴ is a C1-C2 alkyl group,
an N-(α-cyanobenzyl)amide compound represented by the formula (50) wherein R⁵² is a hydrogen atom and R⁵⁵ is a C1-C4 alkyl group,
an N-(α-cyanobenzyl)amide compound represented by the formula (50) wherein R⁵² is a hydrogen atom and R⁵⁵ is a C3-C4 alkynyl group,
an N-(α-cyanobenzyl)amide compound represented by the formula (50) wherein R⁵² is a hydrogen atom and R⁵⁵ is a C1-C2 alkyl group,
an N-(α-cyanobenzyl)amide compound represented by the formula (50) wherein R⁵² is a hydrogen atom and R⁵⁵ is a 2-propynyl group,
an N-(α-cyanobenzyl)amide compound represented by the formula (50) wherein R ⁵² is a hydrogen atom, R⁵⁴ is a C1-C2 alkyl group, and R⁵ is a C3-C4 alkynyl group,
an N-(α-cyanobenzyl)amide compound represented by the formula (50) wherein R⁵² is a hydrogen atom, R⁵⁴ is a C1-C2 alkyl group, and R⁵⁵ is a 2-propynyl group,
an N-(α-cyanobenzyl)amide compound represented by the formula (50) wherein R⁵⁴ is a C1-C4 alkyl group and R⁵⁵ is a C1-C4 alkyl group,
an N-(α-cyanobenzyl)amide compound represented by the formula (50) wherein R⁵⁴ is a C1-C4 alkyl group and R⁵⁵ is a C3-C4 alkynyl group,
an N-(α-cyanobenzyl)amide compound represented by the formula (50) wherein R⁵⁴ is a C1-C4 alkyl group and R⁵⁵ is a C1-C2 alkyl group,
an N-(α-cyanobenzyl)amide compound represented by the formula (50) wherein R⁵⁴ is a C1-C4 alkyl group and R⁵⁵ is a 2-propynyl group,
an N-(α-cyanobenzyl)amide compound represented by the formula (50) wherein R⁵⁴ is a C1-C2 alkyl group and R⁵⁵ is a C1-C4 alkyl group,
an N-(α-cyanobenzyl)amide compound represented by the formula (50) wherein R⁵⁴ is a C1-C2 alkyl group and R⁵⁵ is a C3-C4 alkynyl group,
an N-(α-cyanobenzyl)amide compound represented by the formula (50) wherein R⁵⁴ is a C1-C2 alkyl group and R⁵⁵ is a C1-C2 alkyl group,
an N-(α-cyanobenzyl)amide compound represented by the formula (50) wherein R⁵⁴ is a C1-C2 alkyl group and R⁵⁵ is a 2-propynyl group,
an N-(α-cyanobenzyl)amide compound represented by the formula (50) wherein R⁵² is a hydrogen atom, R⁵⁴ is a C1-C4 alkyl group, and R⁵⁵ is a C3-C4 alkynyl group,
an N-(α-cyanobenzyl)amide compound represented by the formula (50) wherein R⁵² is a hydrogen atom, R⁵⁴ is a C1-C4 alkyl group, and R⁵⁵ is a C1-C2 alkyl group,
an N-(α-cyannbenzyl)amide compound represented by the formula (50) wherein R⁵² is a hydrogen atom, R⁵⁴ is a C1-C4 alkyl group, and R⁵⁵ is a 2-propynyl group,
an N-(α-cyanobenzyl)amide compound represented by the formula (1) wherein R¹ is a halogen atom, a C1-C6 alkyl group, a C2-C6 alkenyl group, a C2-C6 alkynyl group, a C1-C6 haloalkyl group, a C1-C6 alkoxy group, a C1-C6 haloalkoxy group, a C1-C6 alkylthio group, a C1-C6 haloalkylthio group, a di(C1-C6 alkyl)amino group, a phenyl group, a phenoxy group or a cyano group, and R², R³, R⁷, R⁸ and R⁹ are hydrogen atoms,
an N-(α-cyanobenzyl)amide compound represented by the formula (1) wherein R¹ is a halogen atom, a C1-C6 alkyl group, a C2-C6 alkenyl group, a C2-C6 alkynyl group, a C1-C6 haloalkyl group, a C1-C6 alkoxy group, a C1-C6 haloalkoxy group, a C1-C6 alkylthio group, a C1-C6 haloalkylthio group, a di(C1-C6 alkyl)amino group, a phenyl group, a phenoxy group or a cyano group, and R³, R⁶, R⁷, R⁸ and R⁹ are hydrogen atoms,
an N-(α-cyanobenzyl)amide compound represented by the formula (1) wherein R¹ is a halogen atom, a C1-C6 alkyl group, a C2-C6 alkenyl group, a C2-C6 alkynyl group, a C1-C6 haloalkyl group, a C1-C6 alkoxy group, a C1-C6 haloalkoxy group, a C1-C6 alkylthio group, a C1-C6 haloalkylthio group, a di(C1-C6 alkyl)amino group, a phenyl group, a phenoxy group or a cyano group, and R², R³, R⁶, R⁷, R⁸ and R⁹ are hydrogen atoms,
an N-(α-cyanobenzyl)amide compound represented by the formula (1) wherein R¹ is a halogen atom, a C1-C6 alkyl group, a C2-C6 alkenyl group, a C2-C6 alkynyl group, a C1-C6 haloalkyl group, a C1-C6 alkoxy group, a C1-C6 haloalkoxy group, a C1-C6 alkylthio group or a C1-C6 haloalkylthio group, and R³, R⁶, R⁷, R⁸ and R⁹ are hydrogen atoms,
an N-(α-cyanobenzyl)amide compound represented by the formula (1) wherein R¹ is a halogen atom, a C1-C6 alkyl group, a C2-C6 alkenyl group, a C2-C6 alkynyl group, a C1-C6 haloalkyl group, a C1-C6 alkoxy group, a C1-C6 haloalkoxy group, a C1-C6 alkylthio group or a C1-C6 haloalkylthio group, and R², R³, R⁶, R⁷, R⁸ and R⁹ are hydrogen atoms,
an N-(α-cyanobenzyl)amide compound represented by the formula (1) wherein R¹ is a halogen atom, a C1-C6 alkyl group, a C2-C6 alkenyl group, a C2-C6 alkynyl group, a C1-C6 haloalkyl group, a C1-C6 alkylthio group or a C1-C6 haloalkylthio group, and R², R³, R⁶, R⁷, R⁸ and R⁹ are hydrogen atoms,
an N-(α-cyanobenzyl)amide compound represented by the formula (1) wherein R¹ is a halogen atom, a C1-C6 alkyl group, a C2-C6 alkenyl group, a C2-C6 alkynyl group, a C1-C6 haloalkyl group, a C1-C6 alkylthio group or a C1-C6 haloalkylthio group, R² is a hydrogen atom, a halogen atom or a C1-C6 alkyl group, and R³, R⁶, R⁷, R⁸ and R⁹ are hydrogen atoms,
an N-(α-cyanobenzyl)amide compound represented by the formula (50) wherein R⁵¹ is a halogen atom, a C1-C4 alkyl group, a C1-C4 alkoxy group or a C1-C4 haloalkyl group and R⁵² is a hydrogen atom, and
an N-(α-cyanobenzyl)amide compound represented by the formula (50) wherein R⁵¹ and R⁵² are the same or different, and are each a halogen atom, a C1-C4 alkyl group, a C1-C4 alkoxy group or a C1-C4 haloalkyl group.

Next, processes for preparing the compound of the present invention will be explained.

The compound of the present invention represented by the formula (1) can be prepared, for example, according to the following (Process A), (Process B) or (Process C).

### (Process A)

The compound of the present invention can be prepared by reacting a compound represented by the formula (2) (or a salt thereof such as hydrochloride) and a compound represented by the formula (3): 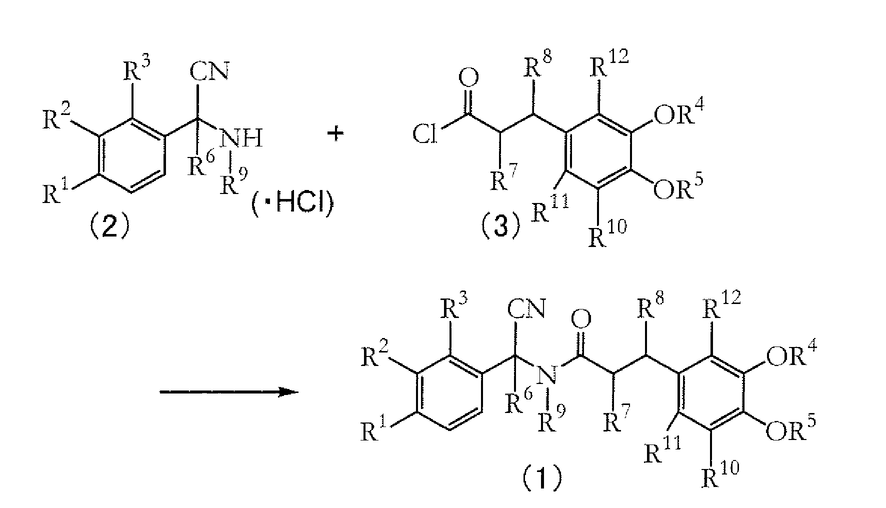 wherein R¹, R², R³, R⁴, R⁵, R⁶, R⁷, R⁸, R⁹, R¹⁰, R¹¹ and R¹² are as defined above.

The reaction is carried out usually in the presence of a solvent and usually in the presence of a base.

The solvent used for the reaction includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene, esters such as ethyl acetate, butyl acetate and the like, nitriles such as acetonitrile, butyronitrile and the like, acid amides such as N,N-dimethylformamide, sulfoxides such as dimethyl sulfoxide and mixtures thereof.

The base used for the reaction includes, for example, carbonates such as sodium carbonate, potassium carbonate and the like, tertiary amines such as triethylamine, diisopropylethylamine, 1,8-diazabicyclo[5.4.0]undec-7-ene, 1,5-diazabicyclo[4.3.0]non-5-ene and the like, and nitrogen-containing aromatic compounds such as pyridine, 4-dimethylaminopyridine and the like.

In the reaction, 1 to 10 mol of a base and 1 to 5 mol of a compound represented by the formula (2) are usually used per 1 mol of a compound represented by the formula (3).

The reaction temperature is usually in the range of - 20 to 100°C, and the reaction time is usually in the range of 0.1 to 24 hours.

After completion of the reaction, a compound represented by the formula (1) can be isolated by, for example, (i) pouring the reaction mixture into water, extracting it with an organic solvent, and then drying and concentrating the resulting organic layer after washing it with acidic water (diluted hydrochloric acid or the like), basic water (an aqueous sodium hydrogen carbonate solution or the like) if necessary, or (ii) concentrating the reaction mixture under reduced pressure after adding a small amount of water and then collecting the obtained solid by filtration. The isolated compound represented by the formula (1) may be further purified by a technique such as chromatography, recrystallization and the like.

### (Process B)

Among the compounds of the present invention, a compound represented by the formula (1-2) in which R⁶ is a C1-C4 alkyl group, a C3-C4 alkenyl group or a C3-C4 alkynyl group can be also prepared by reacting a compound represented by the formula (4) and a compound represented by the formula (5): 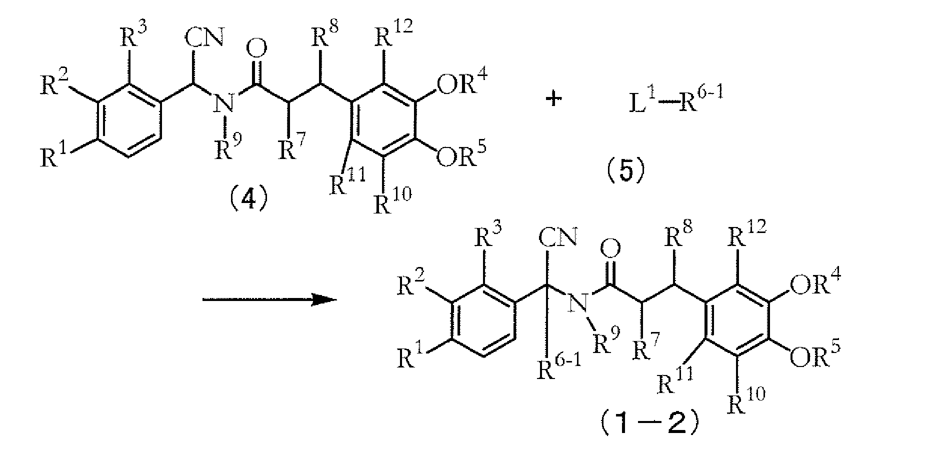 wherein R⁶⁻¹ represents a C1-C4 alkyl group, a C3-C4 alkenyl group or a C3-C4 alkynyl group, L¹ represents a halogen atom, a methanesulfonyloxy group, a trifluoromethanesulfonyloxy group or a p-toluenesulfonyloxy group, and R¹, R², R³, R⁴, R⁵, R⁷, R⁸, R⁹, R¹⁰, R¹¹ and R¹² are as defined above.

The reaction is carried out usually in the presence of a solvent and usually in the presence of a base.

The solvent used for the reaction includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene, esters such as ethyl acetate, butyl acetate and the like, nitriles such as acetonitrile, butyronitrile and the like, acid amides such as N,N-dimethylformamide, sulfoxides such as dimethyl sulfoxide and mixtures thereof.

The base used for the reaction includes, for example, carbonates such as sodium carbonate, potassium carbonate and the like, alkali metal hydrides such as sodium hydride, potassium hydride and the like, tertiary amines such as triethylamine, diisopropylethylamine, 1,8-diazabicyclo[5.4.0]undec-7-ene, 1,5-diazabicyclo[4.3.0]non-5-ene and the like, and nitrogen-containing aromatic compounds such as pyridine, 4-dimethylaminopyridine and the like.

In the reaction, 1 to 10 mol of a base and 1 to 5 mol of a compound represented by the formula (5) are usually used per 1 mol of a compound represented by the formula (4).

The reaction temperature is usually in the range of - 20 to 100°C, and the reaction time is usually in the range of 0.1 to 24 hours.

After completion of the reaction, a compound represented by the formula (1-2) can be isolated by, for example, pouring the reaction mixture into water, extracting it with an organic solvent, and then drying and concentrating the resulting organic layer after washing it with acidic water (diluted hydrochloric acid or the like) or basic water (an aqueous sodium hydrogen carbonate solution or the like) if necessary. The isolated compound represented by the formula (1-2) may be further purified by a technique such as chromatography, recrystallization and the like.

### (Process C)

The compound of the present invention can be also prepared by reacting a compound represented by the formula (2) (or a salt thereof such as hydrochloride etc.) and a compound represented by the formula (6): 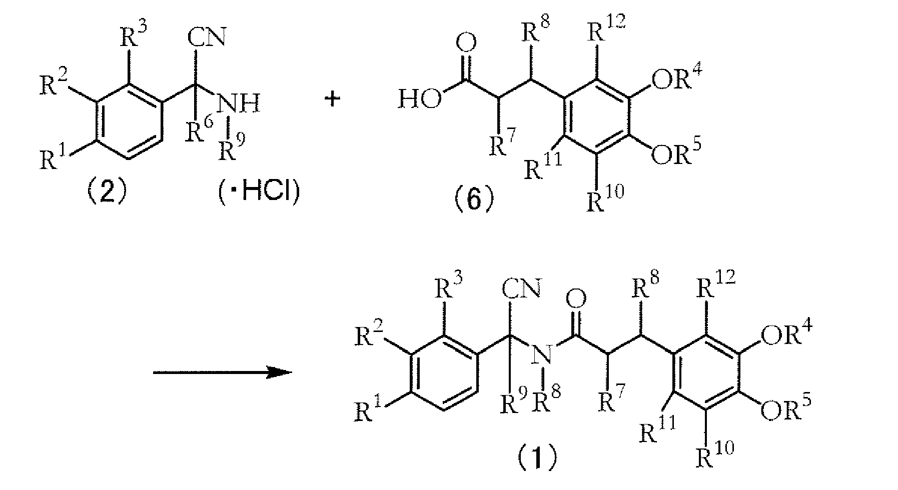 wherein R¹, R², R³, R⁴, R⁵, R⁶, R⁷, R⁸, R⁹, R¹⁰, R¹¹ and R¹² are as defined above.

The reaction is carried out in the presence of a dehydration condensing agent and usually in the presence of a solvent.

The solvent used for the reaction includes, for example, acid amides such as N,N-dimethylformamide, sulfoxides such as dimethyl sulfoxide and the like, nitrogen-containing aromatic compounds such as pyridine, quinoline and the like, and mixtures thereof.

The dehydration condensing agent used for the reaction includes carbodiimides such as 1-ethyl-3-(3-dimethylaminopropyl)carbodiimide hydrochloride (hereinafter, referred to as WSC), 1,3-dicyclohexylcarbodiimide and the like.

In the reaction, 1 to 3 mol of a compound represented by the formula (2) and 1 to 5 mol of a dehydration condensing agent are usually used per 1 mol of a compound represented by the formula (6).

The reaction temperature is usually in the range of 0 to 140°C, and the reaction time is usually in the range of 0.1 to 24 hours.

After completion of the reaction, a compound represented by the formula (1) can be isolated by, for example, (i) pouring the reaction mixture into water, extracting it with an organic solvent, and then drying and concentrating the resulting organic layer after washing it with acidic water (diluted hydrochloric acid or the like), basic water (an aqueous sodium hydrogen carbonate solution or the like) if necessary, or (ii) concentrating the reaction mixture under reduced pressure after adding a small amount of water and then collecting the obtained solid by filtration. The isolated compound represented by the formula (1) may be further purified by a technique such as chromatography, recrystallization and the like.

Next, processes for preparing intermediates for the compound of the present invention will be explained by Reference Process.

### Reference Process

A compound represented by the formula (3) and a compound represented by the formula (6) can be prepared, for example, according to the following scheme: 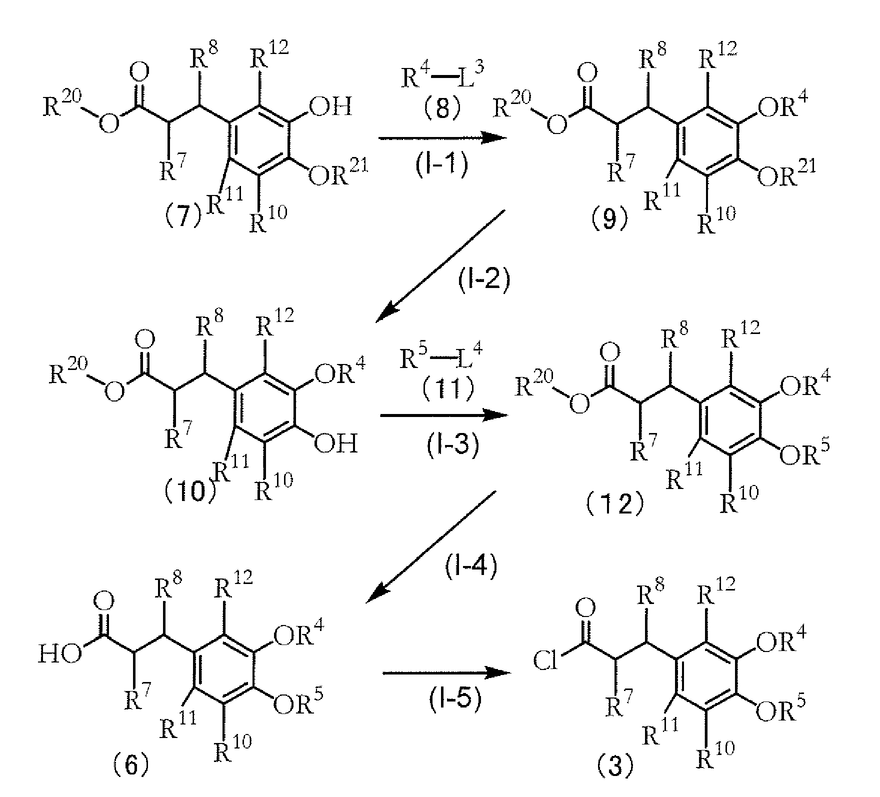 wherein R²⁰ represents a C1-C6 alkyl group (a methyl group, an ethyl group and a propyl group, etc.), R²¹ represents a benzyl group or a methoxymethyl group, L³ and L⁴ independently represent a halogen atom, a methanesulfonyloxy group, a trifluoromethanesulfonyloxy group or a p-toluenesulfonyloxy group, and R⁴, R⁵, R⁷, R⁸, R⁹, R¹⁰, R¹¹ and R¹² are as defined above.

### Step (I-1)

A compound represented by the formula (9) can be prepared by reacting a compound represented by the formula (7) and a compound represented by the formula (8).

The reaction is carried out usually in the presence of a base.

If a solvent is used in the reaction, the solvent includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene, esters such as ethyl acetate, butyl acetate and the like, nitriles such as acetonitrile, butyronitrile and the like, acid amides such as N,N-dimethylformamide, sulfoxides such as dimethyl sulfoxide and mixtures thereof.

The base used for the reaction includes, for example, carbonates such as sodium carbonate, potassium carbonate and the like, alkali metal hydrides such as sodium hydride, potassium hydride and the like, tertiary amines such as triethylamine, diisopropylethylamine, 1,8-diazabicyclo[5.4.0]undec-7-ene, 1,5-diazabicyclo[4.3.0]non-5-ene and the like, and nitrogen-containing aromatic compounds such as pyridine, 4-dimethylaminopyridine and the like.

In the reaction, 1 to 50 mol of a base and 1 to 5 mol of a compound represented by the formula (8) are usually used per 1 mol of a compound represented by the formula (7).

The reaction temperature is usually in the range of 0 to 100°C, and the reaction time is usually in the range of 0.1 to 24 hours.

After completion of the reaction, a compound represented by the formula (9) can be isolated by, for example, pouring the reaction mixture into water, extracting it with an organic solvent and then drying and concentrating the resulting organic layer. The isolated compound represented by the formula (9) may be further purified by a technique such as chromatography, recrystallization and the like.

A compound represented by the formula (7) is the compound described in Tetrahedron Letters, vol. 36, No.51, pp.9369-9372, 1995 or can be produced according to a method similar to that described in the literature.

### Step (I-2)

### (A) In the case that R²¹ is a benzyl group

A compound represented by the formula (10) can be prepared by reacting a compound represented by the formula (9) and hydrogen in the presence of a hydrogenation catalyst.

The reaction is performed under a hydrogen atmosphere and usually in the presence of a solvent.

The solvent used for the reaction includes, for example, alcohols such as methanol, ethanol, propanol and the like, esters such as ethyl acetate, butyl acetate and the like, ethers such as tetrahydrofuran, 1,4-dioxane and the like, and mixtures thereof.

The hydrogenation catalyst used for the reaction includes, for example, transition metal compounds such as palladium carbon, palladium hydroxide, Raney-nickel, platinum oxide and the like.

In the reaction, 0.001 to 0.5 mol of a hydrogenation catalyst is usually used per 1 mol of a compound represented by the formula (9). In the reaction, if necessary, an acid (hydrochloric acid etc.) may be further added. In this case, 0.01 mol to an excessive amount of an acid is usually used per 1 mol of a compound represented by the formula (9).

The reaction is performed usually under a hydrogen atmosphere at 1 to 100 atm. The reaction temperature is usually in a range of -20 to 100°C, and the reaction time is usually in a range of 0.1 to 24 hours.

After completion of the reaction, a compound represented by the formula (10) can be isolated by, for example, filtering the reaction mixture, extracting the filtrate with an organic solvent, and then drying and concentrating the resulting organic layer. The isolated compound represented by the formula (10) may be further purified by a technique such as chromatography, recrystallization and the like.

### (B) In the case that R²¹ is a methoxymethyl group

A compound represented by the formula (10) can be prepared by reacting a compound represented by the formula (9) and water in the presence of an acid.

The reaction is usually performed in the presence of an organic solvent.

The organic solvent used for the reaction includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene and the like, esters such as ethyl acetate, butyl acetate and the like, nitriles such as acetonitrile, butyronitrile and the like, acid amides such as N,N-dimethylformamide and the like, sulfoxides such as dimethyl sulfoxide, alcohols such as methanol, ethanol, propanol and the like, and mixtures thereof.

The acid used for the reaction includes, for example, inorganic acids such as hydrochloric acid, sulfuric acid and the like, and organic acids such as p-toluenesulfonic acid, methanesulfonic acid and the like.

In the reaction, 0.1 to 10 mol of an acid and 3 mol to an excessive amount of water are usually used per 1 mol of a compound represented by the formula (9).

The reaction temperature is usually in a range of 0 to 100°C, and the reaction time is usually in a range of 0.1 to 24 hours.

After completion of the reaction, a compound represented by the formula (10) can be isolated by, for example, extracting the reaction mixture with an organic solvent, and then drying and concentrating the organic layer. The isolated compound represented by the formula (10) may be further purified by a technique such as chromatography, recrystallization and the like.

### Step (I-3)

A compound represented by the formula (12) can be prepared by reacting a compound represented by the formula (10) and a compound represented by the formula (11).

The reaction is usually performed in the presence of a base.

If a solvent is used in the reaction, the solvent includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene and the like, esters such as ethyl acetate, butyl acetate and the like, nitriles such as acetonitrile, butyronitrile and the like, acid amides such as N,N-dimethylformamide, sulfoxides such as dimethyl sulfoxide and the like, and mixtures thereof.

The base used for the reaction includes, for example, carbonates such as sodium carbonate, potassium carbonate and the like, tertiary amines such as triethylamine, diisopropylethylamine, 1,8-diazabicyclo[5.4.0]undec-7-ene, 1,5-diazabicyclo[4.3.0]non-5-ene and the like, and nitrogen-containing aromatic compounds such as pyridine, 4-dimethylaminopyridine and the like.

In the reaction, 1 to 50 mol of a base and 1 to 5 mol of a compound represented by the formula (11) are usually used per 1 mol of a compound represented by the formula (10).

The reaction temperature is usually in a range of 0 to 100°C, and the reaction time is usually in a range of 0.1 to 24 hours.

After completion of the reaction, a compound represented by the formula (12) can be isolated by, for example, filtrating the reaction mixture, after adding an organic solvent if necessary, and then concentrating the filtrate. The isolated compound represented by the formula (12) may be further purified by a technique such as distillation, chromatography, recrystallization and the like.

### Step (I-4)

A compound represented by the formula (6) can be prepared by reacting a compound represented by the formula (12) and water in a presence of a base.

The reaction is performed usually in the presence of an organic solvent.

The organic solvent used for the reaction includes, for example, ethers such as 1,4-dioxane, tetrahydrofuran, ethylene glycol dimethyl ether, tert-butyl methyl ether and the like, aromatic hydrocarbons such as toluene, xylene and the like, halogenated hydrocarbons such as chlorobenzene and the like, nitriles such as acetonitrile, butyronitrile and the like, alcohols such as methanol, ethanol, propanol and the like, and mixtures thereof.

The base used for the reaction includes, for example, alkali metal hydroxides such as lithium hydroxide, sodium hydroxide, potassium hydroxide and the like.

In the reaction, 1 to 10 mol of a base and 1 mol to an excessive amount of water are usually used per 1 mol of a compound represented by the formula (12).

The reaction temperature is usually in a range of 0 to 150°C, and the reaction time is usually in a range of 0.1 to 24 hours.

After completion of the reaction, a compound represented by the formula (6) can be isolated by, for example, adding acidic water (hydrochloric acid etc.) to the reaction mixture, extracting it with an organic solvent, and then drying and concentrating the organic layer. The isolated compound represented by the formula (6) may be further purified by chromatography, recrystallization or the like, or may be used as it is in a next step.

### Step (I-5)

A compound represented by the formula (3) can be prepared by reacting a compound represented by the formula (6) and a chlorinating agent.

If a solvent is used in the reaction, the solvent includes, for example, ethers such as 1,4-dioxane, tetrahydrofuran, ethylene glycol dimethyl ether, tert-butyl methyl ether and the like, aliphatic hydrocarbons such as hexane, heptane and the like, aromatic hydrocarbons such as toluene, xylene and the like, halogenated hydrocarbons such as chlorobenzene and the like, and mixtures thereof.

The chlorinating agent used for the reaction includes thionyl chloride, oxalyl chloride and phosphorus oxychloride.

In the reaction, 1 to 100 mol of a chlorinating agent is usually used per 1 mol of a compound represented by the formula (6).

The reaction temperature is usually in a range of 30 to 150°C, and the reaction time is usually in a range of 0.1 to 24 hours.

After completion of the reaction, a compound represented by the formula (3) can be isolated by, for example, concentrating the reaction mixture as it is. The isolated compound represented by the formula (3) is usually used in a reaction of a next step without purification.

A compound represented by the formula (6-2), that is, the formula (6) in which R⁷ is a hydrogen atom, and a compound represented by the formula (3-2), that is, the formula (3) in which R⁷ is a hydrogen atom can be also prepared, for example, according to the following scheme: 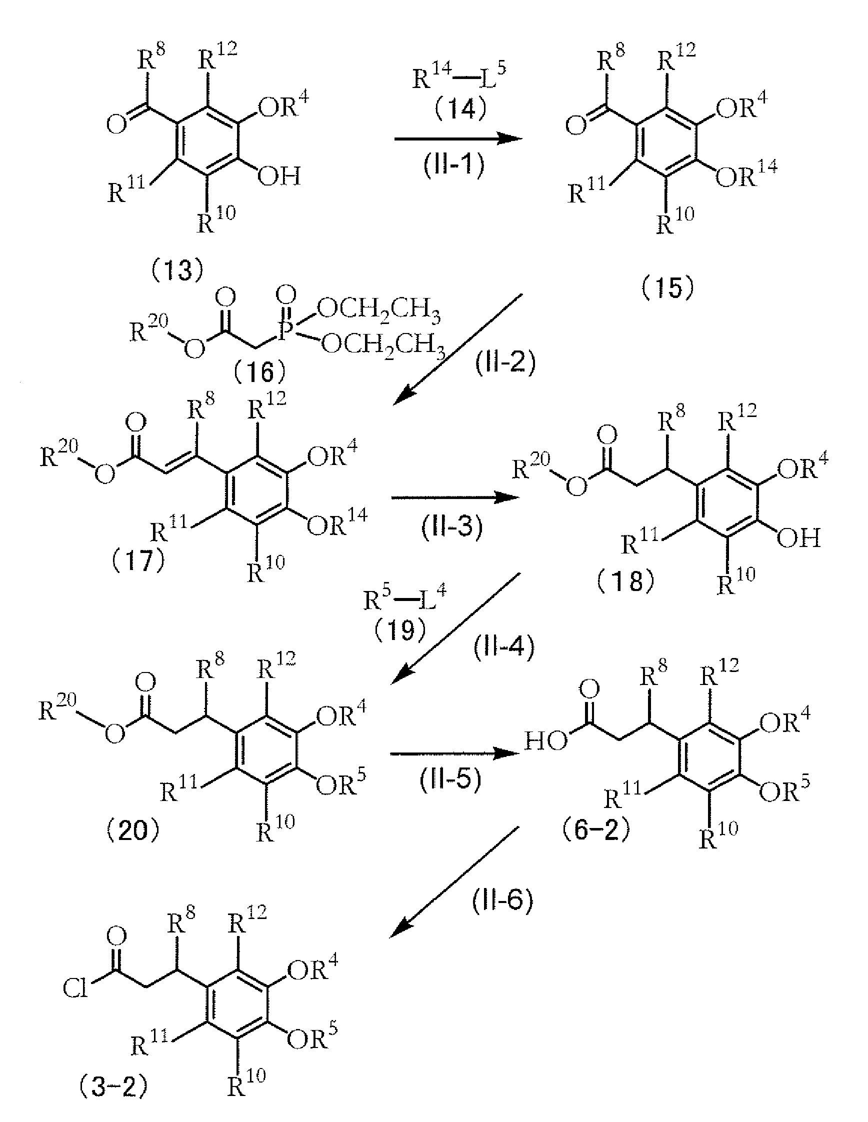 wherein R¹⁴ represents a benzyl group, L⁵ represents a chlorine atom or a bromine atom, and R⁴, R⁵, R⁸, R¹⁰, R¹¹, R¹², R²⁰ and L⁴ are as defined above.

### Step (II-1)

A compound represented by the formula (15) can be prepared by reacting a compound represented by the formula (13) and a compound represented by the formula (14).

The reaction is performed usually in the presence of a base.

If a solvent is used in the reaction, the solvent includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene and the like, esters such as ethyl acetate, butyl acetate and the like, nitriles such as acetonitrile, butyronitrile and the like, acid amides such as N,N-dimethylformamide and the like, sulfoxides such as dimethyl sulfoxide and the like, and mixtures thereof.

The base used for the reaction includes carbonates such as sodium carbonate, potassium carbonate and the like, alkali metal hydrides such as sodium hydride, potassium hydride and the like, tertiary amines such as triethylamine, diisopropylethylamine, 1,8-diazabicyclo[5.4.0]undec-7-ene, 1,5-diazabicyclo[4.3.0]non-5-ene and the like, and nitrogen-containing aromatic compounds such as pyridine, 4-dimethylaminopyridine and the like.

In the reaction, 1 to 50 mol of a base and 1 to 5 mol of a compound represented by the formula (14) are usually used per 1 mol of a compound represented by the formula (13).

The reaction temperature is usually in a range of 0 to 100°C, and the reaction time is usually in a range of 0.1 to 24 hours.

After completion of the reaction, a compound represented by the formula (15) can be isolated by, for example, pouring the reaction mixture into water, extracting it with an organic solvent, and then drying and concentrating the organic layer. The isolated compound represented by the formula (15) may be further purified by a technique such as chromatography, recrystallization and the like.

### Step (II-2)

A compound represented by the formula (16) can be prepared by reacting a compound represented by the formula (15) and a compound represented by the formula (16).

The reaction is performed in the presence of a solvent and in the presence of a base.

The solvent used for the reaction includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene and the like, acid amides such as N,N-dimethylformamide, sulfoxides such as dimethyl sulfoxide and the like, water, and mixtures thereof.

The base used for the reaction includes carbonates such as sodium carbonate, potassium carbonate and the like, alkali metal hydrides such as sodium hydride, potassium hydride and the like, and metal alkoxides such as sodium methoxide, sodium ethoxide, potassium tertiary butoxide and the like.

In the reaction, 1 to 50 mol of a base and 1 to 5 mol of a compound represented by the formula (16) are usually used per 1 mol of a compound represented by the formula (15).

The reaction temperature is usually in a range of -20 to 100°C, and the reaction time is usually in a range of 0.1 to 24 hours.

After completion of the reaction, a compound represented by the formula (17) can be isolated by, for example, pouring the reaction mixture into water, extracting it with an organic solvent, and then drying and concentrating the organic layer. The isolated compound represented by the formula (17) may be further purified by a technique such as chromatography, recrystallization and the like.

### Step (II-3)

A compound represented by the formula (18) can be prepared by reacting a compound represented by the formula (17) and hydrogen in the presence of a hydrogenation catalyst.

The reaction is performed under a hydrogen atmosphere and usually in the presence of a solvent.

The solvent used for the reaction includes, for example, alcohols such as methanol, ethanol, propanol and the like, esters such as ethyl acetate, butyl acetate and the like, ethers such as tetrahydrofuran, 1,4-dioxane and the like, and mixtures thereof.

The hydrogenation catalyst used for the reaction includes transition metal compounds such as palladium carbon, palladium hydroxide, Raney nickel, platinum oxide and the like.

In the reaction, 0.001 to 0.5 mol of a hydrogenation catalyst is usually used per 1 mol of a compound represented by the formula (17). In the reaction, an acid (hydrochloric acid etc.) may be also added if necessary. In this case, 0.01 mol to an excessive amount of an acid is usually used per 1 mol of a compound represented by the formula (17).

The reaction is performed usually under a hydrogen atmosphere at 1 to 100 atm. The reaction temperature is usually in a range of -20 to 100°C, and the reaction time is usually in a range of 0.1 to 24 hours.

After completion of the reaction, a compound represented by the formula (18) can be isolated by, for example, filtering the reaction mixture, extracting the filtrate with an organic solvent, and then drying and concentrating the resulting organic layer. The isolated compound represented by the formula (18) may be further purified by a technique such as chromatography, recrystallization and the like.

### Step (II-4)

A compound represented by the formula (20) can be prepared by reacting a compound represented by the formula (18) and a compound represented by the formula (19).

The reaction is performed usually in the presence of a base.

If a solvent is used in the reaction, the solvent includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene and the like, esters such as ethyl acetate, butyl acetate and the like, nitriles such as acetonitrile, butyronitrile and the like, acid amides such as N,N-dimethylformamide, sulfoxides such as dimethyl sulfoxide and the like, and mixtures thereof.

The base used for the reaction includes, for example, carbonates such as sodium carbonate, potassium carbonate and the like, tertiary amines such as triethylamine, diisopropylethylamine, 1,8-diazabicyclo[5.4.0]undec-7-ene, 1,5-diazabicyclo[4.3.0]non-5-ene and the like, and nitrogen-containing aromatic compounds such as pyridine, 4-dimethylaminopyridine and the like.

The amounts of reagents used for the reaction are usually 1 to 50 mol of a base and 1 to 5 mol of a compound represented by the formula (19) per 1 mol of a compound represented by the formula (18).

The reaction temperature is usually in a range of 0 to 100°C, and the reaction time is usually in a range of 0.1 to 24 hours.

After completion of the reaction, a compound represented by the formula (20) can be isolated by, for example, filtering the reaction mixture, after adding an organic solvent if necessary, and then concentrating the filtrate. The isolated compound represented by the formula (20) may be further purified by a technique such as distillation, chromatography, recrystallization or the like.

### Step (II-5)

A compound represented by the formula (6-2) can be prepared by reacting a compound represented by the formula (20) and water in the presence of a base.

The reaction is performed usually in the presence of an organic solvent.

The organic solvent used for the reaction includes, for example, ethers such as 1,4-dioxane, tetrahydrofuran, ethylene glycol dimethyl ether, tert-butyl methyl ether and the like, aromatic hydrocarbons such as toluene, xylene and the like, halogenated hydrocarbons such as chlorobenzene and the like, nitriles such as acetonitrile, butyronitrile and the like, alcohols such as methanol, ethanol, propanol and the like, and mixtures thereof.

The base used for the reaction includes alkali metal hydroxides such as lithium hydroxide, sodium hydroxide, potassium hydroxide and the like.

In the reaction, 1 to 10 mol of a base and 1 mol to an excessive amount of water are usually used per 1 mol of a compound represented by the formula (20).

The reaction temperature is usually in a range of 0 to 150°C, and the reaction time is usually in a range of 0.1 to 24 hours.

After completion of the reaction, a compound represented by the formula (6-2) can be isolated by, for example, adding acidic water (hydrochloric acid etc.) to the reaction mixture, extracting it with an organic solvent, and then drying and concentrating the organic layer. The isolated compound represented by the formula (6-2) may be further purified by chromatography, recrystallization or the like, or may be used as it is in a next step.

### Step (II-6)

A compound represented by the formula (3-2) can be prepared by reacting a compound represented by the formula (6-2) and a chlorinating agent.

If a solvent is used in the reaction, the solvent includes, for example, ethers such as 1,4-dioxane, tetrahydrofuran, ethylene glycol dimethyl ether, tert-butyl methyl ether and the like, aliphatic hydrocarbons such as hexane, heptane and the like, aromatic hydrocarbons such as toluene, xylene and the like, halogenated hydrocarbons such as chlorobenzene and the like, and mixtures thereof.

The chlorinating agent used for the reaction includes, for example, thionyl chloride, oxalyl chloride and phosphorus oxychloride.

In the reaction, 1 to 100 mol of a chlorinating agent is usually used per 1 mol of a compound represented by the formula (6-2).

The reaction temperature is usually in a range of 30 to 150°C, and the reaction time is usually in a range of 0.1 to 24 hours.

After completion of the reaction, a compound represented by the formula (3-2) can be isolated by, for example, concentrating the reaction mixture as it is. The isolated compound represented by the formula (3-2) is usually used in a next step without purification.

A compound represented by the formula (6-3), that is, the formula (6) in which R⁸ is a hydrogen atom, and a compound represented by the formula (3-3), that is, the formula (3) in which R⁸ is a hydrogen atom can be also prepared, for example, according to the following scheme: 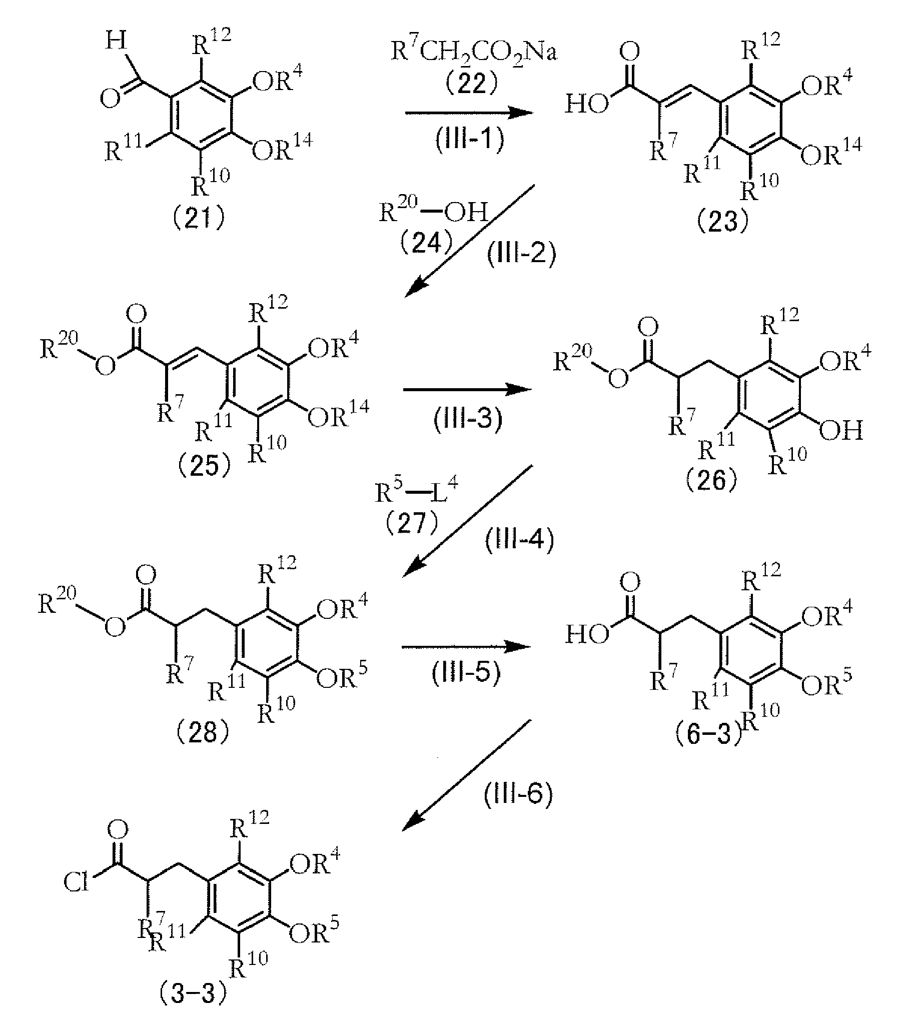 wherein R⁴, R⁵, R⁷, R²⁰, R¹⁴, L⁴, R¹⁰, R¹¹ and R¹² are as defined above.

### Step (III-1)

A compound represented by the formula (23) can be prepared by reacting a compound represented by the formula (21) and a compound represented by the formula (22).

The reaction is performed in the presence of an acid anhydride.

The acid anhydride used for the reaction includes acid anhydride represented by

(R⁷CH₂CO)₂O

wherein R⁷ is as defined above (e.g. acetic anhydride, propionic anhydride, butyric anhydride etc.).

In the reaction, 1 to 5 mol of acid anhydride and 1 to 5 mol of a compound represented by the formula (22) are usually used per 1 mol of a compound represented by the formula (21).

The reaction temperature is usually in a range of 100 to 200°C, and the reaction time is usually in a range of 1 to 24 hours.

After completion of the reaction, a compound represented by the formula (23) can be isolated by, for example, pouring the reaction mixture into water, extracting it with an organic solvent, and then drying and concentrating the organic layer. The isolated compound represented by the formula (23) may be further purified by a technique such as chromatography, recrystallization and the like.

### Step (III-2)

A compound represented by the formula (25) can be prepared by reacting a compound represented by the formula (23) and a compound represented by the formula (24).

The reaction is performed usually in the presence of an acid catalyst.

The acid used for the reaction includes hydrogen halides such as hydrogen chloride, hydrogen bromide and the like, mineral acids such as sulfuric acid and the like, and sulfonic acids such as methanesulfonic acid, paratoluenesulfonic acid and the like.

In the reaction, 3 mol to an excessive amount of a compound represented by the formula (24) and 0.001 to 0.2 mol of an acid are usually used per 1 mol of a compound represented by the formula (23).

The reaction temperature is usually in a range of 50 to 150°C, and the reaction time is usually in a range of 1 to 24 hours.

After completion of the reaction, a compound represented by the formula (25) can be isolated by, for example, pouring the reaction mixture into water, extracting it with an organic solvent, and then drying and concentrating the organic layer. The isolated compound represented by the formula (25) may be further purified by a technique such as chromatography, recrystallization and the like. Alternatively, the reaction mixture is concentrated under reduced pressure to obtain a crude product, which may be used as it is in a next step.

In addition, in the reaction, acid chloride (acetyl chloride etc.) may be used in place of an acid catalyst.

### Step (III-3)

A compound represented by the formula (26) can be prepared by reacting a compound represented by the formula (25) and hydrogen in the presence of a hydrogenation catalyst.

The reaction is performed under a hydrogen atmosphere and usually in the presence of a solvent.

The solvent used for the reaction includes, for example, alcohols such as methanol, ethanol, propanol and the like, esters such as ethyl acetate, butyl acetate and the like, ethers such as tetrahydrofuran, 1,4-dioxane and the like, and mixtures thereof.

The hydrogenation catalyst used for the reaction includes transition metal compounds such as palladium carbon, palladium hydroxide, Raney nickel, platinum oxide and the like.

In the reaction, 0.001 to 0.5 mol of a hydrogenation catalyst is usually used per 1 mol of a compound represented by the formula (25). In the reaction, an acid (hydrochloric acid etc.) may further be added if necessary. In this case, 0.01 mol to an excessive amount of an acid is usually used per 1 mol of a compound represented by the formula (25).

The reaction is performed usually under a hydrogen atmosphere at 1 to 100 atm. The reaction temperature is usually in a range of -20 to 100°C, and the reaction time is usually in a range of 0.1 to 24 hours.

After completion of the reaction, a compound represented by the formula (26) can be isolated by, for example, filtrating the reaction mixture, extracting the filtrate with an organic solvent, and then drying and concentrating the resulting organic layer. The isolated compound represented by the formula (26) may be further purified by a technique such as chromatography, recrystallization and the like.

### Step (III-4)

A compound represented by the formula (28) can be prepared by reacting a compound represented by the formula (26) and a compound represented by the formula (27).

The reaction is performed usually in the presence of a base.

If a solvent is used in the reaction, the solvent includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene and the like, esters such as ethyl acetate, butyl acetate and the like, nitriles such as acetonitrile, butyronitrile and the like, acid amides such as N,N-dimethylformamide and the like, sulfoxides such as dimethyl sulfoxide and the like, and mixtures thereof.

The base used for the reaction includes, for example, carbonates such as sodium carbonate, potassium carbonate and the like, tertiary amines such as triethylamine, diisopropylethylamine, 1,8-diazabicyclo[5.4.0]undec-7-ene, 1,5-diazabicyclo[4.3.0]non-5-ene and the like, and nitrogen-containing aromatic compounds such as pyridine, 4-dimethylaminopyridine and the like.

In the reaction, 1 to 50 mol of a base and 1 to 5 mol of a compound represented by the formula (27) are usually used per 1 mol of a compound represented by the formula (26). The reaction temperature is usually in a range of 0 to 100°C, and the reaction time is usually in a range of 0.1 to 24 hours.

After completion of the reaction, a compound represented by the formula (28) can be isolated by, for example, filtering the reaction mixture, after adding an organic solvent if necessary, and then concentrating the filtrate. The isolated compound represented by the formula (28) may be further purified by a technique such as distillation, chromatography, recrystallization and the like.

### Step (III-5)

A compound represented by the formula (6-3) can be prepared by reacting a compound represented by the formula (28) and water in the presence of a base.

The reaction is performed usually in the presence of an organic solvent.

The organic solvent used for the reaction includes, for example, ethers such as 1,4-dioxane, tetrahydrofuran, ethylene glycol dimethyl ether, tert-butyl methyl ether and the like, aromatic hydrocarbons such as toluene, xylene and the like, halogenated hydrocarbons such as chlorobenzene and the like, nitriles such as acetonitrile, butyronitrile and the like, alcohols such as methanol, ethanol, propanol and the like, and mixtures thereof.

The base used for the reaction includes, for example, alkali metal hydroxides such as lithium hydroxide, sodium hydroxide, potassium hydroxide and the like.

In the reaction, 1 to 10 mol of a base is used usually per 1 mol of a compound represented by the formula (28).

The reaction temperature is usually in a range of 0 to 150°C, and the reaction time is usually in a range of 0.1 to 24 hours.

After completion of the reaction, a compound represented by the formula (6-3) can be isolated by, for example, adding acidic water (hydrochloric acid etc.) to the reaction mixture, extracting it with an organic solvent, and then drying and concentrating the organic layer. The isolated compound represented by the formula (6-3) may be further purified by chromatography, recrystallization or the like, or may be used as it is in a next step.

### Step (111-6)

A compound represented by the formula (3-3) can be prepared by reacting a compound represented by the formula (6-3) and a chlorinating agent.

If a solvent is used in the reaction, the solvent includes, for example, ethers such as 1,4-dioxane, tetrahydrofuran, ethylene glycol dimethyl ether, tert-butyl methyl ether and the like, aliphatic hydrocarbons such as hexane, heptane and the like, aromatic hydrocarbons such as toluene, xylene and the like, halogenated hydrocarbons such as chlorobenzene and the like, and mixtures thereof.

The chlorinating agent used for the reaction includes thionyl chloride, oxalyl chloride and phosphorus oxychloride.

In the reaction, 1 to 100 mol of a chlorinating agent is used usually per 1 mol of a compound represented by the formula (6-3).

The reaction temperature is usually in a range of 30 to 150°C, and the reaction time is usually in a range of 0.1 to 24 hours.

After completion of the reaction, a compound represented by the formula (3-3) can be isolated by concentrating the reaction mixture as it is. The isolated compound represented by the formula (3-3) is usually used in a next step without purification.

A compound represented by the formula (3) can be prepared by, for example, reacting a compound represented by the formula (30) with cyanide, an ammonium salt and a compound represented by the formula (31): 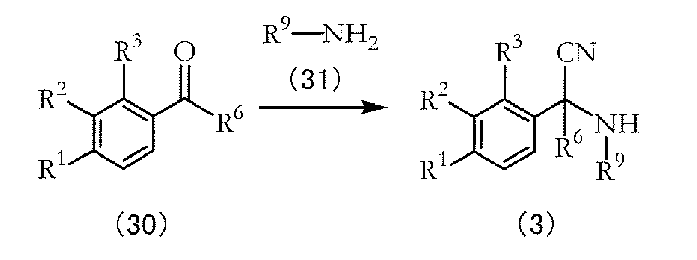 wherein R¹, R², R³, R⁶ and R⁹ are as defined above.

The reaction is performed usually in the presence of a solvent.

The solvent used for the reaction includes, for example, alcohols such as methanol, ethanol, isopropanol and the like, water, and mixtures thereof.

The cyanide used for the reaction includes sodium cyanide and potassium cyanide.

The ammonium salt used for the reaction includes ammonium chloride and ammonium bromide.

In the reaction, 1 to 5 mol of cyanide, 1 to 5 mol of an ammonium salt, and 1 to 50 mol of a compound represented by the formula (31) are usually used per 1 mol of a compound represented by the formula (30).

The reaction temperature is usually in a range of -10 to 100°C, and the reaction time is usually in a range of 1 to 50 hours.

After completion of the reaction, a compound represented by the formula (3) can be isolated by, for example, adding an organic solvent to the reaction mixture if necessary, extracting it with an organic solvent, and then concentrating the organic layer, or may be obtained as hydrochloride by mixing the reaction mixture with hydrochloric acid.

In addition, a compound represented by the formula (3) is the compound described, for example, in Tetrahedron Letters, vol.25, No.41, pp.4583-4586, 1984 or US Patent No. 4041045, or can be prepared according to a method similar to that described in the literatures.

Plant diseases against which the compound of the present invention has controlling effect include, for example, diseases due to Phycomycetes, and specifically following diseases are included: *Peronospora brassicae* of vegetables and radishes; *Peronospora spinaciae* of spinach; *Peronospora* tabacina of tobacco; *Pseudoperonospora cubensis* of gourds; *Plasmopara* viticola of grapes; *Phytophthora cactorum* of apple, strawberry and ginseng; *Phytophora capsici* of tomato and cucumber; *Phytophthora cinnamomi* of pineapple; *Phytophthora infestans* of potato and tomato; *Phytophthora* nicotianae *var. nicotianae* of tobacco, broad bean and leek; *Pythium sp*. of spinach; *Pythium aphanidermatum* of cucumber; *Pythium sp.* of wheat; *Pythium debaryanum* of tobaco; and *Pythium* rot of soybean *(Pythium aphanidermatum, P. debaryanum, P. irregulare, P. myriotylum, P. ultimum).*

The plant disease-controlling composition of the present invention contains the compound of the present invention and an inert carrier. The plant disease-controlling composition of the present invention is a formulation such as emulsifiable concentrate, wettable powder, dry flowable, flowable, dust, granule and the like obtained by mixing the compound of the present invention, an inert carrier (solid carrier or liquid carrier) and, if necessary, a surfactant and/or other adjuvants for formulation. These formulations contain usually 0.1 to 90 % by weight of the compound of the present invention.

Solid carriers used for formulation include, for example, fine powders or granules of minerals such as kaolin clay, attapulgite clay, bentonite, montmorillonite, terra alba, pyrophyllite, talc, diatomaceous earth, calcite and the like; natural organic substances such as corncob powder, walnut shell powder and the like; synthetic organic substances such as urea and the like; salts such as calcium carbonate, ammonium sulfate and the like; synthetic inorganic substances such as synthetic hydrous silicon oxide and the like. Liquid carriers include, for example, aromatic hydrocarbons such as xylene, alkylbenzene, methylnaphthalene and the like; alcohols such as 2-propanol, ethylene glycol, propylene glycol, cellosolve and the like; ketones such as acetone, cyclohexanone, isophorone and the like; vegetable oils such as soybean oil, cottonseed oil and the like; aliphatic hydrocarbons, esters, dimethylsulfoxide, acetonitrile and water.

Surfactants include, for example, anionic surfactants such as alkylsulfuric acid ester salt, alkylarylsulfonic acid salt, dialkylsulfosuccinic acid salt, polyoxyethylenealkylaryletherphosphoric acid ester salt, lignin sulfonic acid salt, polycondensed naphthalenesulfonateformaldehyde and the like; and nonionic surfactants such as polyoxyethylenealkylarylether, polyoxyethylenealkylpolyoxypropylene block copolymer, sorbitan fatty acid ester and the like.

Other adjuvants for formulation include, for example, water-soluble polymers such as polyvinylalcohol, polyvinylpyrrolidone and the like; Arabian gum; alginic acid and its salt; polysaccharides such as CMC(carboxymethylcellulose), xanthan gum and the like; inorganic substances such as alminum magnesium silicate, alumina sol and the like; and preservatives, colorants, PAP(isopropyl acidic phosphate), stabilizing agents such as BHT and the like.

By treating the foliage of plants with the plant diseases-controlling composition of the present invention, said plants can be protected from plant diseases. By treating soil with the plant diseases-controlling composition of the present invention, plants growing on said soil can be protected from plant diseases.

When the plant diseases-controlling composition of the present invention is used for the foliage treatment of plants or the soil treatment, the amount used thereof may vary depending on the kind of a crop which is a plant to be protected, the kind of a disease to be controlled, the infestation level of a disease to be controlled, the formulation type, the treatment timing, weather conditions and the like, and is usually 1 to 5000 g, preferably 5 to 1000 g of the compound of the present invention per 10000 m².

When an emulsifiable concentrate, wettable powder, flowable or the like is used, it is usually sprayed after diluted with water. In this case, the concentration of the compound of the present invention is usually in the range of from 0.0001 to 3% by weight, preferably from 0.0005 to 1% by weight. A dust, granule or the like is usually directly used without dilution.

The plant diseases-controlling composition of the present invention can be also used for the disinfection treatment of seeds and the like. A method for seed disinfection seeds includes, for example, a method comprising soaking seeds of a plant in the plant diseases-controlling composition of the present invention prepared so as to contain 1 to 1,000 ppm of the compound of the present invention, a method comprising spraying on or applying to seeds of a plant the plant diseases-controlling composition of the present invention containing 1 to 1,000 ppm of the compound of the present invention, and a method comprising coating seeds of a plant with the plant diseases-controlling composition of the present invention which is formulated to dust.

A method for controlling a plant disease of the present invention usually comprises treating a plant on which the occurrence of a disease is predicted or a soil where said plant is grown, and/or treating a plant on which the occurrence of a disease has been confirmed or soil where said plant is grown, with an effective amount of the plant disease-controlling composition of the present invention.

The plant diseases-controlling composition of the present invention is usually used for agriculture or gardening, that is, for controlling plant diseases on plowed fields, paddy fields, orchards, tea fields, pastures, lawn and the like.

The plant diseases-controlling composition of the present invention may be used together with other fungicides, insecticides, acaricides, nematicides, herbicides, plant growth regulators and/or fertilizers.

Examples of active ingredients of such other plant diseases-controlling agents include, for example, chlorothalonil; fluazinam; dichlofluanid; fosetyl-Al; cyclic imide derivatives such as captan, captafol, folpet and the like; dithiocarbamate derivatives such as maneb, mancozeb, thiuram, ziram, zineb, propineb and the like; inorganic or organic copper derivatives such as basic copper sulfate, basic copper chloride, copper hydroxide, copper-oxinate and the like; acylalanine derivatives such as metalaxyl, furalaxyl, ofurace, cyprofuram, benalaxyl, oxadixyl and the like; strobilurine like compound such as kresoxim-methyl, azoxystrobin, trifloxystrobin, picoxystrobin, pyraclostrobin, dimoxystrobin and the like; anilinopyrimidine derivatives such as cyprodinil, pyrimethanil, mepanipyrim and the like; phenyl pyrrole derivatives such as fenpiclonil, fludioxonil and the like; imide derivatives such as procymidone, iprodione, vinclozolin and the like; benzimidazole derivatives such as carbendazim, benomyl, thiabendazole, thiophanate methyl and the like; amine derivatives such as fenpropimorph, tridemorph, fenpropidin, spiroxamine and the like; azole derivatives such as propiconazole, triadimenol, prochloraz, penconazole, tebuconazole, flusilazole, diniconazole, bromuconazole, epoxiconazole, difenoconazole, cyproconazole, metconazole, triflumizole, tetraconazole, myclobutanil, fenbuconazole, hexaconazole, fluquinconazole, triticonazole, bitertanol, imazalil, flutriafol and the like; cymoxanil; dimethomorph; famoxyadone, fenamidone; iprovalicarb; benthiavalicarb; cyazofamid, zoxamide, ethaboxam; boscalid; fenhexamid; quinoxyfen; proquinazid; diethofencarb; and acibenzolar S-methyl; guazatine; and penthiopyrad.

Specific examples of the compound of the present invention are described below. N-(α-cyanobenzyl)amide compounds represented by the formulas (i) to (LXXii):

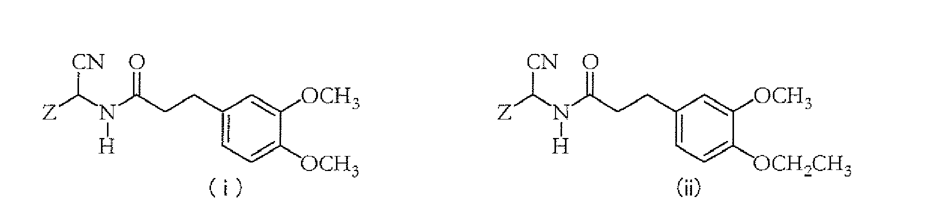

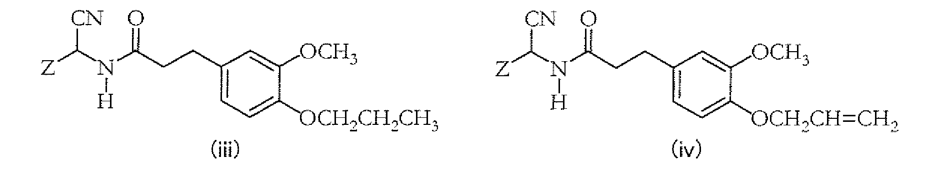

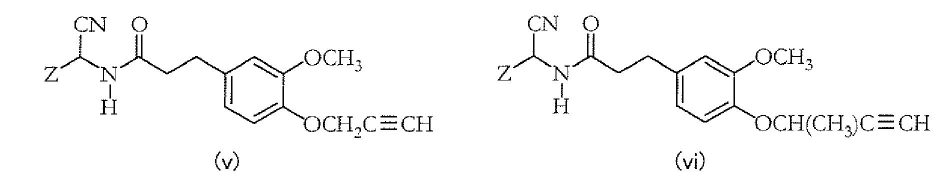

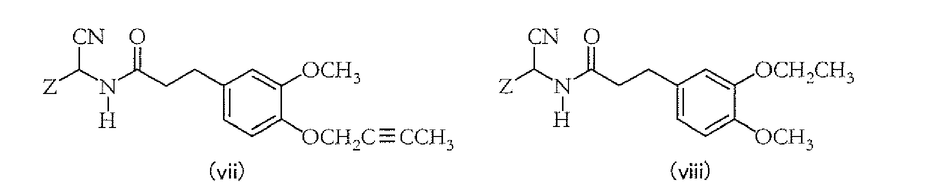

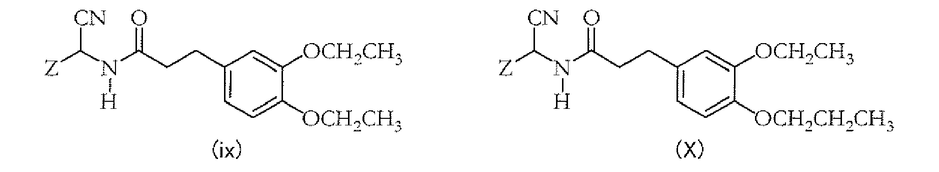

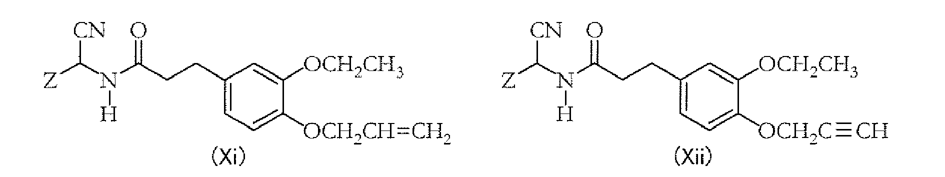

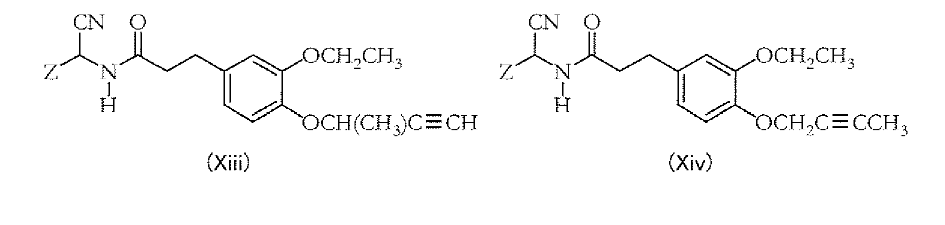

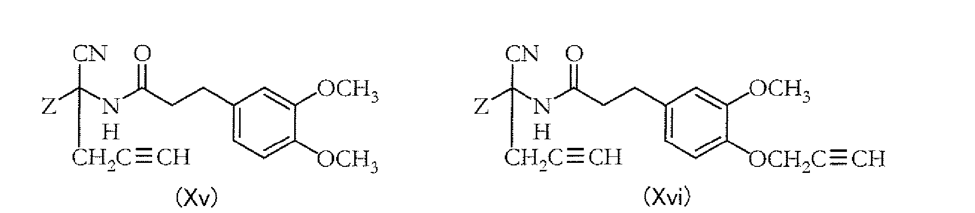

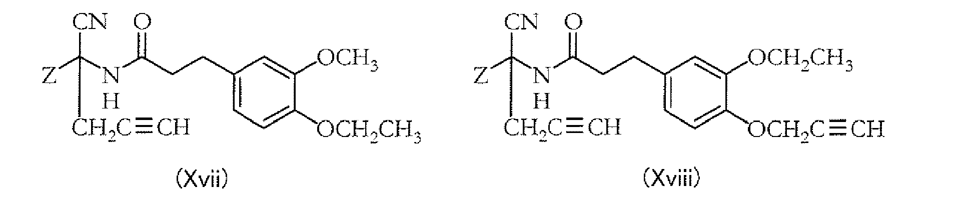

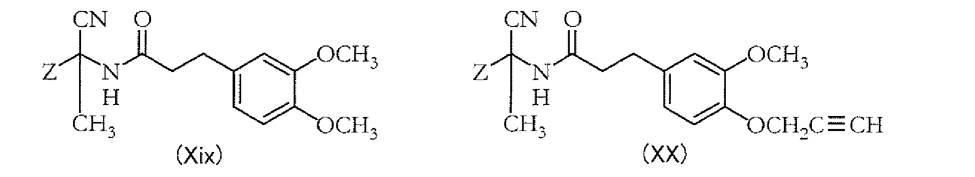

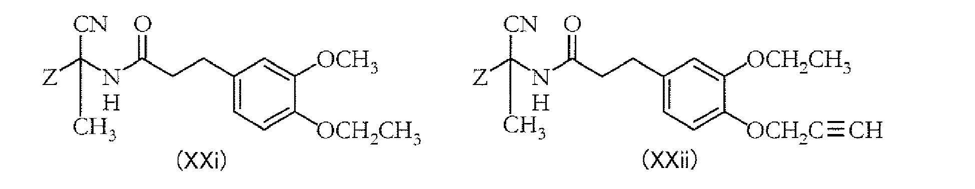

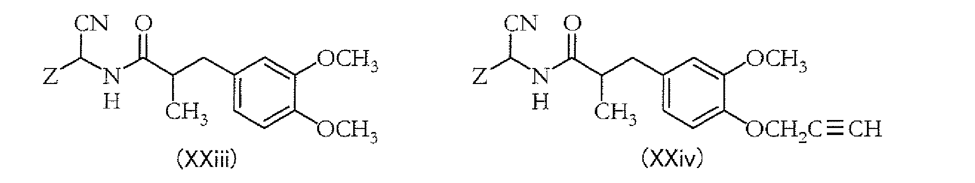

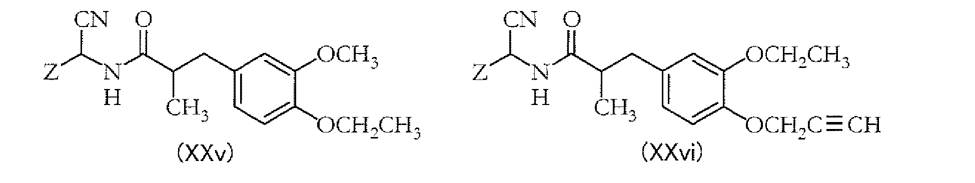

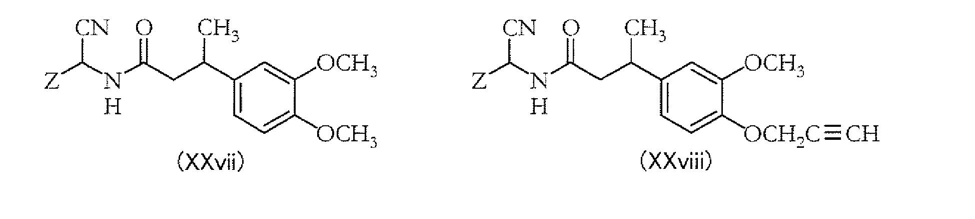

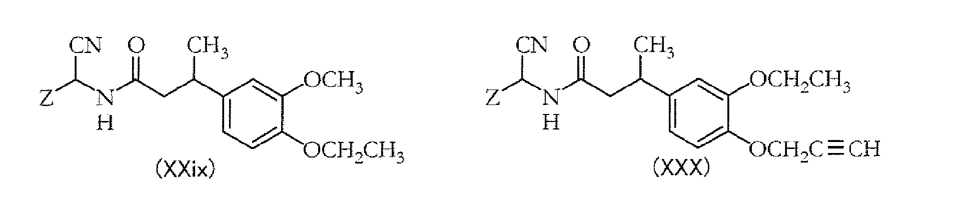

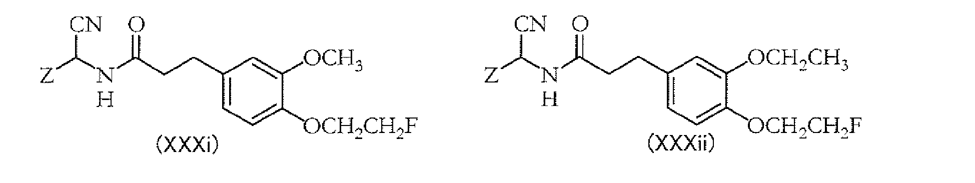

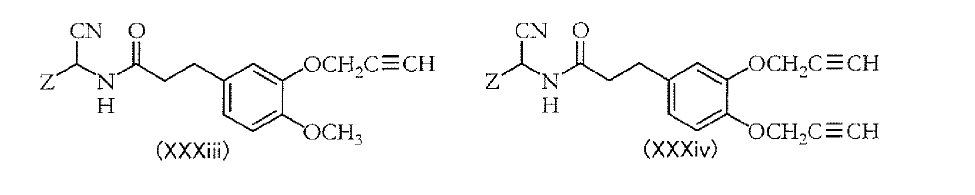

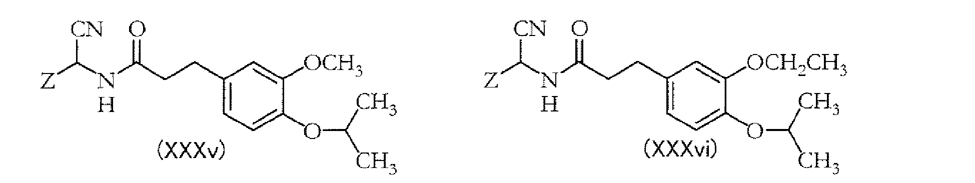

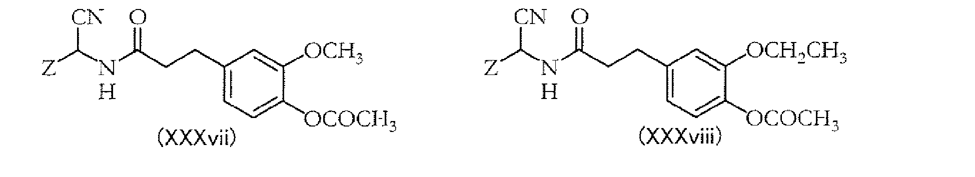

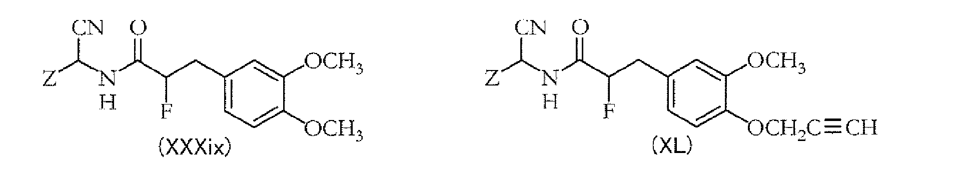

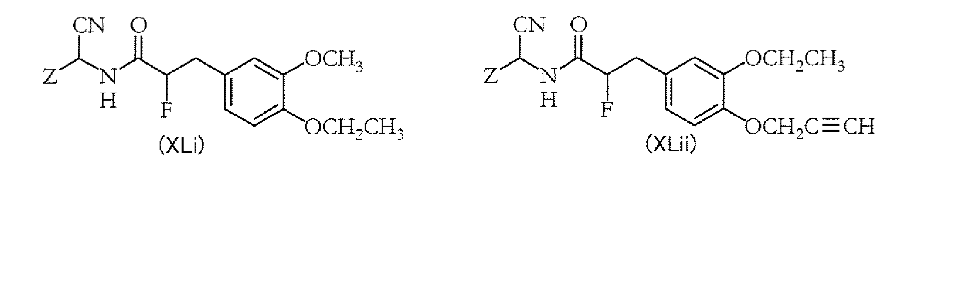

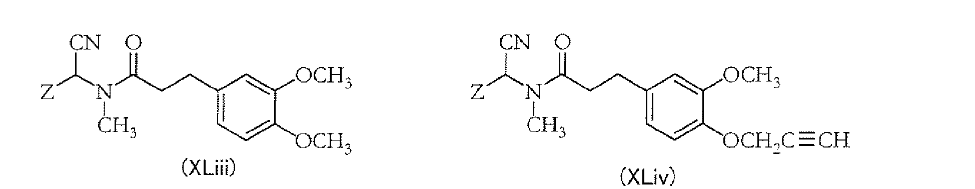

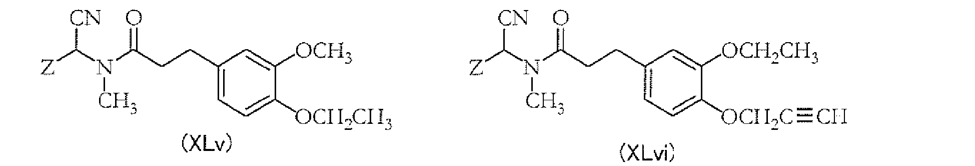

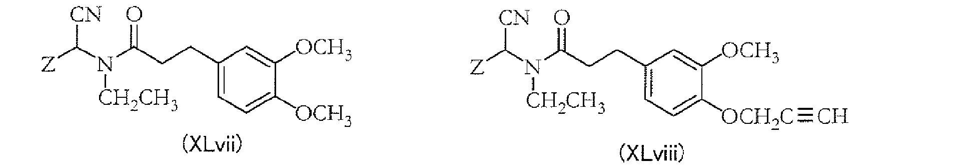

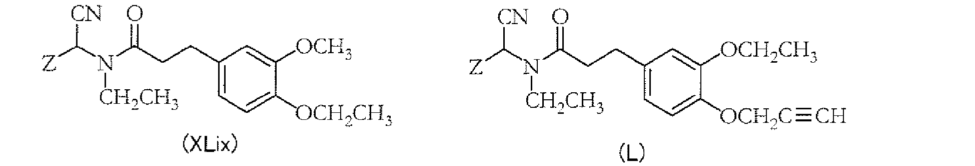

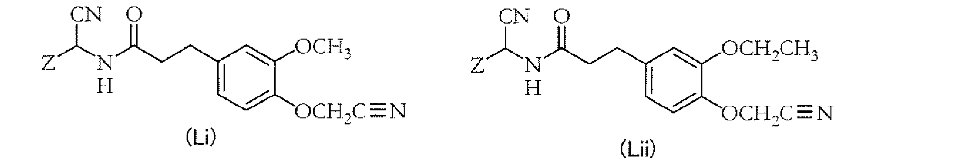

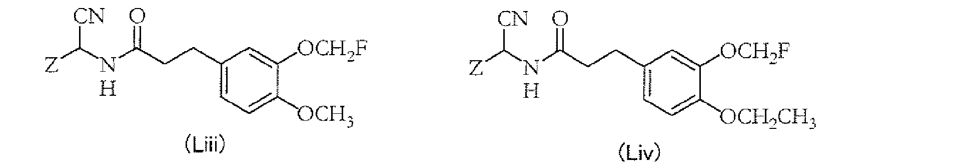

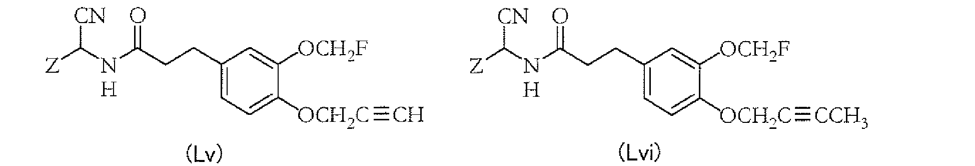

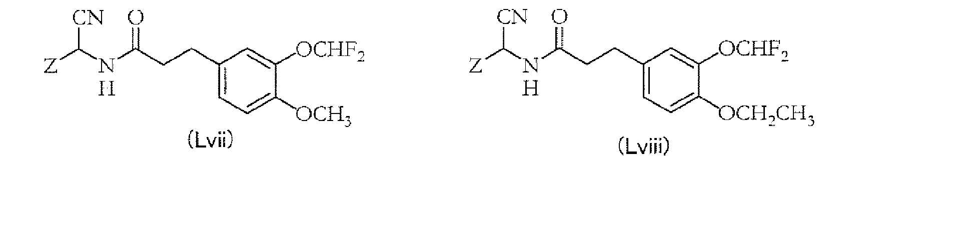

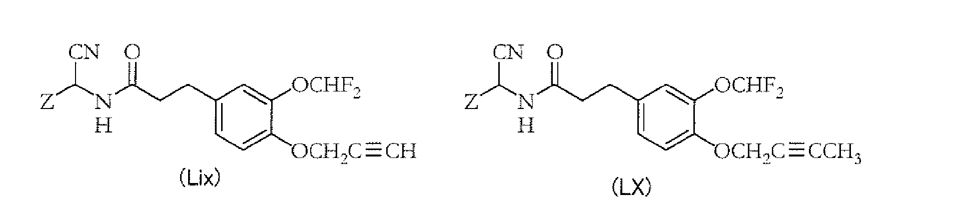

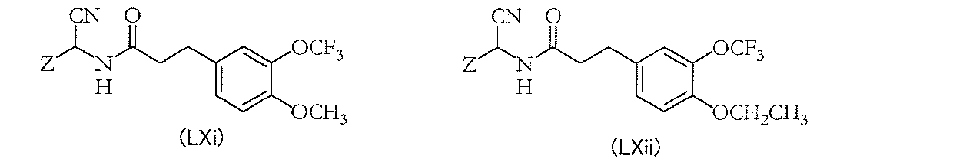

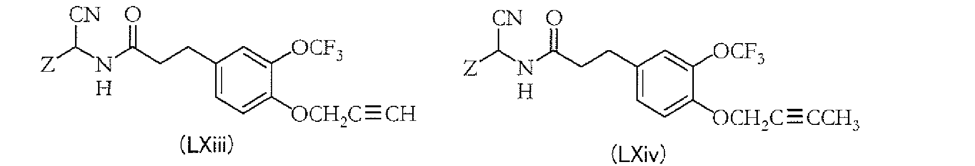

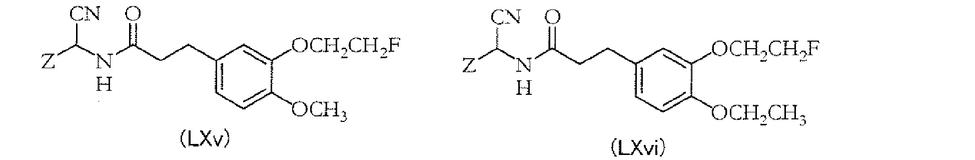

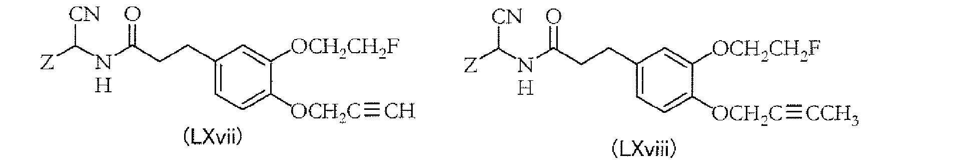

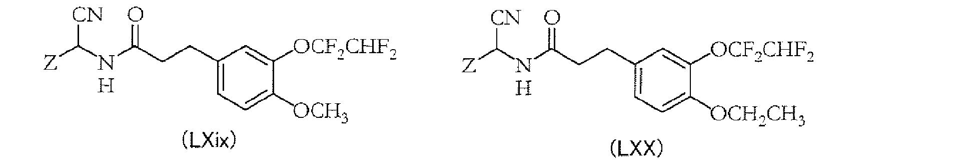

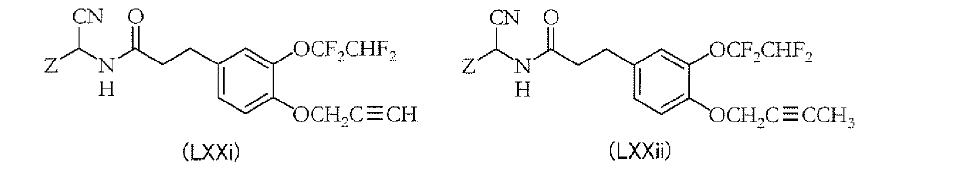

In the formulas (i) to (LXXii), Z represents any one of the following groups:
phenyl group, 4-fluorophenyl group, 3-fluorophenyl group, 2-fluorophenyl group, 4-chlorophenyl group, 3-chlorophenyl group, 2-chlorophenyl group, 4-bromophenyl group, 3-bromophenyl group, 2-bromophenyl group, 4-iodophenyl group, 3-iodophenyl group, 2-iodophenyl group, 4-methylphenyl group, 3-methylphenyl group, 2-methylphenyl group, 4-ethylphenyl group, 3-ethylphenyl group, 2-ethylphenyl group, 4-propylphenyl group, 3-propylphenyl group, 2-propylphenyl group, 4-isopropylphenyl group, 4-butylphenyl group, 3-butylphenyl group, 4-(sec-butyl)phenyl group, 4-isobutylphenyl group, 4-(tert-butyl)phenyl group, 4-cyclopropylphenyl group, 4-cyclobutylphenyl group, 4-cyclopentyl group, 4-cyclohexylphenyl group, 3-fluoro-4-cyclopropylphenyl group, 3-fluoro-4-cylcobutylphenyl group, 3-fluoro-4-cyclopentylphenyl group, 3-fluoro-4-cyclohexylphenyl group, 4-(fluoromethyl)phenyl group, 4-(difluoromethyl)phenyl group, 4-(trifluoromethyl)phenyl group, 4-methoxyphenyl group, 4-ethoxyphenyl group, 3,4-difluorophenyl group, 2,4-difluorophenyl group, 4-chloro-3-fluorophenyl group, 4-chloro-2-fluorophenyl group, 4-bromo-3-fluorophenyl group, 4-bromo-2-fluorophenyl group, 3-fluoro-4-methylphenyl group, 2-fluoro-4-methylphenyl group, 4-ethyl-3-flurophenyl group, 4-ethyl-2-fluorophenyl group, 3-fluoro-4-(trifluoromethyl)phenyl group, 2-fluoro-4-(trifluoromethyl)phenyl group, 3-fluoro-4-methoxyphenyl group, 2-fluoro-4-methoxyphenyl group, 4-fluoro-3-chlorophenyl group, 4-fluoro-2-chlorophenyl group, 3,4-dichlorophenyl group, 2,4-dichlorophenyl group, 4-bromo-3-chlorophenyl group, 4-bromo-2-chlorophenyl group, 3-chloro-4-methylphenyl group, 2-chloro-4-methylphenyl group, 3-chloro-4-ethylphenyl group, 2-chloro-4-ethylphenyl group, 3-chloro-4-(trifluoromethyl)phenyl group, 2-chloro-4-(trifluoromethyl)phenyl group, 3-chloro-4-methoxyphenyl group, 2-chloro-4-methoxyphenyl group, 3-bromo-4-fluorophenyl group, 2-bromo-4-fluorophenyl group, 3-bromo-4-chlorophenyl group, 2-bromo-4-chlorophenyl group, 3,4-dibromophenyl group, 2,4-dibromophenyl group, 3-bromo-4-methylphenyl group, 2-bromo-4-methylphenyl group, 3-bromo-4-ethylphenyl group, 2-bromo-4-ethylphenyl group, 3-bromo-4-(trifluoromethyl)phenyl group, 2-bromo-4-(trifluoromethyl)phenyl group, 3-bromo-4-methoxyphenyl group, 2-bromo-4-methoxyphenyl group, 4-fluoro-3-methylphenyl group, 4-fluoro-2-methylphenyl group, 4-chloro-3-methylphenyl group, 4-chloro-2-methylphenyl group, 4-bromo-3-methylphenyl group, 4-bromo-2-methylphenyl group, 3,4-dimethylphenyl group, 2,4-dimethylphenyl group, 4-ethyl-3-methylphenyl group, 4-ethyl-2-methylphenyl group, 3-methyl-4-(trifluoromethyl)phenyl group, 2-methyl-4-(trifluoromethyl)phenyl group, 4-methoxy-3-methylphenyl group, 4-methoxy-2-methylphenyl group, 3-ethyl-4-fluorophenyl group, 2-ethyl-4-fluorophenyl group, 4-chloro-3-ethylphenyl group, 4-chloro-2-ethylphenyl group, 4-bromo-3-ethylphenyl group, 4-bromo-2-ethylphenyl group, 3-ethyl-4-methylphenyl group, 2-ethyl-4-methylphenyl group, 3,4-diethylphenyl group, 2,4-diethylphenyl group, 3-ethyl-4-(trifluoromethyl)phenyl group, 2-ethyl-4-(trifluoromethyl)phenyl group, 3-ethyl-4-methoxyphenyl group, 2-ethyl-4-methoxyphenyl group, 2,3-difluorophenyl group, 2,3-dichlorophenyl group, 2,3-dibromophenyl group, 2,3-dimethylphenyl group, 2,3-diethylphenyl group, 4-cyanophenyl group, 4-cyano-3-methylphenyl group, 4-cyano-3-ethylphenyl group, 4-cyano-3-fluorophenyl group, 4-cyano-3-chlorophenyl group, 4-cyano-3-bromophenyl group, 4-trifluoromethoxyphenyl group, 3-methyl-4-trifluoromethoxyphenyl group, 3-ethyl-4-trifluoromethoxyphenyl group, 3-fluoro-4-trifluoromethoxyphenyl group, 3-chloro-4-trifluoromethoxyphenyl group, 3-bromo-4-trifluoromethoxyphenyl group, 4-difluoromethoxyphenyl group, 3-methyl-4-difluoromethoxyphenyl group, 3-ethyl-4-difluoromethoxyphenyl group, 3-fluoro-4-difluoromethoxyphenyl group, 3-chloro-4-difluoromethoxyphenyl group, 3-bromo-4-difluoromethoxyphenyl group, 4-fluoromethoxyphenyl group, 3-methyl-4-fluoromethoxyphenyl group, 3-ethyl-4-fluoromethoxyphenyl group, 3-fluoro-4-fluoromethoxyphenyl group, 3-chloro-4-fluoromethoxyphenyl group, 3-bromo-4-fluoromethoxyphenyl group, 4-(2,2,2-trifluoroethoxy)phenyl group, 4-(1,1,2,2-tetrafluoroethoxy)phenyl group, 4-(2-fluoroethoxy)phenyl group, 4-methylthiophenyl group, 3-methyl-4-methylthiophenyl group, 2-ethyl-4-methylthiophenyl group, 3-fluoro-4-methylthiophenyl group, 3-chloro-4-methylthiophenyl group, 3-bromo-4-methylthiophenyl group, 4-(fluoromethylthio)phenyl group, 4-(difluoromethylthio)phenyl group, 4-(trifluoromethylthio)phenyl group, 4-(2,2,2-trifluoroethylthio)phenyl group, 4-(1,1,2,2-tetrafluoroethylthio)phenyl group, 4-(2-fluoroethylthio)phenyl group, 4-dimethylaminophenyl group, 3-methyl-4-dimethylaminophenyl group, 3-ethyl-4-dimethylaminophenyl group, 3-fluoro-4-dimethylaminophenyl group, 3-chloro-4-dimethylaminophenyl group, 3-bromo-4-dimethylaminophenyl group, 4-phenoxyphenyl group, 3-methyl-4-phenoxyphenyl group, 3-ethyl-4-phenoxyphenyl group, 3-fluoro-4-phenoxyphenyl group, 3-chloro-4-phenoxyphenyl group, 3-bromo-4-phenoxyphenyl group, 4-phenylphenyl group, 4-(2-fluoraphenyl)phenyl group, 4-(3-fluorophenyl)phenyl group, 4-(4-fluorophenyl)phenyl group, 4-(3,4-diflucrophenyl)phenyl group, 4-(2-chlorophenyl)phenyl group, 4-(3-chlorophenyl)phenyl group, 4-(4-chlorophenyl)phenyl group, 4-(3,4-dichlorophenyl)phenyl group, 4-(2-bromaphenyl)phenyl group, 4-(3-bromophenyl)phenyl group, 4-(4-bromophenyl)phenyl group, 4-(2-methylphenyl)phenyl group, 4-(3-methylphenyl)phenyl group, 4-(4-methylphenyl)phenyl group, 4-(2-ethylphenyl)phenyl group, 4-(3-ethylphenyl)phenyl group, 4-(4-ethylphenyl)phenyl group, 4-(2-trifluoromethylphenyl)phenyl group, 4-(3-trifluoromethylphenyl)phenyl group, 4-(4-trifluoromethylphenyl)phenyl group, 4-(2-methoxyphenyl)phenyl group, 4-(3-methoxyphenyl)phenyl group, 4-(4-methoxyphenyl)phenyl group, 4-phenylthiophenyl group, 4-(2-fluorophenylthio)phenyl group, 4-(3-fluorophenylthio)phenyl group, 4-(4-fluorophenylthio)phenyl group, 4-(3,4-difluorophenylthio)phenyl group, 4-(2-chlorophenylthio)phenyl group, 4-(3-chlorophenylthio)phenyl group, 4-(4-chlorophenylthio)phenyl group, 4-(3,4-dichlorophenylthio)phenyl group, 4-(2-bromophenyltnio)phenyl group, 4-(3-bromophenylthio)phenyl group, 4-(4-bromophenylthio)phenyl group, 4-(2-methylphenylthio)phenyl group, 4-(3-methylphenylthio)phenyl group, 4-(4-methylphenylthio)phenyl group, 4-(2-ethylphenylthio)phenyl group, 4-(3-ethylphenylthio)phenyl group, 4-(4-ethylphenylthio)phenyl group, 4-(2-trifluoromethylphenylthio)phenyl group, 4-(3-trifluoromethylphenylthio)phenyl group, 4-(4-trifluoromethylphenylthio)phenyl group, 4-(2-methoxyphenylthio)phenyl group, 4-(3-methoxyphenylthio)phenyl group, 4-(4-methoxyphenylthio)phenyl group, 4-benzoylphenyl group, 4-(2-fluorobenzoyl)phenyl group, 4-(3-fluorobenzoyl)phenyl group, 4-(4-fluornbenzoyl)phenyl group, 4-(3,4-difluorobenzoyl)phenyl group, 4-(2-chlorobenzoyl)phenyl group, 4-(3-chlorobenzoyl)phenyl group, 4-(4-chlorobenzoyl)phenyl group, 4-(3,4-dichlorobenzoyl)phenyl group, 4-(2-bromobenzoyl)phenyl group, 4-(3-bromobenzoyl)phenyl group, 4-(4-bromobenzoyl)phenyl group, 4-(2-methylbenzoyl)phenyl group, 4-(3-methylbenzoyl)phenyl group, 4-(4-methylbenzoyl)phenyl group, 4-(2-ethylbenzoyl)phenyl group, 4-(3-ethylbenzoyl)phenyl group, 4-(4-ethylbenzoyl)phenyl group, 4-(2-trifluoromethylbenzoyl)phenyl group, 4-(3-trifluoromethylbenzoyl)phenyl group, 4-(4-trifluoromethylbenzoyl)phenyl group, 4-(2-methoxybenzoyl)phenyl group, 4-benzoyloxyphenyl group, 4-(2-fluorobenzoyloxy)phenyl group, 4-(3-fluorobenzoyloxy)phenyl group, 4-(4-fluorobenzoyloxy)phenyl group, 4-(3,4-difluorobenzoyloxy)phenyl group, 4-(2-chlorobenzoyloxy)phenyl group, 4-(3-chlorobenzoyloxy)phenyl group, 4-(4-chlorobenzoyloxy)phenyl group, 4-(3,4-dichlorobenzoyloxy)phenyl group, 4-(2-bromobenzoyloxy)phenyl group, 4-(3-bromobenzoyloxy)phenyl group, 4-(4-bromobenzoyloxy)phenyl group, 4-(2-methylbenzoyloxy)phenyl group, 4-(3-methylbenzoyloxy)phenyl group, 4-(4-methylbenzoyloxy)phenyl group, 4-(2-ethylbenzoyloxy)phenyl group, 4-(3-ethylbenzoyloxy)phenyl group, 4-(4-ethylbenzoyloxy)phenyl group, 4-(2-trifluoromethylbenzoyloxy)phenyl group, 4-(3-trifluoromethylbenzoyloxy)phenyl group, 4-(4-trifluoromethylbenzoyloxy)phenyl group, 4-(2-methoxybenzoyloxy)phenyl group, 4-(3-methoxybenzoyloxy)phenyl group, 4-(4-methoxybenzoyloxy)phenyl group, 3-methyl-4-phenylphenyl group, 3-ethyl-4-phenylphenyl group, 3-fluoro-4-phenylphenyl group, 3-chloro-4-phenylphenyl group, 3-bromo-4-phenylphenyl group, 4-(trimethylsilylethynyl)phenyl group, 4-(triethylsilylethynyl)phenyl group, 4-(hydroxymethyl)phenyl group, 4-(1-hydroxyethyl)phenyl group, 4-(2-hydroxyethyl)phenyl group, 4-(3-hydroxypropyl)phenyl group, 4-(ethoxycyanomethyl)phenyl group, 4-(methoxycyanomethyl)phenyl group, 4-trimethylsilylphenyl group, 4-triethylsilylphenyl group, 4-triisopropylsilylphenyl group, 4-(tert-butyldiemethylsilyl)phenyl group, indan-5-yl group, 1-methyl-indan-5-yl group, 2-methyl-indan-5-yl group, 3-methyl-indan-5-yl group, 1,1-dimethyl-indan-5-yl group, 2,2-dimethyl-indan-5-yl group, 3,3-dimethyl-indan-5-yl group, 1,2,3,4-tetrahydronaphthalen-6-yl group, 1-methyl-1,2,3,4-tetrahydranaphthalen-6-yl group, 2-methyl-1,2,3,4-tetrahydronaphthalen-6-yl group, 3-methyl-1,2,3,4-tetrahydronaphthalen-6-yl group, 4-methyl-1,2,3,4-tetrahydronaphthalen-6-yl group, 2,2-dimethyl-1,2,3,4-tetrahydronaphthalen-6-yl group, 3,3-dimethyl-1,2,3,4-tetrahydronaphthalen-6-yl group, 2,3-dimethyl-1,2,3,4-tetrahydronaphthalen-6-yl group, 2,2,3,3-tetramethyl-1,2,3,4-tetrahydronaphthalen-6-yl group, 6,7,8,9-tetrahydro-5H-benzocyclohepten-2-yl group, 5,6,7,8,9,10-hexahydro-benzocycloocten-2-yl group, 2-naphthyl group, 5-fluoronaphthalen-2-yl group, 6-fluoronaphthalen-2-yl group, 7-fluoronaphthalen-2-yl group, 8-fluoronaphthalen-2-yl group, 5-chloronaphthalen-2-yl group, 6-chloronaphthalen-2-yl group, 7-chloronaphthalen-2-yl group, 8-chloronaphthalen-2-yl group, 5-bromonaphthalen-2-yl group, 6-bromonaphthalen-2-yl group, 7-bromonaphthalen-2-yl group, 8-bromonaphthalen-2-yl group, 5-methylnaphthalen-2-yl group, 6-methylnaphthalen-2-yl group, 7-methylnaphthalen-2-yl group, 8-methylnaphthalen-2-yl group, 5-methoxynaphthalen-2-yl group, 6-methoxynaphthalen-2-yl group, 7-methoxynaphthalen-2-yl group, 8-methoxynaphthalen-2-yl group, 5-trifluoromethylnaphthalen-2-yl group, 6-trifluoromethylnaphthalen-2-yl group, 7-trifluoromethylnaphthalen-2-yl group, 8-trifluoromethylnaphthalen-2-yl group, 5,6-difluoronaphthalen-2-yl group, 5,6-dichloronaphthalen-2-yl group, 5,6-dimethylnaphthalen-2-yl group, 5-fluoro-6-methylnaphthalen-2-yl group, 6-fluoro-5-methylnaphthalen-2-yl group, 5-chloro-6-methylnaphthalen-2-yl group, 6-chloro-5-methylnaphthalen-2-yl group, 6-chloro-5-fluoronaphthalen-2-yl group, and 5-chloro-6-fluoronaphthalen-2-yl group.

Hereinafter, the present invention will be further explained in detail by way of Preparation Examples, Formulation Examples , Test Examples and the like, but the present invention is not limited to these Examples.

First, Preparation Examples of the compound of the present invention will be described.

### Preparation Example 1

8.0 g of 2-amino-2-(4-chlorophenyl)acetonitrile hydrochloride, 17 ml of diisopropylethylamine and 150 ml of tetrahydrofuran were mixed, and thereto a mixture of 8.3 g of 3-{3-methoxy-4-(2-propynyloxy)ptenyl}propionyl chloride and 30 ml of tetrahydrofuran was added at 0 to 5°C. The mixture was stirred at room temperature for 1 hour. After the reaction mixture was concentrated under reduced pressure, water was added and the mixture was extracted with ethyl acetate. The organic layer was washed successively with 5% hydrochloric acid, water, an aqueous saturated sodium hydrogen carbonate solution and an aqueous saturated sodium chloride solution, dried over magnesium sulfate, and then concentrated under reduced pressure. The residue was subjected to silica gel column purification to obtain 8.6 g of N-{1-(4-chlorophenyl)-1-cyanomethyl}-3-{3-methoxy-4-(2-propynyloxy)phenyl}propanamide (hereinafter, referred to as the compound 1 of the present invention). 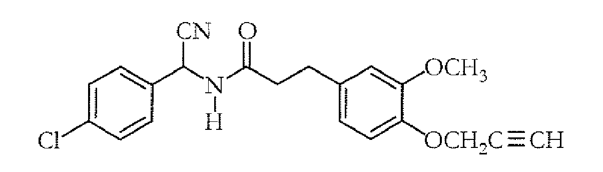 ¹H-NMR (CDCl₃, TMS) δ (ppm): 7.35-7.38 (2H, m), 7.22-7.24 (2H, m), 6.94 (1H, d, J = 7.8 Hz), 6.69-6.72 (2H, m), 6.08 (1H, d, J = 8.5 Hz), 5.87 (1H, br.d, J = 8.5 Hz), 4.73 (2H, d, J = 2.4 Hz), 3.82 (3H, s), 2.95 (2H, t, J = 7.3 Hz), 2.48-2.63 (3H, m)

### Preparation Example 2

2 g of N-{1-(4-chlorophenyl)-1-cyanomethyl}-3-{3-methoxy-4-(2-propynyloxy)phenyl}propanamide, 2 g of potassium carbonate, 1 ml of propargyl bromide and 30 ml of acetonitrile were mixed and stirred at 80°C for 30 minutes. The reaction mixture was allowed to cool to around room temperature and then filtered. The resulting filtrate was concentrated under reduced pressure. The residue was subjected to silica gel column purification to obtain 0.6 g of N-{1-(1-(4-chlorophenyl)-1-cyano-3-butynyl)-3-{3-methoxy-4-(2-propynyloxy)phenyl}propanamide (hereinafter, referred to as the compound 2 of the present invention). 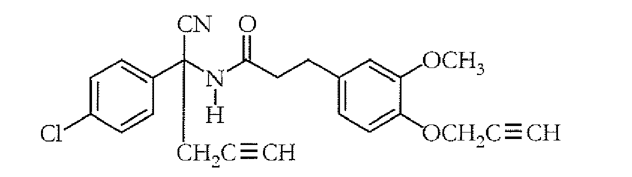 ¹H-NMR (CDCl₃, TMS) δ (ppm): 7.33-7.35 (2H, m), 7.27-7.29 (2H, m), 6.99 (1H, d, J = 8.3 Hz), 6.72-6.75 (2H, m), 6.03 (1H, s), 4.76 (2H, d, J = 2.4 Hz), 3.83 (3H, s), 2.92-2.97 (4H, m), 2.57-2.61 (2H, m), 2.50 (1H, t, J = 2.4 Hz), 2.21 (1H, t, J = 2.7 Hz)

### Preparation Example 3

0.5 g of 2-amino-2-(4-chlorophenyl)acetonitrile hydrochloride, 0.61 g of 3-(3,4-dimethoxyphenyl)propionyl chloride, 0.59 g of WSC and 10 ml of dimethylformamide were mixed and stirred at 80°C for 3 hours. The reaction mixture was allowed to cool to around room temperature and then concentrated under reduced pressure. Thereto water was added and the mixture was extracted with ethyl acetate. The organic layer was washed successively with 5% hydrochloride acid, water, an aqueous saturated sodium hydrogen carbonate solution and an aqueous saturated sodium chloride solution, dried over magnesium sulfate, and then concentrated under reduced pressure. The residue was subjected to silica gel column purification to obtain 0.30 g of N-{1-(4-chlorophenyl)-1-cyanomethyl}-3-(3,4-dimethoxyphenyl)propanamide (hereinafter, referred to as the compound 3 of the present invention). 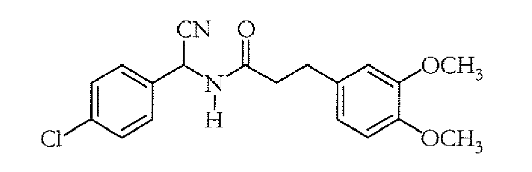 ¹H-NMR (CDCl₃, TMS) δ (ppm): 7.34-7.37 (2H, m), 7.22-7.24 (2H, m), 6.76 6 (1H, d, J = 8.5 Hz), 6.69-6.71 (2H, m), 6.09 (1H, d, J = 8.5 Hz), 5.77 (1H, br.d), 3.85 (3H, s), 3.84 (3H, s), 2.95 (2H, t, J = 7.2 Hz), 2.47-2.63 (2H, m)

### Preparation Example 4

0.33 g of 2-amino-2-phenylacetonitrile hydrochloride, 0.88 ml of diisopropylethylamine and 10 ml of tetrahydrofuran were mixed, and thereto a mixture of 0.50 g of 3-{3-methoxy-4-(2-propynyloxy)phenyl}propionyl chloride and 3 ml of tetrahydrofuran was added at 0 to 5°C. Then, the mixture was stirred at room temperature for 1 hour and then concentrated under reduced pressure. Thereto water was added and the mixture was extracted with ethyl acetate. The organic layer was washed successively with 5% hydrochloric acid, water, an aqueous saturated sodium hydrogen carbonate solution and an aqueous saturated sodium chloride solution, dried over magnesium sulfate, and then concentrated under reduced pressure. The residue was subjected to silica gel column purification to obtain 0.39 g of N-(1-phenyl-1-cyanomethyl)-3-{3-metnoxy-4-(2-propynyloxy)phenyl}propanamide (hereinafter, referred to as the compound 4 of the present invention). 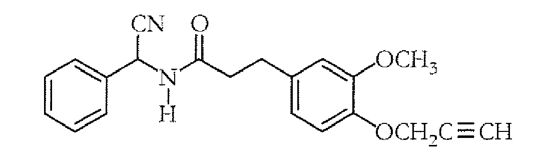 ¹H-NMR (CDCl₃, TMS) δ (ppm): 7.38-7.41 (3H, m), 7.31-7.34 (2H, m), 6.95 (1H, d, J = 8.2 Hz), 6. 70-6. 73 (2H, m), 6. 10 (1H, d, J = 8.5 Hz), 5.80 (1H, br.d), 4.73 (2H, d, J = 2.4 Hz), 3.83 (3H, s), 2.96 (2H, t, J = 7.3 Hz), 2.48-2.62 (3H, m)

### Preparation Example 5

Using 0.40 g of 2-amino-2-(4-methylphenyl)acetonitrile hydrochloride and 0.50 g of 3-{3-methoxy-4-(2-propynyloxy)phenyl}propionyl chloride, 0.54 g of N-{1-(4-methylphenyl)-1-cyanomethyl}-3-{3-me thoxy-4-(2-propynylaxy)phenyl}propanamide (hereinafter, referred to as the compound 5 of the present invention) was obtained in the same way as Preparation Example 4. 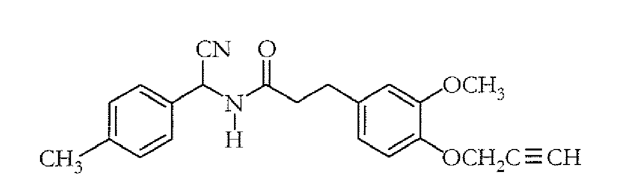 ¹H-NMR (CDCl₃, TMS) δ (ppm): 7.19-7.23 (4H, m), 6.94 (1H, d, J = 7.8 Hz), 6.70-6.72 (2H, m), 6.04 (1H, d, J = 8.2 Hz), 5.75 (1H, br.d, J = 8.2 Hz), 4.73 (2H, d, J = 2.2 Hz), 3.83 (3H, s), 2.95 (2H, t, J = 7.5 Hz), 2.46-2.60 (3H, m), 2.36 (3H, s)

### Preparation Example 6

Using 0.52 g of 2-amino-2-(3,4-dichlorophenyl)acetonitrile hydrochloride and 0.50 g of 3-{3-methoxy-4-(2-propynyloxy)phenyl}propionyl chloride, 0.45 g of N-{1-(3,4-dichlorophenyl)-1-cyanomethyl}-3-{3-methoxy-4-(2-propynyloxy)phenyl}propanamide (hereinafter, referred to as the compound 6 of the present invention) was obtained in the same way as Preparation Example 4. 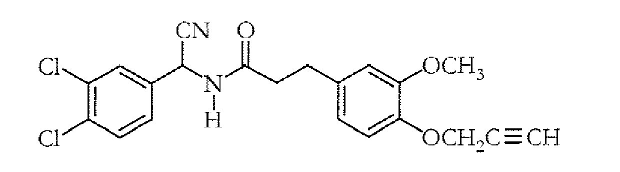 ¹H-NMR (CDCl₃ , TMS) δ (ppm): 7. 44-7. 48 (2H, m), 7.11 (1H, dd, J = 8.5 Hz, 2.2 Hz), 6.94 (1H, d, J = 7.8 Hz), 6.70-6.72 (2H, m), 6.10 (1H, d, J = 8.7 Hz), 5.93 (1H, br.d), 4.73 (2H, d, J = 2.4 Hz), 3. 83 (3H, s), 2.95 (2H, t, J = 7.7 Hz), 2.49-2.60 (3H, m)

### Preparation Example 7

Using 0.52 g of 2-amino-2-(3-chlorophenyl)acetonitrile hydrochloride and 0.50 g of 3-{3-methoxy-4-(2-propynyloxy)phenyl}propionyl chloride, 0.45 g of N-{1-(3-chlorophenyl)-1-cyanomethyl}-3-{3-methoxy-4-(2-propynyloxy)phenyl}propanamide (hereinafter, referred to as the compound 7 of the present invention) was obtained in the same way as Preparation Example 4. 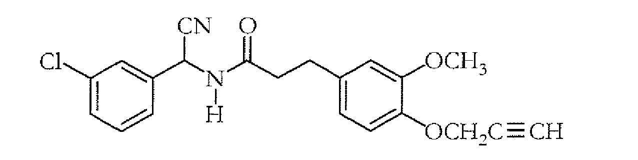 ¹H-NMR (CDCl₃, TMS) δ (ppm): 7.31-7.39 (3H, m), 7.19 (1H, d, J = 7.6 Hz), 6.95 (1H, d, J = 8.0 Hz), 6.70-6.72 (2H, m), 6.11 (1H, d, J = 8.3 Hz), 5.87 (1H, br.d), 4.73 (2H, d, J = 2.4 Hz), 3.83 (3H, s), 2.96 (2H, t, J = 7.3 Hz), 2.48-2.63 (3H. m)

### Preparation Example 8

Using 0.35 g of 2-amino-2-(4-methoxyphenyl)acetonitrile hydrochloride and 0.30 g of 3-{3-methoxy-4-(2-propynyloxy)phenyl}propionyl chloride, 0.24 g of N-{1-(4-methoxyphenyl)-1-cyanomethyl}-3-{3-methoxy-4-(2-propynyloxy)phenyl}propanamide (hereinafter, referred to as the compound 8 of the present invention) was obtained in the same way as Preparation Example 4. 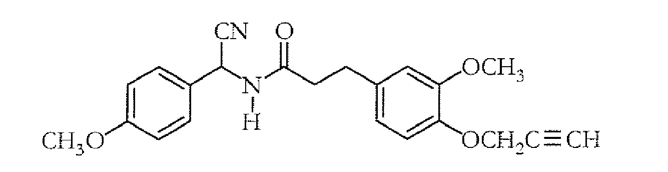 ¹H-NMR (CDCl₃, TMS) δ (ppm): 7.28-7. 30 (2H, m), 6.92-6.95 (3H, m), 6.70-7.00 (2H, m), 6.00 (1H, d, J = 8.0 Hz), 5.74 (1H, br.d), 4.73 (2H, d, J = 2.4 Hz), 3.83 (3H, s), 3.82 (3H, s), 2.95 (2H, t, J = 7.4 Hz), 2.48-2.50 (3H, m)

### Preparation Example 9

Using 0.73 g of 2-amino-2-(4-bromophenyl)acetonitrile hydrochloride and 0.50 g of 3-{3-methoxy-4-(2-propynyloxy)phenyl}propionyl chloride, 0.67 g of N-{1-(4-bromophenyl)-1-cyanomethyl}-3-{3-methoxy-4-(2-propynyloxy)phenyl}propanamide (hereinafter, referred to as the compound 9 of the present invention) was obtained in the same way as Preparation Example 4. 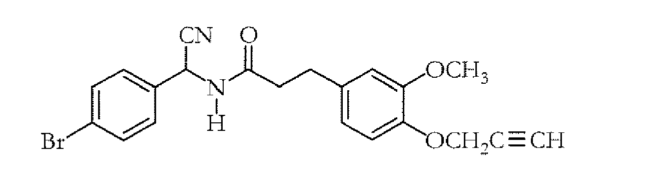 ¹H-NMR (CDCl₃, TMS) δ (ppm): 7.50-7.54 (2H, m), 7.15-7.17 (2H, m), 6.94 (1H, d, J = 8.0 Hz), 6.69-6.71 (2H, m), 6.07 (1H, d, J = 8.2 Hz), 5.92 (1H, br.d), 4.74 (2H, d, J = 2.2 Hz), 3.82 (3H, s), 2.95 (2H, t, J = 7.2 Hz), 2.47-2.62 (3H, m)

### Preparation Example 10

0.53 g of 4-ethylbenzaldehyde, 0.55 ml of trimethylsilyl cyanide and 0.03 g of zinc iodide were mixed and stirred at room temperature for 15 minutes. Thereto 1 ml of a 10% solution of ammonia in methanol was added and stirred at 40°C for 2 hours. The mixture was allowed to cool to room temperature and then concentrated under reduced pressure. To the residue were added 0.68 ml of diisopropylethylamine and 10 ml of tetrahydrofuran. Thereto a mixed solution of 0.50 g of 3-{3-methoxy-4-(2-propynyloxy)phenyl}propionyl chloride and 3 ml of tetrahydrofuran was added at 0 to 5°C, and stirred at room temperature for 1 hour. Water was then added to the reaction mixture and the mixture was extracted with ethyl acetate. The organic layer was washed successively with 5% hydrochloric acid, water, an aqueous saturated sodium hydrogen carbonate solution and an aqueous saturated sodium chloride solution, dried over magnesium sulfate, and then concentrated under reduced pressure. The residue was subjected to silica gel column purification to obtain 0.73 g of N-{1-(4-ethylphenyl)-1-cyanomethyl}-3-{3-methoxy-4-(2-propynyloxy)phenyl}propanamide (hereinafter, referred to as the compound 10 of the present invention). 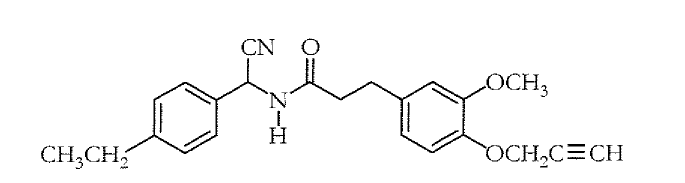 ¹H-NMR (CDCl₃, TMS) δ (ppm) : 7.20-7.25 (4H, m), 6.94 (1H, d, J = 7.8 Hz), 6.70-6.72 (2H, m), 6.04 (1H, d, J = 8.3 Hz), 5.83 (1H, br.d), 4.73 (2H, d, J = 2.4 Hz), 3.82 (3H, s), 2.96 (2H, t, J = 7.6 Hz), 2.66 (2H, q, J = 7.6 Hz), 2.46-2.58 (3H, m), 1.23 (3H, t, J = 7.6 Hz)

### Preparation Example 11

Using 1.24 g of 2-naphthoaldehyde and 1.0 g of 3-{3-methoxy-4-(2-propynyloxy)phenyl}propionyl chloride, 0.92 g of N-{1-(naphthalen-2-yl)-1-cyanomethyl}-3-{3-methoxy-4-(2-propynyloxy)phenyl}propanamide (hereinafter, referred to as the compound 11 of the present invention) was obtained in the same way as Preparation Example 10. 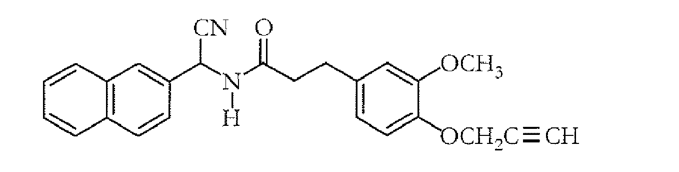 ¹H-NMR (CDCl₃, TMS) δ (ppm): 7.84-7.91 (4H, m), 7.54-7.57 (2H, m), 7.26-7.28 (1H, m), 6.90 (1H, d, J = 8.0 Hz), 6.68-6.73 (2H, m), 6.27 (1H, d, J = 8.5 Hz), 5.88 (1H, br.d, J = 8.5 Hz), 4.69 (2H, d, J = 2.5 Hz), 3.81 (3H, s), 2.97 (2H, t, J = 7.5 Hz), 2.49-2.64 (2H, m), 2.46 (1H, t, J = 2.4 Hz) Preparation Example 12

Using 0.49 g of 4-fluorobenzaldehyde and 0.50 g of 3-{3-methoxy-4-(2-propynyloxy)phenyl}propionyl chloride, 0.43 g of N-{1-(4-fluorophenyl)-1-cyanomethyl}-3-{3-methoxy-4-(2-propynyloxy)phenyl}propanamide (hereinafter, referred to as the compound 12 of the present invention) was obtained in the same way as Preparation Example 10. 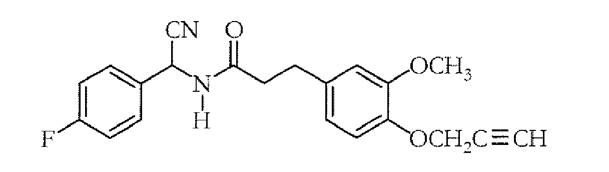 ¹H-NMR (CDCl₃, TMS) δ (ppm): 7.26-7.30 (2H, m), 7.06-7.11 (2H, m), 6.94 (1H, d, J = 8.0 Hz), 6.70-6.72 ;2H, m), 6.08 (1H, d, J = 8.2 Hz), 5.83 (1H, br.d), 4.73 (2H, d, J = 2.4 Hz), 3.83 (3H, s), 2.95 (2H, t, J = 7.2 Hz), 2.48-2.62 (3H, m)

### Preparation Example 13

Using 0.69 g of 4-(trifluoromethyl)benzaldehyde and 0.50 g of 3-{3-methoxy-4-(2-propynyloxy)phenyl}propionyl chloride, 0.43 g of N-{1-(4-trifluoromethylphenyl)-1-cyanomethyl}-3-{3-methoxy-4-(2-propynyloxy)phenyl}propanamide (hereinafter, referred to as the compound 13 of the present invention) was obtained in the same way as Preparation Example 10. 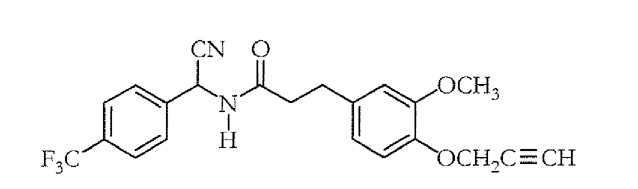 ¹H-NMR (CDCl₃ , TMS) δ (ppm): 7.65-7.67 (2H, m), 7.41-7.43 (2H, m), 6.95 (1H, d, J = 8.2 Hz), 6.71-6.73 (2H, m), 6.20 (1H, d, J = 8.5 Hz), 5.91 (1H, br.d), 4.74 (2H, d, J = 2.2 Hz), 3.82 (3H, s), 2.96 (2H, t, J = 7.3 Hz), 2.48-2.65 (3H, m)

### Preparation Example 14

Using 0.65 g of 5,6,7,8-tetrahydronaphthalene-2-carbaldehyde and 0.50 g of 3-{3-methoxy-4-(2-propynyloxy)phenyl}propionyl chloride, 0.27 g of N-{1-(5, 6, 7, 8-tetrahydronaphthalen-2-yl)-1-cyanomethyl}-3-{3-methoxy-4-(2-propynyloxy)phenyl}propanamide (hereinafter, referred to as the compound 14 of the present invention) was obtained in the same way as Preparation Example 10. 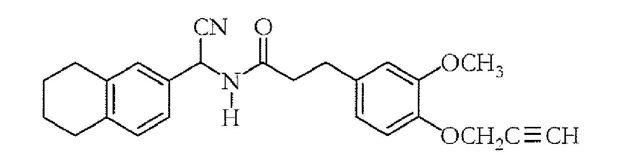 ¹H-NMR (CDCl₃, TMS) δ (ppm) : 7.07-7.09 (2H, m), 7.02 (1H, dd, J = 8.0 Hz, 2.2 Hz), 6.95 (1H, d, J = 7.8 Hz), 6.70-6.72 (2H, m), 5.89 (1H, d, J = 8.3 Hz), 5.76 (1H, br.d, J = 8.2 Hz), 4.73 (2H, d, J = 2.4 Hz), 3.83 (3H, s), 2.94 (2H, t, J = 7.5 Hz), 2.50-2.80 (4H, m), 2.49-2.58 (2H, m), 2.48 (1H, t, J = 2.5 Hz), 1.77-1.80 (4H, m)

### Preparation Example 15

Using 0.65 g of 5,6,7,8-tetrahydronaphthalene-2-carbaldehyde and 0.45 g of 3-(3,4-dimethoxyphenyl)propionyl chloride, 0.21 g of N-{1-(5,6,7,8-tetrahydronaphthalen-2-yl)-1-cyanomethyl}-3-(3,4-dimethoxyphenyl)propanamide (hereinafter, referred to as the compound 15 of the present invention) was obtained in the same way as Preparation Example 10. 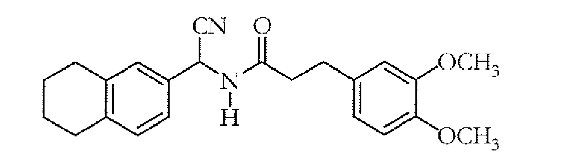 ¹H-NMR (CDCl₃, TMS) δ (ppm): 7.00-7.08 (3H, m), 6.70-6.79 (3H, m), 5.98 (1H, d, J = 8.2 Hz), 5.74 (1H, br.d), 3.85 (3H, s), 3.84 (3H, s), 2.94 (2H, t, J = 7.5 Hz), 2.74-2.75 (4H, m), 2.48-2.56 (2H, m), 1.77-1.81 (4H, m)

### Preparation Example 16

406 mg of 2-amino-2-(4-chlorophenyl)acetonitrile hydrochloride, 517 mg of 3-{3,4-bis(2-propynyloxy)phenyl}propionic acid and 10 ml of pyridine were mixed. Thereto 420 mg of WSC was added, and the mixture was stirred at room temperature for 1.5 hours. Water was then added to the reaction mixture, and the mixture was extracted with ethyl acetate. The organic layer was washed successively with 5% hydrochloric acid, water and an aqueous saturated sodium chloride solution, dried over magnesium sulfate, and then concentrated under reduced pressure. The residue was subjected to silica gel column purification to obtain 254 mg of N-{1-(4-chlorophenyl)-1-cyanomethyl}-3-{3,4-bis(2-propynyloxy)phenyl}propanamide (hereinafter, referred to as the compound 16 of the present invention). 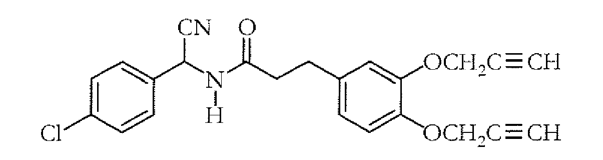 ¹H-NMR (CDCl₃, TMS) δ (ppm): 7.33-7.39 (2H, m), 7.20-7.25 (2H, m), 6.95 (1H, d, J = 8.2 Hz), 6.88 (1H, d, J = 1.9 Hz), 6.77 (1H dd, J = 1.9 Hz, 8.2 Hz), 6.07 (1H, d, J = 8.4 Hz), 5.84 (1H, br.d), 4.70-4.75 (4H, m), 2.95 (2H, t, J = 7.2 Hz), 2.44-2.65 (4H, m)

### Preparation Example 17

457 mg of 2-amino-2-(4-chlorophenyl)acetonitrile hydrochloride, 379 mg of triethylamine and tetrahydrofuran were mixed. Thereto 400 mg of 3-{3-ethoxy-4-(2-propynyloxy)phenyl}propionyl chloride was added at 0 to 5°C, and the mixture was stirred at room temperature for 1.5 hours. After the reaction mixture was concentrated under reduced pressure, water was added and the mixture was extracted with ethyl acetate. The organic layer was washed successively with 5% hydrochloric acid, an aqueous saturated sodium hydrogen carbonate solution, water and an aqueous saturated sodium chloride solution, dried over magnesium sulfate, and then concentrated under reduced pressure. The residue was subjected to silica gel column purification to obtain 493 mg of N-{1-(4-chlorophenyl)-1-cyanomethyl}-3-{3-ethoxy-4-(2-propynyloxy)phenyl}propanamide (hereinafter, referred to as the compound 17 of the present invention). 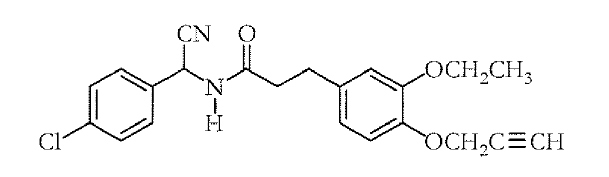 ¹H-NMR (CDCl₃, TMS) δ (ppm): 7.34-7.38 (2H, m), 7.20-7.24 (2H, m), 6.95 (1H, d, J = 8.0 Hz), 6.72 (1H, d, J = 1.9 Hz), 6.69 (1H, dd, J = 1.9 Hz, 8.0 Hz), 6.08 (1H, d, J = 8.4 Hz), 5.80 (1H, br.d), 4.74 (2H, d, J = 2.4 Hz), 4.00-4.08 (2H, m), 2.93 (2H, t, J = 7.2 Hz), 2.46-2.65 (3H, m), 1.42 (3H, t, J = 7.0 Hz)

### Preparation Example 18

In the same way as Preparation Example 16, 144 mg of N-{1-(4-chlorophenyl)-1-cyanomethyl}-3-{4-methoxy-3-(2-propynyloxy)phenyl}propanamide (hereinafter, referred to as the compound 18 of the present invention) was obtained from 305 mg of 2-amino-2-(4-chlorophenyl)acetonitrile hydrochloride and 380 mg of 3-{4-methoxy-3-(2-propynyloxy)phenyl}propionic acid. 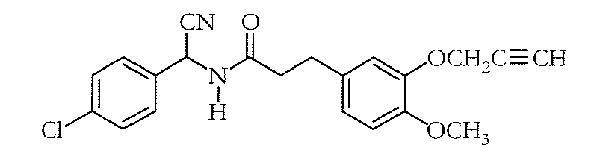 ¹H-NMR (CDCl₃, TMS) δ (ppm): 7.18-7.35 (4H, m), 6.73-6.85 (3H, m), 6.27 (1H, br.d), 6.04 (1H, d, J = 8.3 Hz), 4.70 (2H, d, J = 2.2 Hz), 3.81 (3H, s), 2.91 (2H, t, J = 7.3 Hz), 2.44-2.60 (3H, m)

### Preparation Example 19

In the same way as Preparation Example 16, 300 mg of N-{1-(4-chlorophenyl)-1-cyanomethyl}-3-(3-methoxy-4-ethoxyphenyl)propanamide (hereinafter, referred to as the compound 19 of the present invention) was obtained from 406 mg of 2-amino-2-(4-chlorophenyl)acetonitrile hydrochloride and 448 mg of 3-(3-methoxy-4-ethoxyphenyl)propionic acid. 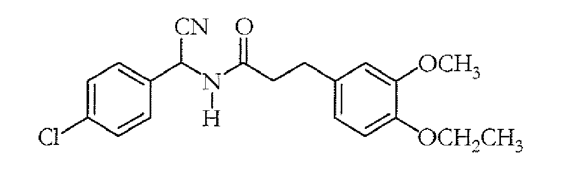 ¹H-NMR (CDCl₃, TMS) δ (ppm): 7.20-7.37 (4H, m), 6.65-6.78 (3H, m), 6.09 (1H d, J = 8.3 Hz), 5.83 (1H, br.d), 4.06 (2H, q, J = 7.0 Hz), 3.83 (3H, s), 2.93 (2H, t, J = 7.1 Hz), 2.45-2.64 (2H, m), 1.45 (3H, t, J = 7.0 Hz)

### Preparation Example 20

In the same way as Preparation Example 16, 247 mg of N-{1-(4-chlorophenyl)-1-cyanomethyl}-3-{3-methoxy-4-(2-propenyloxy)phenyl}propanamide (hereinafter, referred to as the compound 20 of the present invention) was obtained from 406 mg of 2-amino-2-(4-chlorophenyl)acetonitrile hydrochloride and 476 mg of 3-{3-methoxy-4-(2-propenyloxy)phenyl}propionic acid. 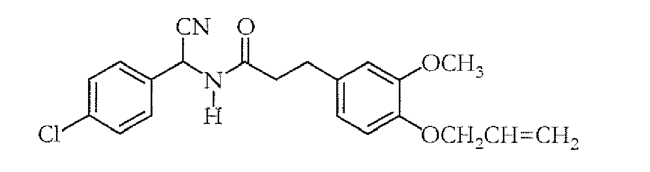 ¹H-NMR (CDCl₃, TMS) δ (ppm): 7.20-7.40 (4H, m), 6.63-6.79 (3H, m), 6.02-6.14 (2H, m), 5.84 (1H, br.d), 5.24-5.46 (2H, m), 4.55-4.60 (2H, m), 3.83 (3H, s), 2.93 (2H, t, J = 7.3 Hz), 2.45-2.70 (2H, m)

### Preparation Example 21

In the same way as Preparation Example 10, 0.33 g of N-{1-(2-chlorophenyl)-1-cyanomethyl}-3-{3-methoxy-4-(2-propynyloxy)phenyl}propanamide (hereinafter, referred to as the compound 21 of the present invention) was obtained from 0.56 g of 2-chlorobenzaldehyde and 0.50 g of 3-{3-methoxy-4-(2-propynyloxy)phenyl}propionyl chloride. 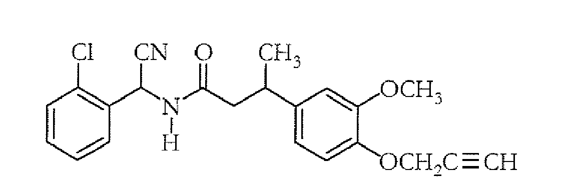 ¹H-NMR (CDCl₃, TMS) δ (ppm): 7.52 (1H, dd, J = 5.6 Hz, 1.9 Hz), 7.30-7.45 (3H, m), 6.93 (1H, d, J = 8.9 Hz), 6.69-6.71 (2H, m), 6.24 (1H, d, J = 8.0 Hz), 5.85 (1H, br.d), 4.72 (2H, d, J = 2.4 Hz), 3.81 (3H, s), 2.92-2.95 (2H, m), 2.51-2.56 (2H, m), 2.49 (1H, t, J = 2.4 Hz)

### Preparation Example 22

457 mg of 2-amino-2-(4-chlorophenyl)acetonitrile hydrochloride, 379 mg of triethylamine and 10 ml of tetrahydrofuran were mixed. Thereto a mixture of 400 mg of 3-{3-methoxy-4-(2-propynyloxy)phenyl}butyryl chloride and 5 ml of tetrahydrofuran was added at 0 to 5°C. The mixture was stirred at room temperature for 2 hours and then concentrated under reduced pressure. To the residue was added water, and the mixture was extracted with ethyl acetate. The organic layer was washed successively with 5% hydrochloric acid, water, an aqueous saturated sodium hydrogen carbonate solution and an aqueous saturated sodium chloride solution, dried over magnesium sulfate, and then concentrated under reduced pressure. The residue was subjected to silica gel column purification to obtain 460 mg of N-{1-(4-chlorophenyl)-1-cyanomethyl}-3-(3-methoxy-4-(2-propynyloxy)phenyl)butylamide (hereinafter, referred to as the compound 22 of the present invention). 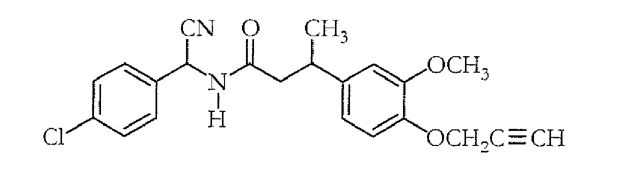 ¹H-NMR (CDCl₃, TMS) δ (ppm): 7.20-7.40 (3H, m), 6.90-7.04 (2H, m), 6.68-6.75 (2H, m), 5.96-6.05 (1H, m), 5.70-5.82 (1H, m), 4.74 (2H, d, J = 2.4 Hz), 3.84 (1.5H, s), 3.80 (1.5H, s), 3.19-3.30 (1H, m), 2.36-2.62 (3H, m), 1.29-1.36 (3H. m)

### Preparation Example 23

In the same way as Preparation Example 22, 370 mg of N-{1-(4-chlorophenyl)-1-cyanomethyl}-3-{3-methoxy-4-(2-propynyloxy)phenyl}-2-methylpropanamide (hereinafter, referred to as the compound 23 of the present invention) was obtained from 457 mg of 2-amino-2-(4-chlorophenyl)acetonitrile hydrochloride and 400 mg of 3-{3-methoxy-4-(2-propynyloxy)phenyl}-2-methyl-propionyl chloride. 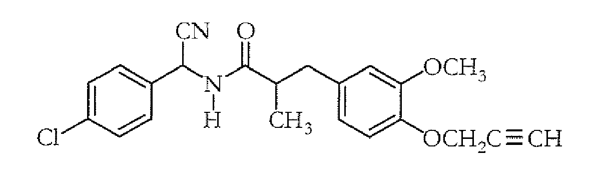 ¹H-NMR (CDCl₃, TMS) δ (ppm): 7.24-7.38 (3H, m), 6.86-7.02 (2H, m), 6.62-6.72 (2H, m), 5.91-6.03 (2H, m), 4.72 (2H, d, J = 2.1 Hz), 3.83 (1.5H, s), 3.76 (1.5H, s), 2.53-2.70 (4H, m), 1.20-1.28 (3H, m)

### Preparation Example 24

In the same way as Preparation Example 16, 380 mg of N-{1-(4-chlorophenyl)-1-cyanomethyl}-3-(3-methoxy-4-propoxyphenyl)propanamide (hereinafter, referred to as the compound 24 of the present invention) was obtained from 406 mg of 2-amino-2-(4-chlorophenyl)acetonitrile hydrochloride and 476 mg of 3-(3-methoxy-4-propoxyphenyl)propionic acid. 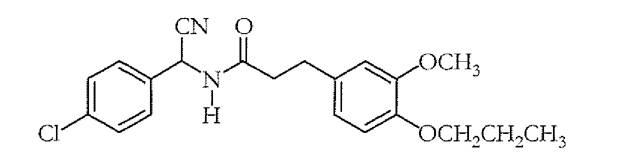 ¹H-NMR (CDCl₃, TMS) δ (ppm): 7.31-7.37 (2H, m), 7.20-7.24 (2H, m), 6.64-6.75 (3H, m), 6.09 (1H, d, J = 8.5 Hz), 5.84 (1H, d, J = 8.5 Hz), 3.93 (2H, t, J = 6.8 Hz), 3.82 (3H, s), 2.93 (2H, t, J = 7.2 Hz), 2.46-2.63 (2H, m), 1.81-1.90 (2H, m), 1.04 (3H, t, J = 7.3 Hz)

### Preparation Example 25

In the same way as Preparation Example 16, 398 mg of N-{1-(4-chlorophenyl)-1-cyanomethyl}-3-(4-isopropoxy-3-methoxyphenyl)propanamide (hereinafter, referred to as the compound 25 of the present invention) was obtained from 406 mg of 2-amino-2-(4-chlorophenyl)acetonitrile hydrochloride and 477 mg of 3-(4-isopropoxy-3-methoxyphenyl)propionic acid. 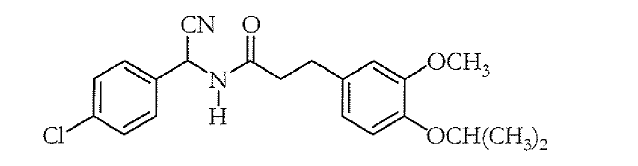 ¹H-NMR (CDCl₃, TMS) δ (ppm) : 7.33-7.38 (2H, m), 7.20-7.26 (2H, m), 6.78 (1H, d, J = 8.1 Hz), 6.64-6.70 (2H, m), 6.09 (1H, d, J = 8.5 Hz), 5.89 (1H, d, J = 8.3 Hz), 4.42-4.50 (1H, m), 3.80 (3H, s), 2.93 (2H, t, J = 7.2 Hz), 2.46-2.64 (2H, m), 1.35 (6H, d, J = 6.3 Hz)

### Preparation Example 26

In the same way as Preparation Example 16, 420 mg of N-{1-(4-chlorophenyl)-1-cyanomethyl}-3-(4-acetoxy-3-methoxyphenyl)propanamide (hereinafter, referred to as the compound 26 of the present invention) was obtained from 406 mg of 2-amino-2-(4-chlorophenyl)acetonitrile hydrochloride and 476 mg of 3-(4-acetoxy-3-methoxyphenyl)propionic acid. 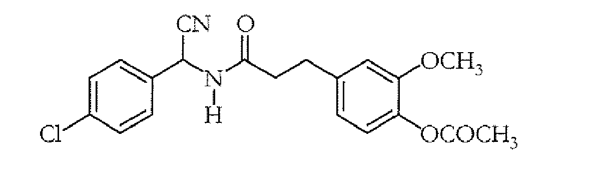 ¹H-NMR (CDCl₃, TMS) δ (ppm): 7.37 (2H, d, J = 8.7 Hz), 7.26 (2H, d, J = 8.3 Hz), 6.90 (1H, d, J = 8.0 Hz), 6.76 (1H, d, J = 1.7 Hz), 6.71 (1H, dd, J = 8.2 Hz, 1.9 Hz), 6.13 (1H, br.d), 6.05 (1H, d, J = 8.2 Hz), 3.77 (3H, s), 2.96 (2H, t, J = 7.5 Hz), 2.44-2.61 (2H, m), 2.30 (3H, s)

### Preparation Example 27

In the same way as Preparation Example 16, 155 mg of N-{1-(4-chlorophenyl)-1-cyanomethyl}-3-{4-(2-fluoroethoxy)-3-methoxyphenyl}propanamide (hereinafter, referred to as the compound 27 of the present invention) was obtained from 203 mg of 2-amino-2-(4-chlorophenyl)acetonitrile hydrochloride and 242 mg of 3-{4-(2-fluoroethoxy)-3-methoxyphenyl}propionic acid. 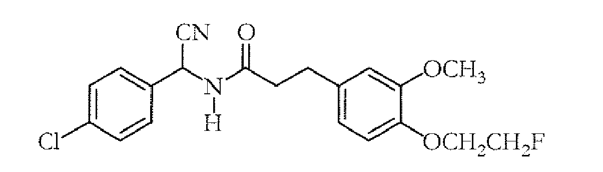 ¹H-NMR (CDCl₃, TMS) δ (ppm): 7.36 (2H, d, J = 8.7 Hz), 7.24 (2H, d, J = 8.3 Hz), 6.81 (1H, d, J = 7.9 Hz), 6.72 (1H, d, J = 1.9 Hz), 6.68 (1H, dd, J = 7.9 Hz, 1.9 Hz), 6.08 (1H, d, J = 8.3 Hz), 5.82 (1H, d, J = 8.7 Hz), 4.76 (2H, ddd, J = 47.4 Hz, 4.3 Hz, 4.3 Hz), 4.23 (2H, ddd, J = 27.7 Hz, 4.3 Hz, 4.3 Hz), 3.82 (3H, s), 2.94 (2H, t, J = 7.3 Hz), 2.45-2.63 (2H, m)

### Preparation Example 28

In the same way as Preparation Example 16, 155 mg of N-{1-(4-ohlorophenyl)-1-cyanomethyl}-3-{4-(2-butynyloxy)-3-methoxyphenyl}propanamide (hereinafter, referred to as the compound 28 of the present invention) was obtained from 203 mg of 2-amino-2-(4-chlorophenyl)acetonitirle hydrochloride and 248 mg of 3-{4-(2-butynyloxy)-3-methoxyphenyl}propionic acid. 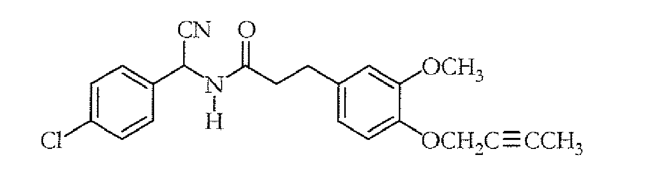 ¹H-NMR (CDCl₃, TMS) δ (ppm): 7.36 (2H, d, J = 8.7 Hz), 7.21 (2H, d, J = 8.4 Hz), 6.92 (1H, d, J = 8.7 Hz), 6.68-6.73 (2H, m), 6.08 (1 H, d, J = 8.4 Hz), 5.75 (1H, d, J = 8.0 Hz), 4.69 (2H, q, J = 2.4 Hz), 3.82 (3H, s), 2.95 (2H, t, J = 7.2 Hz), 2.46-2.64 (2H, m), 1.82 (3H, t, J = 2.4 Hz)

### Preparation Example 29

In the same way as Preparation Example 16, 207 mg of N-{1-(4-chlorophenyl)-1-cyanomethyl}-3-{3-methoxy-4-(1-methyl-2-propynyloxy)phenyl}propanamide (hereinafter, referred to as the compound 29 of the present invention) was obtained from 244 mg of 2-amino-2-(4-chlorophenyl)acetonitrile hydrochloride and 300 mg of 3-{3-methoxy-4-(1-methyl-2-propynyloxy)phenyl}propionic acid. 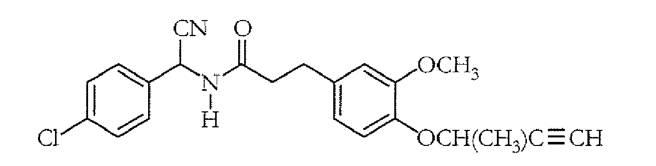 ¹H-NMR (CDCl₃, TMS) δ (ppm): 7.36 (2H, d, J = 8.5 Hz), 7.22 (2H, dd, J = 8.1 Hz, 3.8 Hz), 6.99 (1H, dd, J = 7.8 Hz, 1.4 Hz), 6.67-6.73 (2H, m), 6.06-6.11 (1H, m), 5.76 (1H, d, J = 8.5 Hz), 4.82-4.96 (1H, m), 3.81 (1.5H, s), 3.80 (1.5H, s), 2.95 (2H, t, J = 7.2 Hz), 2.47-2.65 (2H, m), 2.43-2.46 (1H, m), 1. 70 (3H, d, J = 6.6 Hz)

### Preparation Example 30

0.74 g of 4-isopropylbenzaldehyde, 0.71 ml of trimethtylsilyl cyanide and 53 mg of zinc iodide were mixed and stirred at room temperature for 15 minutes. Thereto 8 ml of a 10% solution of ammonia in methanol was added and stirred at 40°C for 2 hours. The mixture was allowed to cool to around room temperature and then concentrated under reduced pressure. To the resulting residue were added 0.7 g of 3-{3-methoxy-4-(2-propynyloxy)phenyl}propionic acid and 5 ml of pyridine, and then was added 0.7 g of WSC. The mixture was stirred at room temperature for 4 hours. Water was then added to the reaction mixture, and the mixture was extracted with ethyl acetate. The organic layer was washed successively with 5% hydrochloric acid, water, an aqueous saturated sodium hydrogen carbonate solution and an aqueous saturated sodium chloride solution, dried over magnesium sulfate, and then concentrated under reduced pressure. The residue was subjected to silica gel column purification to obtain 0.93 g of N-{1-(4-isopropylphenyl)-1-cyanomethyl}-3-{3-methoxy-4-(2-propynyloxy)phenyl}propanamide (hereinafter, referred to as the compound 30 of the present invention). 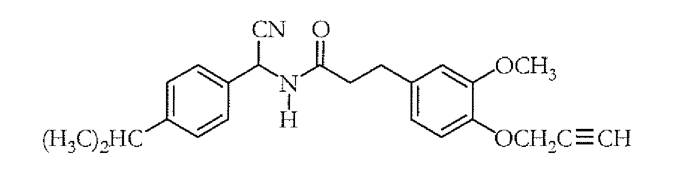 ¹H-NMR (CDCl₃, TMS) δ (ppm): 7.24 (4H, s), 6.94 (1H, d, J = 8.3 Hz), 6.68-6.75 (2H, m), 6.03 (1H, d, J = 8.3 Hz), 5.92 (1H, d, J = 8.3 Hz), 4.72 (2H, d, J = 2.4 Hz), 3.81 (3H, s), 2.86-2.98 (3H, m), 2.46-2.58 (2H, m), 2.48 (1H, t, J = 2.4 Hz), 1.24 (6H, d, J = 7.1 Hz)

### Preparation Example 31

In the same way as Preparation Example 30, 480 mg of N-{1-(4-cyanophenyl)-1-cyanomethyl}-3-{3-methoxy-4-(2-propynyloxy)phenyl}propanamide (hereinafter, referred to as the compound 31 of the present invention) was obtained from 656 mg of 4-cyanobenzaldehyde and 0.7 g of 3-{3-methoxy-4-(2-propynyloxy)phenyl}propionic acid. 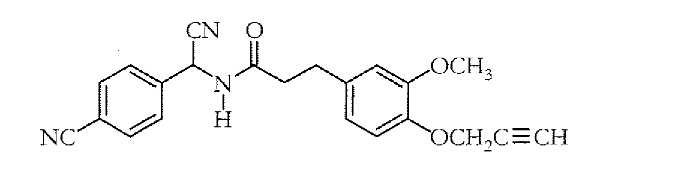 ¹H-NMR (CDCl₃, TMS) δ (ppm) : 7.68 (2H, d, J = 7.5 Hz), 7.38 (2H, d, J = 8.3 Hz), 6.96 (1H, d, J = 7.5 Hz), 6.65-6.74 (2H, m), 6.21 (1H, d, J = 9.1 Hz), 5.92 (1H, d, J = 8.7 Hz), 4.75 (2H, d, J = 2.4 Hz), 3.82 (3H, s), 2.96 (2H, t, J = 7.1 Hz), 2.48-2.68 (3H, m)

### Preparation Example 32

In the same way as Preparation Example 30, 0.93 g of N-{1-(4-trifluoromethoxyphenyl)-1-cyanomethyl}-3-{3-methoxy-4-(2-propynyloxy)phenyl}propanamide (hereinafter, referred to as the compound 32 of the present invention) was obtained from 951 mg of 4-trifluoromethoxybenzaldehyde and 0.7 g of 3-{3-methoxy-4-(2-propynyloxy)phenyl}propionic acid. 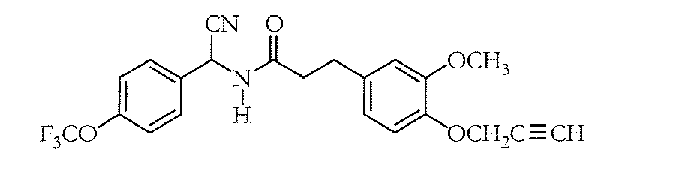 ¹H-NMR (CDCl₃, TMS) δ (ppm): 7.34 (2H, d, J = 8.7 Hz), 7.24 (2H, d, J = 7.9 Hz), 6.94 (1H, d, J = 7. 9 Hz), 6.69-6.75 (2H, m), 6.13 (1H, d, J = 8.3 Hz), 5. 88 (1H, d, J = 8.3 Hz), 4.73 (2H, d, J = 2.4 Hz), 3.82 (3H, s), 2.95 (2H, t, J = 7.1 Hz), 2.48-2.65 (2H, m), 2. 48 (1H, t, J = 2.4 Hz)

### Preparation Example 33

In the same way as Preparation Example 30, 608 mg of N-{1-(4-methylthiophenyl)-1-cyanomethyl}-3-{3-methoxy-4-(2-propynyloxy)phenyl}propanamide (hereinafter, referred to as the compound 33 of the present invention) was obtained from 0.76 g of 4-methylthiobenzaldehyde and 0.7 g of 3-{3-methoxy-4-(2-propynyloxy)phenyl}propionic acid. 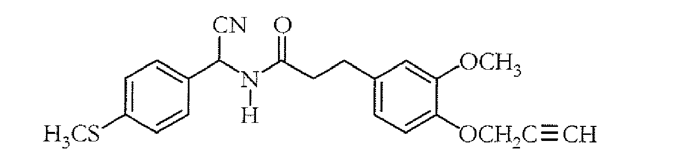 ¹H-NMR (CDCl₃, TMS) δ (ppm): 7.17-7.25 (4H, m), 6.93 (1H, d, J = 7.8 Hz), 6.68-6.74 (2H, m), 6.03 (1H, d, J = 8.3 Hz), 5.78 (1H, d, J = 8.3 Hz), 4.73 (2H, d, J = 2.4 Hz), 3.82 (3H, s), 2.94 (2H, t, J = 7.3 Hz), 2.47-2.62 (6H, m)

### Preparation Example 34

In the same way as Preparation Example 30, 75 mg of N-{1-(4-dimethylaminophenyl)-1-cyanomethyl}-3-{3-methoxy-4-(2-propynyloxy)phenyl}propanamide (hereinafter, referred to as the compound 34 of the present invention) was obtained from 0.75 g of 4-dimethylaminobenzaldehyde and 0.7 g of 3-{3-methoxy-4-(2-propynyloxy)phenyl}propionic acid. 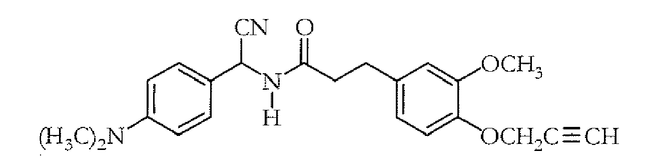 ¹H-NMR (CDCl₃ , TMS) δ (ppm) : 7.17 (2H, d, J = 8.5 Hz), 6.93 (1H, d, J = 8.1 Hz), 6.63-6.75 (4H, m), 5.91 (1H, d, J = 7.8 Hz), 5.69 (1H, d, J = 8.0 Hz), 4.72 (2H, d, J = 2.4 Hz), 3.82 (3H, s), 2.96 (6H, s), 2.93 (2H, t, J = 7.6 Hz), 2.43-2.58 (3H, m)

### Preparation Example 35

In the same way as Preparation Example 30, 0.39 g of N-{1-(2,4-dichlorophenyl)-1-cyanomethyl}-3-{3-methoxy-4-(2-propynyloxy)phenyl}propanamide (hereinafter, referred to as the compound 35 of the present invention) was obtained from 0.88 g of 2,4-dichlorobenzaldehyde and 0.7 g of 3-{3-methoxy-4-(2-propynyloxy)phenyl}propionic acid. 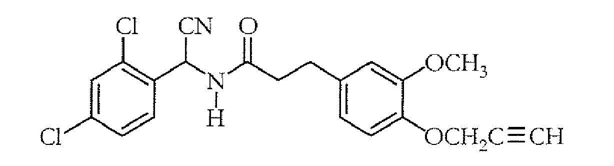 ¹H-NMR (CDCl₃, TMS) δ (ppm) : 7.43-7.46 (1H, m), 7.42 (1H, s), 7.31 (1H, dd, J = 8.3 Hz, 2.2 Hz), 6.92 (1H, d, J = 8.5 Hz), 6.67-6.71 (2H, m), 6.18 (1H, d, J = 7.8 Hz), 5.88 (1H, d, J = 8.0 Hz), 4.73 (2H, d, J = 2.4 Hz), 3.81 (3H, s), 2.92 (2H, t, J = 7.3 Hz), 2.48-2.57 (3H, m)

### Preparation Example 36

In the same way as Preparation Example 30, 315 mg of N-{1-(4-chloro-2-fluorophenyl)-1-cyanomethyl}-3-{3-methoxy-4-(2-propynyloxy)phenyl}propanamide (hereinafter, referred to as the compound 36 of the present invention) was obtained from 951 mg of 4-chloro-2-fluorobenzaldehyde and 0.7 g of 3-{3-methoxy-4-(2-propynyloxy)phenyl}propionic acid.
The compound 36 of the present invention 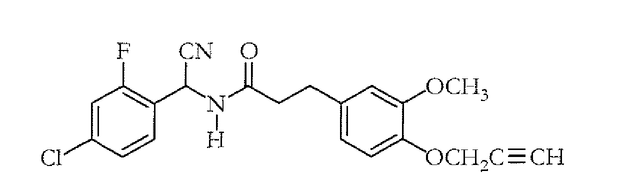 ¹H-NMR (CDCl₃, TMS) δ (ppm): 7.31-7.38 (1H, m), 7.14-7.22 (2H, m), 6.91 (1H, d, J = 8.7 Hz), 6.65-6.70 (2H, m), 6.13 (1H, d, J = 8.2 Hz), 6.01 (1H, d, J = 8.4 Hz), 4.72 (2H, d, J = 2.4 Hz), 3.80 (3H, s), 2.88-2.95 (2H, m), 2.45-2.60 (3H, m)

### Preparation Example 37

In the same way as Preparation Example 30, 0.72 g of N-{1-(2,4-difluorophenyl)-1-cyanomethyl}-3-{3-methoxy-4-(2-propynyloxy)phenyl}propanamide (hereinafter, referred to as the compound 37 of the present invention) was obtained from 0.71 g of 2,4-difluorobenzaldehyde and 0.7 g of 3-{3-methoxy-4-(2-propynyloxy)phenyl}propionic acid. 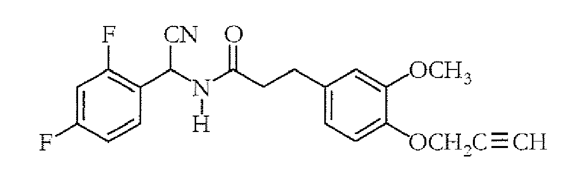 ¹H-NMR (CDCl₃, TMS) δ (ppm): 7.35-7.43 (1H, m), 6.85-6.95 (3H, m), 6.65-6.69 (2H, m), 6.13 (1H, d, J = 8.2 Hz), 6. 06 (1H, d, J = 8.2 Hz), 4.71 (2H, d, J = 2.4 Hz), 3.80 (3H, s), 2.88-2.94 (2H, m), 2.48-2.58 (3H, m)

### Preparation Example 38

In the same way as Preparation Example 30, 0.45 g of N-{1-(4-phenoxyphenyl)-1-cyanomethyl}-3-{3-methoxy-4-(2-propynyioxy)phenyl}propanamide (hereinafter, referred to as the compound 38 of the present invention) was obtained from 0.99 g of 4-phenoxybenzaldehyde and 0.7 g of 3-{3-methoxy-4-(2-propynyloxy)phenyl}propionic acid. 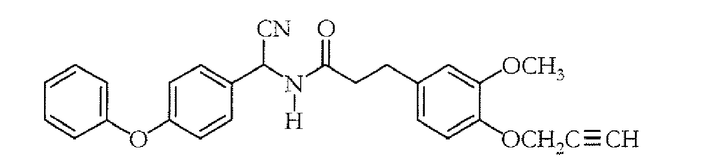 ¹H-NMR (CDCl₃, TMS) δ (ppm): 7.24-7. 40 (2H, m), 7.26-7.30 (2H, m), 7.14-7.19 (1H, m), 6.99-7.03 (4H, m), 6.93 (1H, d, J = 8.0 Hz), G.69-6.74 (2H, m), 6.05 (1H, d, J = 8.2 Hz), 5.87 (1H, d, J = 7.7 Hz), 4.70 (2H, d, J = 2.4 Hz), 3.82 (3H, s), 2.95 (2H, t, J = 7.4 Hz), 2.48-2.61 (2H, m), 2.46 (1H, t, J = 2.4 Hz)

### Preparation Example 39

In the same way as Preparation Example 30, 242 mg of N-{1-(biphenyl-4-yl)-1-cyanomethyl}-3-{3-methoxy-4-(2-propynyloxy)phenyl}propanamide (hereinafter, referred to as the compound 39 of the present invention) was obtained from 0.91 g of 4-phenylbenzaldehyde and 0.7 g of 3-{3-methoxy-4-(2-propynyloxy)phenyl}propionic acid. 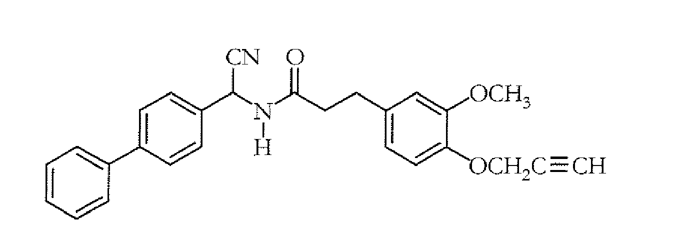 ¹H-NMR (CDCl₃, TMS) δ (ppm): 7.55-7.63 (4H, m), 7.43-7.49 (2H, m), 7.36-7.42 (3H, m), 6.95 (1H, d, J = 8.4 Hz), 6.70-6.74 (2H, m), 6.1.4 (1H, d, J = 8.2 Hz), 5. 80 (1H, d, J = 8.0 Hz), 4.72 (2H, d, J = 2.4 Hz), 3.82 (3H, s), 2.97 (2H, t, J = 7.4 Hz), 2.49-2.65 (2H, m), 2.45 (1H, t, J = 2.4 Hz)

### Preparation Example 40

In the same way as Preparation Example 16, 0.55 g of N-{1-(4-chlorophenyl)-1-cyanoethyl}-3-{3-methoxy-4-(2-propynyloxy)phenyl}propanamide (hereinafter, referred to as the compound 40 of the present invention) was obtained from 0.50 g of 2-amino-2-(4-chlorophenyl)propionitrile hydrochloride and 0.49 g of 3-{3,4-bis(2-propynyloxy)phenyl}propionic acid. 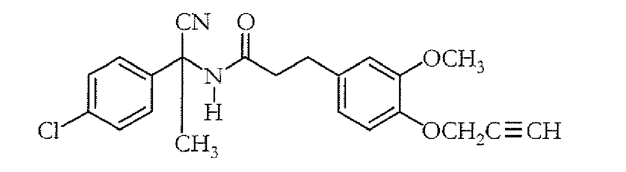 ¹H-NMR (CDCl₃, TMS) δ (ppm): 7.31 (2H, d, J = 8.6 Hz), 7.25 (2H, d, J = 8.6 Hz), 6.96 (1H, d, J = 8.7 Hz), 6.69-6.71 (2H, m), 6.02 (1H, s), 4.75 (1H, d, J = 2.4 Hz), 3.80 (3H, s), 2.89 (2H, t, J = 7.1 Hz), 2.48-2.53 (3H, m), 1.78 (3H)

### Preparation Example 41

In the same way as Preparation Example 30, 250 mg of N-{1-(indan-5-yl)-1-cyanomethyl}-3-{3-methoxy-4-(2-propynyloxy)phenyl}propanamide (hereinafter, referred to as the compound 41 of the present invention) was obtained from 731 mg of indane-5-carbaldehyde and 0.7 g of 3-{3-methoxy-4-(2-propynyloxy)phenyl}propionic acid. 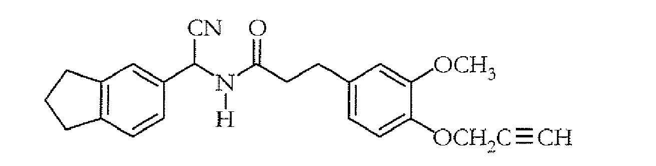 ¹H-NMR (CDCl₃ , TMS) δ (ppm): 7.20-7.25 (2H, m), 7.05-7.10 (1H, m), 6.94 (1H, d, J = 7.9 Hz), 6.68-6.74 (2H, m), 6.01 (1H, d, J = 8.2 Hz), 5.74 (1H, d, J = 7.4 Hz), 4.72 (2H, d, J = 2.4 Hz), 3.82 (3H, s), 2.85-2.98 (6H, m), 2.45-2.60 (3H, m), 2.03-2.14 (2H, m)

### Preparation Example 42

0.23 g of 2-amino-2-(4-bromophenyl)acetonitrile hydrochloride, 0.20 g of 2-fluoro-3-{3-methoxy-4-(2-propynyloxy)phenyl}propionic acid, 0.18 g of WSC, 10 ml of dimethylformamide and 1 ml of pyridine were mixed and stirred at room temperature for 3 hours. Water was then added to the reaction mixture, and the mixture was extracted with ethyl acetate. The organic layer was washed successively with 5% hydrochloric acid twice, water, an aqueous saturated sodium hydrogen carbonate solution and an aqueous saturated sodium chloride solution, dried over magnesium sulfate, and then concentrated under reduced pressure. The residue was subjected to silica gel column purification to obtain 0.27 g of N-{1-(4-bromophenyl)-1-cyanomethyl}-2-fluoro-3-{3-methoxy-4-(2-propynyloxy)phenyl}propanamide (hereinafter, referred to as the compound 42 of the present invention). 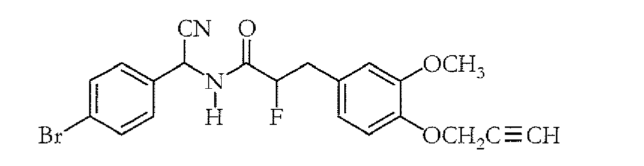 ¹H-NMR (CDCl₃, TMS) δ (ppm): 7.56 (1H, d, J = 8.5 Hz), 7.49 (1H, d, J = 8.5 Hz), 7.29 (1H, d, J = 8. 5 Hz), 6.88-7.01 (2H, m), 6.71-6.79 (2H, m), 6.54-6.52 (1H, m), 5.98-6.01 (1H, m), 5.11-5.35 (1H, m), 4.75-4.76 (2H, m), 3.87 (1.5H, s), 3.79 (1.5H, s), 3.10-3.34 (2H, m), 2.50-2.51 (1H, m)

### Preparation Example 43

Using 0.59 g of 2-amino-2-(4-methylphenyl)acetonitrile hydrochloride and 0.50 g of 2-fluoro-3-{3-methoxy-4-(2-propynyloxy)phenyl}propionyl chloride, 0.34 g of N-{1-(4-methylphenyl)-1-cyanomethyl}-2-fluoro-3-{3-methoxy-4-(2-propynyloxy)phenyl}propanamide (hereinafter, referred to as present compound 43 was obtained in the same way as Preparation Example 4. 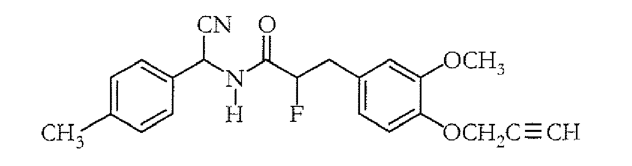 ¹H-NMR (CDCl₃, TMS) δ (ppm): 7.19-7.30 (2H, m), 7.15 (1H, d, J = 8.0 Hz) , 6.91-7.02 (2H, m), 6.73-6.80 (2H, m), 6.52-6.60 (1H, m), 5.95-5.98 (1H, m), 5.07-5.32 (1H, m), 4.72-4.75 (2H, m), 3.87 (1.5H, s), 3.79 (1.5H, s), 3.11-3.34 (2H, m), 2.48-2.50 (1H, m), 2.37 (1.5H, s), 2.35 (1.5H, s)

### Preparation Example 44

In the same way as Preparation Example 30, 0.59 g of N-{1-(4-difluoromethoxyphenyl)-1-cyanomethyl}-3-{3-methoxy-4-(2-propynyloxy)phenyl}propanamide (hereinafter, referred to as the compound 44 of the present invention) was obtained from 0.55 g of 4-difluoromethoxybenzaldehyde and 0.50 g of 3-{3-methoxy-4-(2-propynyloxy)phenyl}propionic acid. 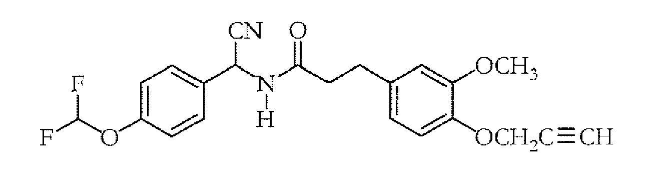 ¹H-NMR (CDCl₃, TMS) δ (ppm): 7.29 (2H, d, J = 8.5 Hz), 7.13 (2H, d, J = 8.5 Hz), 6.94 (1H, d, J = 8.8 Hz), 6.70-6.72 (2H, m), 6.55 (1H, t, J = 73 Hz), 6.08 (1H, d, J = 8.3 Hz), 5.91 (1H, br.d, J = 8.3 Hz), 4.72 (2H, d, J = 2.2 Hz), 3.80 (3H, s), 2.95 (2H, t, J = 7.3 Hz), 2.47-2.62 (3H, m)

### Preparation Example 45

0.59 g of 4-bromobenzaldehyde, 0.46 ml of trimethylsilyl cyanide and 34 mg of zinc iodide were mixed and stirred at room temperature for 20 minutes. Thereto 4 ml of a 40% solution of methylamine in methanol was added and stirred at 40°C for 2 hours. The mixture was allowed to cool to around room temperature and then concentrated under reduced pressure. To the resulting residue were added 0.50 g of 3-{3-methoxy-4-(2-propynyloxy)phenyl}propionic acid, 10 mg of dimethylformamide and 1 ml of pyridine, and then was added 0.45 g of WSC. The mixture was stirred at room temperature for 4 hours. Water was then added to the reaction mixture, and the mixture was extracted with ethyl acetate. The organic layer was washed successively with 5% hydrochloric acid thrice, water, an aqueous saturated sodium hydrogen carbonate solution and an aqueous saturated sodium chloride solution, dried over magnesium sulfate, and then concentrated under reduced pressure. The residue was subjected to silica gel column purification to obtain 0.24 g of N-{1-(4-bromophenyl)-1-cyanomethyl}-N-methyl-3-{3-methoxy-4-(2-propynyloxy)phenyl}propanamide (hereinafter, referred to as the compound 45 of the present invention). 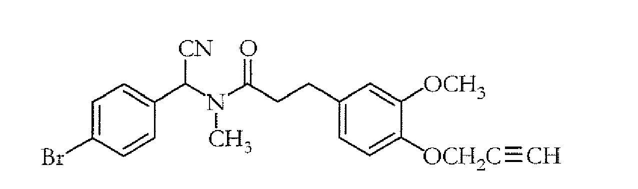 ¹H-NMP, (CDCl₃, TMS) δ (ppm): 7.54 (2H, d, J8.5 Hz), 7.23 (2H, d, J = 8.5 Hz), 7 . 81 (1H, s), 6.97 (1H, d, J = 8.2 Hz), 6 . 78 (1H, d, J = 1.9 Hz), 6.75 (1H, dd, J = 8.2 Hz, 1.9 Hz), 4.75 (2H, d, J = 2.4 Hz), 3.86 (3H, s), 2.99 (2H, t, J = 7.7 Hz), 2.81 (3H, s), 2.70 (2H, t, J = 7.7 Hz), 2.50 (1H, t, J = 2.4 Hz)

### Preparation Example 46

In the same way as Preparation Example 30, 0.60 g of N-{1-(4-iodophenyl)-1-cyanomethyl}-3-{3-methoxy-4-(2-propynyloxy)phenyl}propanamide (hereinafter, referred to as the compound 46 of the present invention) was obtained from 1.0 g of 4-iodobenzaldehyde and 0.50 g of 3-{3-methoxy-4-(2-propynyloxy)phenyl}propionic acid. 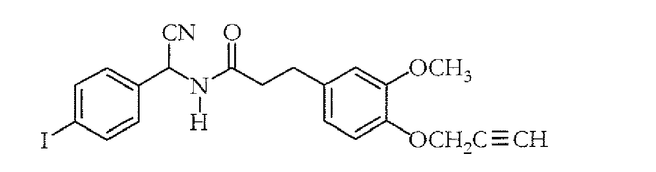 ¹H-NMR (CDCl₃, TMS) δ (ppm): 7.71 (2H, d, J = 8.4 Hz), 7.02 (2H, d, J = 8.4 Hz), 6.93 (1H, d, J = 7.7 Hz), 6.68-6.70 (2H, m), 6.02-6.07 (2H, m), 4.73 (2H, d, J = 2.4 Hz), 3.81 (3H, s), 2.93 (2H, t, J = 7.2 Hz), 2.46-2.61 (3H, m)

### Preparation Example 47

In the same way as Preparation Example 30, 0.28 g of N-{1-(4-butylphenyl-1-cyanomethyl)-3-{3-methoxy-4-(2-propynyloxy)phenyl}propanamide (hereinafter, referred to as the compound 47 of the present invention) was obtained from 0.52 g of 4-n-butylbenzaldehyde and 0.50 g of 3-{3-methoxy-4-(2-propynyloxy)phenyl}propionic acid. 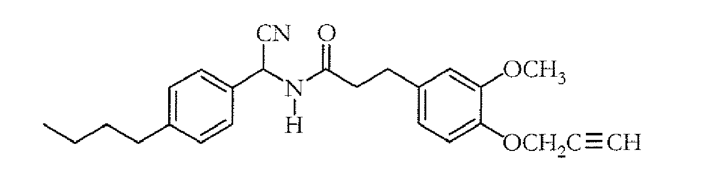 ¹H-NMR (CDCl₃, TMS) δ (ppm): 7.23 (2H, d, J = 7.9 Hz), 7.19 (2H, d, J = 7.9 Hz), 6.94 (1H, d, J = 7.9 Hz), 6.69-6.72 (2H, m), 6.03 (1H, d, J = 8.3 Hz), 5.85 (1H, br.d, J = 8.3 Hz), 4.72 (2H, d, J = 2.4 Hz), 3.82 (3H, s), 2.95 (2H, t, J = 7.5 Hz), 2.61 (2H, t, J = 7.5 Hz), 2.46-2.55 (3H, m), 1.54-1.62 (2H, m), 1.30-1.39 (2H, m), 0.93 (3H, t, J = 7.1 Hz)

### Preparation Example 48

In the same way as Preparation Example 30, 1.2 g of N-[1-{4-(trimethylsilylethynyl)phenyl}-1-cyanomethyl]-3-{3-methoxy-4-(2-propynyloxy)phenyl}propanamide (hereinafter, referred to as the compound 48 of the present invention) was obtained from 2.1 g of 4-(trimethylsilylethylnyl)benzaldehyde and 1.0 g of 3-{3-methoxy-4-(2-propynyloxy)phenyl}propionic acid. 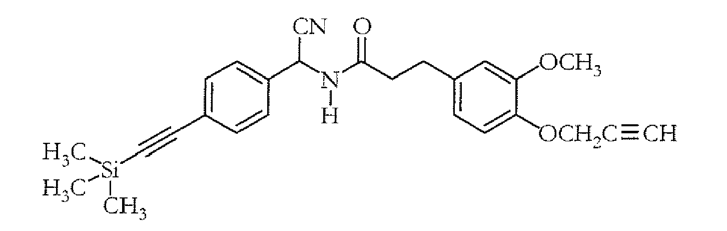 ¹H-NMR (CDCl₃, TMS) δ (ppm): 7.46 (2H, d, J = 8.3 Hz), 7.22 (2H, d, J = 8.3 Hz), 6.92 (1H, d, J = 8.3 Hz), 6.67-6.72 (2H, m), 6.09 (1H, d, J = 8.3 Hz), 6.02 (1H, br.d, J = 8.3 Hz), 4.72 (2H, d, J = 2.8 Hz), 3.81 (3H, s), 2.94 (2H, t, J = 7.1 Hz), 2.47-2.61 (3H, m), 0.26 (9H, s)

### Preparation Example 49

0.77 g of N-[1-{4-(trimethylsilylethylnyl)phenyl}-1-cyanomethyl]-3-{3-methoxy-4-(2-propynyloxy)phenyl}propanamide, 1.0 g of potassium carbonate and 10 ml of methanol were stirred under reflux for 3 hours. The mixture was cooled and then filtered. The solvent was distilled off under reduced pressure. The residue was purified by a silica gel column to obtain 0.1 g of N-{1-(4-ethylnylphenyl)-1-cyanomethyl}-3-{3-methoxy-4-(2-propynyloxy)phenyl}propanamide (hereinafter, referred to as the compound 49 of the present invention). 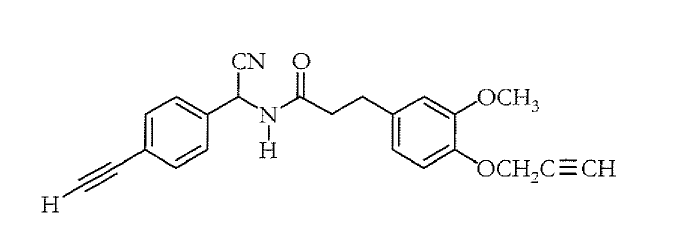 ¹H-NMR (CDCl₃, TMS) δ (ppm): 7.50 (2H, d, J = 8.3 Hz), 7.26 (2H, d, J = 8.3 Hz), 6.93 (1H, d, J = 8.3 Hz), 6.69-6.72 (2H, m), 6.11 (1H, d, J = 8.7 Hz), 5.98 (1H, br.d, J = 8.3 Hz), 4.73 (2H, d, J = 2.4 Hz), 3.82 (3H, s), 3.14 (1H, s), 2.95 (2H, t, J = 7.1 Hz), 2.48-2.62 (3H, m)

### Preparation Example 50

2.66 g of 4-diethoxymethylbenzaldehyde, 1.82 ml of trimethylsilyl cyanide and 0.14 g of zinc iodide were mixed and stirred at room temperature for 20 minutes. Thereto 20 ml of a 10% solution of ammonia in methanol was added and stirred at 40°C for 2 hours. The mixture was allowed to cool to around room temperature and then concentrated under reduced pressure. To the resulting residue were added 2.0 g of 3-{3-methoxy-4-(2-propynyloxy)phenyl}propionic acid, 50 ml of dimethylformamide and 3 ml of pyridine, and then was added 1.8 g of WSC. The mixture was stirred at room temperature for 4 hours. To the resulting mixture, 5 ml of 5% hydrochloric acid was then added and stirred at room temperature for 1 hour. Water was then added, and the mixture was extracted with ethyl acetate. The organic layer was washed successively with 5% hydrochloric acid thrice, water, an aqueous saturated sodium hydrogen carbonate solution and an aqueous saturated sodium chloride solution, dried over magnesium sulfate, and then concentrated under reduced pressure. To 1.8 g of the resulting residue, 0.09 g of sodium borohydride and 30 ml of methanol were added and stirred at room temperature for 1 hour. An aqueous saturated ammonium chloride solution was then added to the mixture, followed by the extraction with ethyl acetate. The organic layer was washed successively with an aqueous saturated sodium hydrogen carbonate solution and an aqueous saturated sodium chloride solution, dried over magnesium sulfate, and then concentrated under reduced pressure. The residue was subjected to silica gel column purification to obtain 0.79 g of N-[1-(4-{hydroxymethyl}phenyl)-1-cyanomethyl]-3-{3-methoxy-4-(2-propynyloxy)phenyl}propanamide (hereinafter, referred to as the compound 50 of the present invention) and 0.32 g of N-[1-4-{(ethoxy)cyanomethyl}phenyl]-1-cyanomethyl}-3-{3-methoxy-4-(2-propynyloxy)phenyl}propanamide (hereinafter, referred to as the compound 51 of the present invention). The compound 50 of the present invention 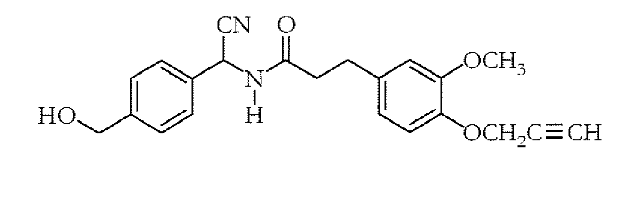 ¹H-NMR (CDCl₃, TMS) δ (ppm): 7.70 (2H, d, J = 8.3 Hz), 7.26 (2H, d, J = 8.3 Hz), 6.92 (1H, d, J = 8.3 Hz), 6.68-6.71 (2H, m), 6.06 (1H, d, J = 8.3 Hz), 5.89 9 (1H, br.d, J = 8.3 Hz), 4.70-4.72 (4H, m), 3.80 (3H, s), 2.94 (2H, t, J = 7.1 Hz), 2.46-2.62 (3H, m), 1.96 (1H, br.t)
The compound 51 of the present invention 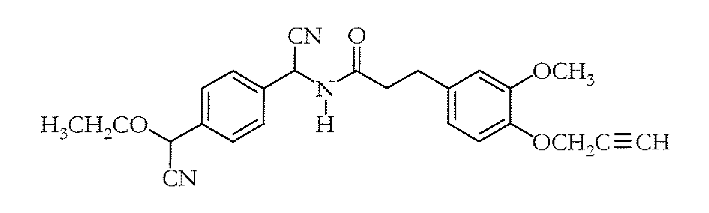 ¹H-NMR (CDCl₃, TMS) δ (ppm): 7.53 (2H, d, J = 8.3 Hz) , 7.37 (2H, d), 6.95 (1H, d, J = 8.5 Hz), 6.70-6.72 (2H, m), 6.14 (1H, d, J = 8.3 Hz), 5.88 (1H, br.d, J = 8.3 Hz), 5.25 (1H, s), 4.73 (2H, d, J = 2.8 Hz), 3.82-3.90 (4H, m), 3.64-3.71 (1H, m), 2.96 (2H, t, J = 7.1 Hz) , 2.49-2.60 (3H, m), 1.32 (3H, t, J = 6.7 Hz)

### Preparation Example 51

In the same way as Preparation Example 30, 0.49 g of N-{1-(4-cyclopropylphenyl)-1-cyanomethyl}-3-{3-methoxy-4-(2-propynyloxy)phenyl}propanamide (hereinafter, referred to as the compound 52 of the present invention) was obtained from 0.47 g of 4-cyclopropylbenzaldehyde and 0.50 g of 3-{3-methoxy-4-(2-propynyloxy)phenyl}propionic acid. 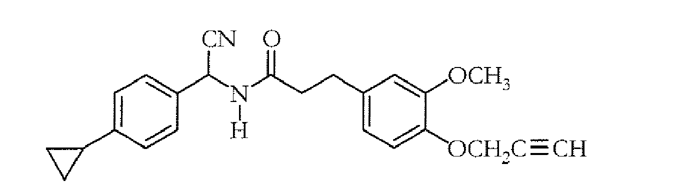 ¹H-NMR (CDCl₃, TMS) δ (ppm): 7.19 (2H, d, J = 8.3 Hz), 7.07 (2H, d, J = 8.3 Hz), 6.94 (1H, d, J = 7.9 Hz), 6.70-6.72 (2H, m), 6.02 (1H, d, J = 7.9 Hz), 5.78 (1H, br.d, J = 8.7 Hz), 4.73 (2H, d, J = 2.4 Hz), 3.82 (3H, s), 2.94 (2H, t, J = 7.5 Hz), 2.45-2.59 (3H, m), 1.86-1.93 (1H, m), 0.98-1.03 (2H, m), 0.68-0.72 (2H, m)

### Preparation Example 52

In the same way as Preparation Example 30, 0.29 g of N-{1-(4-chloro-3-fluorophenyl)-1-cyanomethyl}-3-{3-methoxy-4-(2-propynyloxy)phenyl}propanamide (hereinafter, referred to as the compound 53 of the present invention) was obtained from 0.51 g of 4-chloro-3-fluorobenzaldehyde and 0.50 g of 3-{3-methoxy-4-(2-propynylxoy)phenyl}propionic acid. 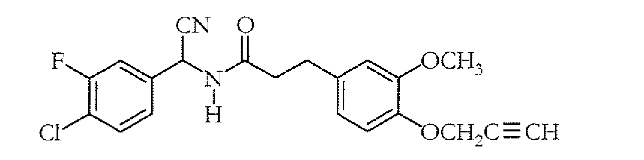 ¹H-NMR (CDCl₃, TMS) δ (ppm): 7.43 (1H, t, J = 7.9 Hz), 7.15 (1H, dd, J = 9.1 Hz, 2.0 Hz), 7.02-7.04 (1H, m), 6.95 (1H, d, J = 7.9 Hz), 6.70-6.72 (2H, m), 6.12 (1H, d, J = 8.7 Hz), 5.91 (1H, br.d, J = 8.7 Hz), 4.74 (2H, d, J = 2.4 Hz), 3.83 (3H, s), 2.96 (2H, t, J = 7.5 Hz), 2.52-2.64 (2H, m), 2.49 (1H, t, J = 2.4 Hz)

### Preparation Example 53

In the same way as Preparation Example 30, 0.53 g of N-{1-(4-tert-butylphenyl)-1-cyanomethyl}-3-{3-methoxy-4-(2-propynyloxy)phenyl}propanamide (hereinafter, referred to as the compound 54 of the present invention) was obtained from 0.52 g of 4-t-butylbenzaldehyde and 0.50 g of 3-{3-methoxy-4-(2-propynyloxy)phenyl}propionic acid. 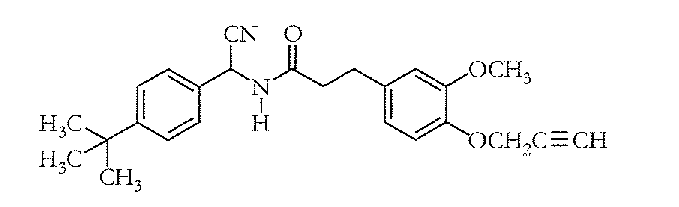 ¹H-NMR (CDCl₃, TMS) δ (ppm): 7.41 (2H, d, J = 8.3 Hz), 7.26 (2H, d, J = 8.3 Hz), 6.95 (1H, d, J = 7.9 Hz), 6.70-6.73 (2H, m), 6.04 (1H, d, J = 8.3 Hz), 5.88 (1H, br.d, J = 8.3 Hz), 4.72 (2H, d, J = 2.8 Hz), 3.82 (3H, s), 2.95 (2H, t, J = 7.1 Hz), 2.46-2.59 (3H, m), 1.31 (9H, s)

### Preparation Example 54

In the same way as Preparation Example 30, 0.48 g of N-{1-(4-trifluoromethylthiophenyl)-1-cyanomethyl}-3-{3-methoxy-4-(2-propynyloxy)phenyl}propanamide (hereinafter, referred to as the compound 55 of the present invention) was obtained from 0.62 g of 4-trifluoromethylthiobenzaldehyde and 0.50 g of 3-{3-methoxy-4-(2-propynyloxy)phenyl}propionic acid. 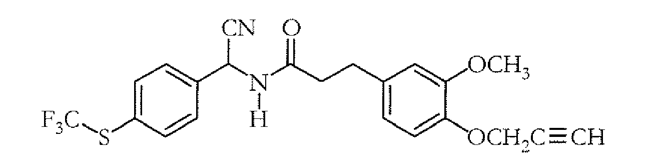 ¹H-NMR (CDCl₃, TMS) δ (ppm): 7.68 (2H, d, J = 8.3 Hz), 7.36 (2H, d, J = 8.3 Hz), 6.96 (1H, d, J = 7.9 Hz), 6.71-6.73 (2H, m), 6.18 (1H, d, J = 8.3 Hz), 5.87 (1H, br.d, J = 8.3 Hz), 4.74 (2H, d, J = 2.4 Hz), 3.83 (3H, s), 2.97 (2H, t, J = 7.1 Hz), 2.50-2.65 (2H, m), 2.49 (1H, t, J = 2.4 Hz), 1.55 (3H, s)

### Preparation Example 55

In the same way as Preparation Example 30, 0.48 g of N-{1-(3-trifluoromethylphenyl)-1-cyanomethyl}-3-{3-methoxy-4-(2-propynyloxy)phenyl}propanamide (hereinafter, referred to as the compound 56 of the present invention) was obtained from 0.56 g of 3-trifluoromethylbenzaldehyde and 0.50 g of 3-{3-methoxy-4-(2-propynyloxy) phenyl}propionic acid. 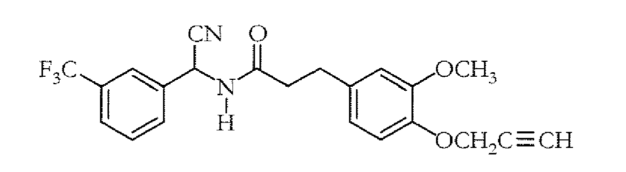 ¹H-NMR (CDCl₃, TMS) δ (ppm): 7.67 (2H, m), 7.55 (1H, t, J = 8.3 Hz), 7.48 (1H, d, J = 7.5 Hz), 6.95 (1H, d, J = 7.9 Hz), 6.70-6.73 (2H, m), 6.21 (1H, d, J = 8.3 Hz), 5.90 (1H, br.d, J = 8.7 Hz), 4.73 (2H, d, J = 2.4 Hz), 3.83 (3H, s), 2.97 (2H, t, J = 7.5 Hz), 2.54-2.64 (2H, m), 2.48 (1H, t, J = 2.4 Hz)

### Preparation Example 56

In the same way as Preparation Example 30, 0.43 g of N-{1-(1,1,2,2-tetrafluoroethoxy)phenyl}-1-cyanomethyl}-3-{3-methoxy-4-(2-propynyloxy)phenyl}propanamide (hereinafter, referred to as the compound 57 of the present invention) was obtained from 0.71 g of 4-(1,1,2,2-tetrafluoroethoxy)benzaldehyde and 0.50 g of 3-{3-methoxy-4-(2-propynyloxy)phenyl}propionic acid. 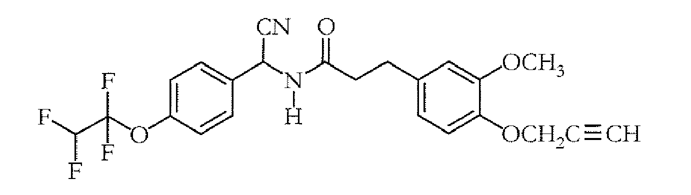 ¹H-NMR (CDCl₃, TMS) δ (ppm) : 7.33 (1H, d, J = 8.3 Hz), 7.24 (1H, d, J = 8.7 Hz), 6.95 (1H, d, J = 8.3 Hz), 6.70-6.73 (2H, m), 6.12 (1H, d, J = 8.3 Hz), 5.92 (1H, tt, J = 15 Hz, 2.4 Hz), 5.85 (1H, br.d, J = 8.3 Hz), 4.73 (2H, d, J = 2.4 Hz), 3.83 (3H, s), 2.96 (2H, t, J = 7.1 Hz), 2.51-2.64 (2H, m), 2.48 (1H, t, J = 2.4 Hz)

### Preparation Example 57

A mixture of 0.21 g of 2-amino-2-(4-chlorophenyl)acetonitrile hydrochloride, 0.25 g of WSC, 0.3 g of 3-{5-fluoro-3-methoxy-4-(2-propynyloxy)phenyl}propionic acid, 10 ml of dimethylformamide and 1 ml of pyridine was stirred at 50°C for 4 hours. Water was then added, and the mixture was extracted with ethyl acetate. The organic layer was washed successively with 5% hydrochloric acid, water, an aqueous saturated sodium hydrogen carbonate solution and an aqueous saturated sodium chloride solution, dried over magnesium sulfate, and then concentrated under reduced pressure. The residue was subjected to silica gel column purification to obtain 0.15 g of N-{1-(4-chlorophenyl)-1-cyanomethyl}-3-(5-fluoro-3-methoxy-4-(2-propynyloxy)phenyl)propanamide (hereinafter, referred to as the compound 58 of the present invention). 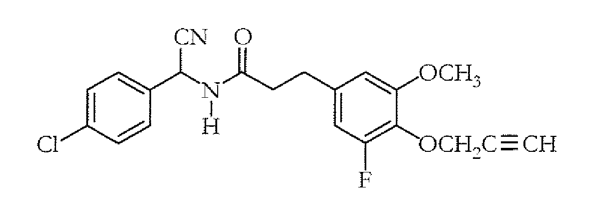 ¹H-NMR (CDCl₃, TMS) δ (ppm) : 7.37-7.40 (2H, m), 7.29-7.31 (2H, m), 6.53-6.61 (3H, m), 6.09 (1H, d, J = 8.7 Hz), 5.95 (1H, br.d, J = 8.3 Hz), 4.73 (2H, d, J = 2.4 Hz), 3.85 (3H, s), 2.94 (2H, t, J = 7.1 Hz), 2.50-2.69 (3H, m)

### Preparation Example 58

In the same way as Preparation Example 30, 0.35 g of N-{1-(3-fluoro-4-methylphenyl)-1-cyanomethyl}-3-{3-methoxy-4-(2-propynyloxy)phenyl}propanamide (hereinafter, referred to as the compound 59 of the present invention) was obtained from 0.62 g of 3-fluoro-4-ethylbenzaldehyde and 0.50 g of 3-{3-methoxy-4-(2-propynyloxy)phenyl}propionic acid. 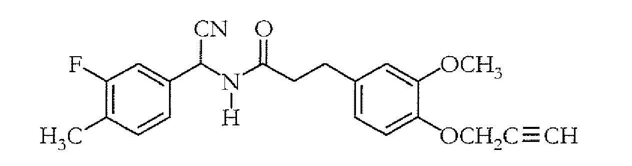 ¹H-NMR (CDCl₃, TMS) δ (ppm): 7.20 (1H, t, J = 7.5 Hz), 6.99 (2H, d, J = 8.7), 6.94 (1H, d, J = 7.5 Hz), 6.69-6.72 (2H, m), 6.96 (1H, d, J = 8.3 Hz), 5.90 (1H, br.d, J = 8.3 Hz), 4.72 (2H, d, J = 2.4 Hz), 3. 82 (3H, s), 2.95 (2H, t, J = 7.5 Hz), 2.50-2.61 (2H, m), 2.48 (1H, t, J = 2.4 Hz), 2.28 (3H, d, J = 1.6)

### Preparation Example 59

In the same way as Preparation Example 57, 0.15 g of N-{1-(6-chloro-4-methylphenyl)-1-cyanomethyl}-3-{3-methoxy-4-(2-propynyloxy)phenyl}propanamide (hereinafter, referred to as the compound 60 of the present invention) was obtained from 0.19 g of 2-amino-2-(4-chlorophenyl)acetonitrile hydrochloride, and 3-{6-chloro-3-methoxy-4-(2-propynyloxy)phenyl}propionic acid.
The compound 60 of the present invention 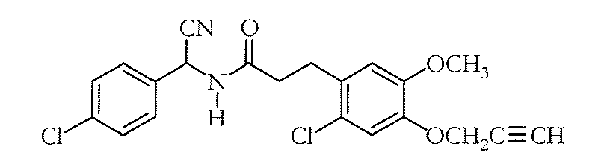 ¹H-NMR (CDCl₃, TMS) δ (ppm) : 7.36-7.38 (2H, m), 7.25-7.29 (2H, m), 7.00 (1H, s), 6.79 (1H, s), 6.08 (1H, d, J = 7.9 Hz), 5.89 (1H, br.d, J = 8.3 Hz), 4.73 (2H, d, J = 2.4 Hz), 3.82 (3H, s), 3.05 (2H, t, J = 7.1 Hz), 2.54-2.61 (2H, m), 2.53 (1H, t, J = 2.4 Hz)

### Preparation Example 60

In the same way as Preparation Example 30, 0.25 g of N-{1-(4-trimethylsilylphenyl)-1-cyanomethyl}-3-{3-methoxy-4-(2-propynyloxy)phenyl}propanamide (hereinafter, referred to as the compound 61 of the present invention) was obtained from 0.58 g of 4-trimethylsilylbenzaldehyde and 0.50 g of 3-{3-methoxy-4-(2-propynyloxy)phenyl}propionic acid. 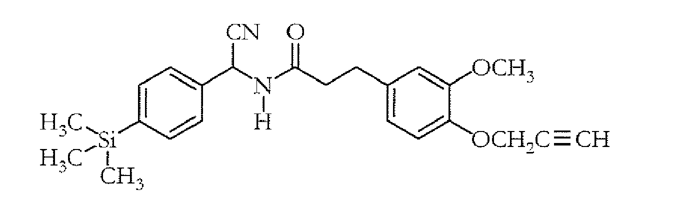 ¹H-NMR (CDCl₃ , TMS) δ (ppm): 7.55 (2H, d, J = 8.3 Hz), 7.31 (2H, d, J = 8.3 Hz), 6.95 (1H, d, J = 7.9 Hz), 6.70-6.73 (2H, m), 6.08 (1H, d, J = 8.3 Hz), 5.78 (1H, br.d, J = 7.9 Hz), 4.73 (2H, d, J = 2.4 Hz), 3.83 (3H, s), 2.96 (2H, t, J = 7.5 Hz), 2.51-2.58 (2H, m), 2.48 (1H, t, J = 2.4 Hz), 0.27 (9H, s)

### Preparation Example 61

By subjecting 0.64 g of N-{1-(4-chlorophenyl)-1-cyanomethyl}-3-(3-methoxy-4-(2-propynyloxy)phenyl)propanamide (the compound 1 of the present invention) to high performance liquid chromatography (column:CHIRALPAK AD (2 cmφ × 25cm), column temperature: room temperature, mobile phase: hexane/2-propanol/trifluoroacetic acid = 70/30/0.1, flow rate: 6 ml/min), the optical isomers were separated to obtain 0.27 g of (+)-N-{1-(4-chlorophenyl)-1-cyanomethyl}-3-(3-methoxyl-4-(2-propynyloxy)phenyl)propanamide (hereinafter, referred to as the compound 62 of the present invention) and 0.27 g of (-)-N-{1-(4-chlorophenyl)-1-cyanomethyl}-3-(3-methoxy-4-(2-propynyloxy)phenyl)propanamide (hereinafter, referred to as the compound 63 of the present invention). 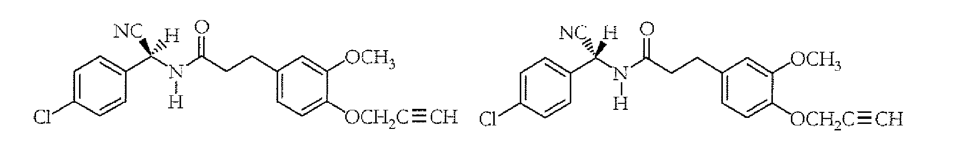 The compound 62 of the present invention
Specific rotation: [α]_{D}²² +6.3 degree (C1.0, chloroform)
¹H-NMR (CDCl₃, TMS) δ (ppm): 7.35-7.38 (2H, m), 7.22-7.24 (2H, m), 6.94 (1H, d, J = 7.8 Hz), 6.69-6.72 (2H, m), 6.08 (1H, d, J = 8.5 Hz), 5.87 (1H, br.d, J = 8.5 Hz), 4.73 (2H, d, J = 2.4 Hz), 3.82 (3H, s), 2.95 (2H, t, J = 7.3 Hz), 2.48-2.63 (3H, m)
The compound 63 of the present invention
Specific rotation: [α] _{D}²² -6.4 degree (C 1.0, chloroform)
¹H-NMR (CDCl₃, TMS) δ (ppm): 7. 35-7. 38 (2H, m), 7.22-7.24 (2H, m), 6.94 (1H, d, J = 7.8 Hz), 6.69-6.72 (2H, m), 6.08 (1H, d, J = 8.5 Hz), 5.87 (1H, br.d, J = 8.5 Hz), 4.73 (2H, d, J = 2.4 Hz), 3.82 (3H, s), 2.95 (2H, t, J = 7. 3 Hz), 2.48-2.63 (3H, m)

### Preparation Example 62

In the same way as Preparation Example 16, 0.34 g of N-{1-(4-bromophenyl)-1-cyanomethyl}-3-(3-methoxy-4-ethoxyphenyl)propanamide (hereinafter, referred to as the compound 64 of the present invention) was obtained from 0.36 g of 2-amino-2-(4-bromophenyl)acetonitrile hydrochloride and 0.30 g of 3-(3-methoxy-4-ethoxyphenyl)propionic acid. 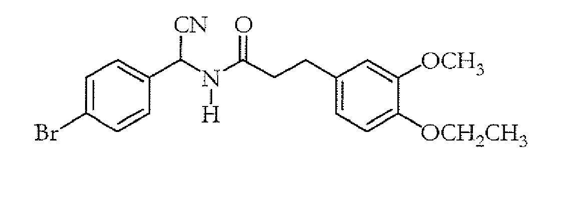 ¹H-NMR (CDCl₃, TMS) δ (ppm): 7.51 (2H, d, J = 8.7 Hz), 7.15 (2H, d, J = 8.7 Hz), 6.75 (1H, d, J = 7.9 Hz), 6.66-6.70 (2H, m), 6.07 (1H, d, J = 8.3 Hz), 5.79 (1H, br.d J = 8.3 Hz), 4.06 (2H, q, J = 7.1 Hz), 3.83 (3H, s), 2.94 (2H, t, J = 7.1 Hz), 2.47-2.63 (2H, m), 1.46 (3H, t, J = 7.1 Hz)

### Preparation Example 63

In the same way as Preparation Example 16, 0.24 g of N-{1-(4-methylphenyl)-1-cyanomethyl}-3-(3-methoxy-4-ethoxyphenyl)propanamide (hereinafter, referred to as the compound 65 of the present invention) was obtained from 0.27 g of 2-amino-2-(4-methylphenyl)acetonitrile hydrochloride and 0.30 g of 3-(3-methoxy-4-ethoxyphenyl)propionic acid. 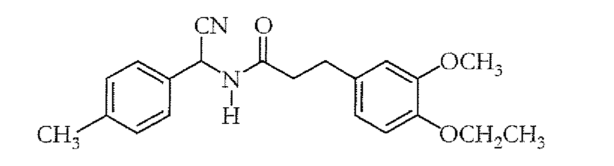 ¹H-NMR (CDCl₃, TMS) δ (ppm): 7.17-7.22 (4H, m), 6.77 (1H, d, J = 7.9 Hz), 6.67-6.70 (2H, m), 6.04 (1H, d, J = 7.9 Hz), 5.75 (1H, br.d, J = 7.9 Hz), 4.06 (2H, q, J = 7.1 Hz), 3.83 (3H, s), 2.94 (2H, t, J = 7.1 Hz), 2.46-2.59 (2H, m), 2.36 (3H, s), 1.45 (3H, t, J = 7.1 Hz)

Then, preparation of intermediates will be described as Reference Examples.

### Reference Example 1

50 g of 3-(4-hydroxy-3-methoxyphenyl)acrylic acid, 0.5 g of 5% palladium carbon, about 0.05 g of 36% hydrochloric acid, 250 ml of ethanol and 100 ml of tetrahydrofuran were mixed and stirred under a hydrogen atmosphere. After absorption of a hydrogen gas was stopped, the reaction mixture was filtered. The filtrate was concentrated under reduced pressure to obtain 52 g of 3-(4-hydroxy-3-methoxyphenyl)propionic acid. 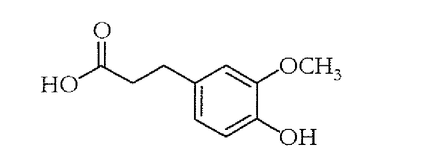 ¹H-NMR (CDCl₃, TMS) δ (ppm): 6.83 (1H, dd, J = 7.3 Hz, 0.8 Hz), 6.81-6.70 (2H, m), 3.86 (3H, s), 2.88 (2H, t, J = 7.6 Hz), 2.65 (2H, t, J = 7.6 Hz)

### Reference Example 2

50 g of 3-(4-hydroxy-3-methoxyphenyl)propionic acid, 50 ml of propargyl bromide, 88 g of potassium carbonate and 500 ml of acetonitrile were mixed and stirred at 80°C for 3 hours. After the reaction mixture was allowed to cool to around room temperature, ethyl acetate was added and the mixture was filtered. The filtrate was concentrated under reduced pressure to obtain 67 g of 2-propynyl 3-{3-methoxy-4-(2-propynyloxy)phenyl}propionate. 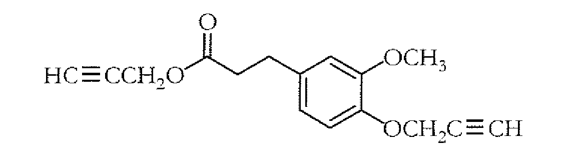 ¹H-NMR (CDCl₃, TMS) δ (ppm): 6.96 (1H, d, J = 7.8 Hz), 6.68-6.75 (2H, m), 4.73 (2H, d, J = 2.2 Hz), 4.68 (2H, d, J = 2.2 Hz), 3.87 (3H, s), 2.93 (2H, t, J = 7.3 Hz), 2.67 (2H, t, J = 7.3 Hz), 2.47-2.50 (2H, m)

### Reference Example 3

67 g of 2-propynyl 3-{3-methoxy-4-(2-propynyloxy)phenyl}propionate, 8.08 g of lithium hydroxide, 400 ml of tetrahydrofuran and 200 ml of water were mixed and stirred at 65°C for 3 hours. After the reaction mixture was allowed to cool to around room temperature, water was added and the mixture was concentrated under reduced pressure. To the residue was added 5% hydrochloric acid, and the mixture was extracted with chloroform thrice. The organic layer was dried over magnesium sulfate and then concentrated under reduced pressure. The residue was washed with hexane to obtain 51 g of 3-{3-methoxy-4-(2-propynyloxy)phenyl}propionic acid. 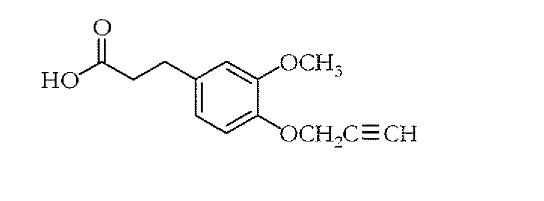 ¹H-NMR (CDCl₃, TMS) δ (ppm): 6.96 (1H, d, J = 8.2 Hz), 6.73-6.75 (2H, m), 4.73 (2H, d, J = 2. 4 Hz), 3.85 (3H, s), 2.91 (2H, t, J = 8 Hz), 2.67 (2H, t, J = 8 Hz), 2.49 (1H, t, J = 2.4 Hz)

### Reference Example 4

12.7 g of 3-{3-methoxy-4-(2-propynyloxy)phenyl}propionic acid, 4.3 ml of thionyl chloride, 100 ml of toluene and about 0.05 g of N,N-dimethylformamide were mixed and stirred at 80°C for 30 minutes. The reaction mixed was allowed to cool to around room temperature, and then concentrated under reduced pressure to obtain 14.6 g of 3-{3-methoxy-4-(2-propynyloxy)phenyl}propionyl chloride. 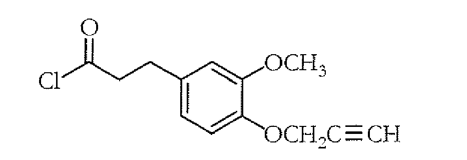 ¹H-NMR (CDCl₃, TMS) δ (ppm): 6.97 (1H, d, J = 8.8 Hz), 6.72-6.74 (2H, m), 4.73 (2H, d, J = 2.4 Hz), 3.87 (3H, s), 3.19 (2H, t, J = 7.2 Hz), 2.99 (2H, t, J = 7.2 Hz), 2.49 (1H, t, J = 2.4 Hz)

### Reference Example 5

25 g of 3-ethoxy-4-hydroxybenzaldehyde, 27 g of benzyl bromide, 25 g of potassium carbonate and 250 ml of acetonitrile were mixed and stirred under reflux for 3 hours. After the reaction mixture was allowed to cool to room temperature, ethyl acetate was added and the mixture was filtered. The resulting filtrate was concentrated under reduced pressure. The residue was washed with tert-butyl methyl ether and hexane to obtain 36 g of 4-benzyloxy-3-ethoxybenzaldehyde. 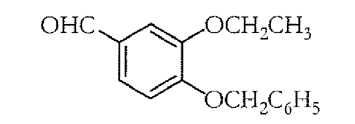 ¹H-NMR (CDCl₃ , TMS) δ (ppm): 9.82 (1H, s), 7.28-7.44 (7H, m), 6.98 (1H, d, J = 8.2 Hz), 5.24 (2H, s), 4.18 (2H, q, J = 7.0 Hz), 1.49 (3H, t, J = 7.0 Hz)

### Reference Example 6

19 g of ethyl diethylphosphonoacetate and 400 ml of tetrahydrofuran were mixed, and thereto 3.3 g of sodium hydride (content 60%) was added under ice-cooling. The mixture was stirred for 10 minutes. Thereto a mixture of 20 g of 4-benzyloxy-3-ethoxybenzaldehyde and 50 ml of tetrahydrofuran was gradually added under ice-cooling and then stirred at room temperature for hour. After water was added, the resulting mixture was concentrated under reduced pressure and then extracted with ethyl acetate. The resulting organic layer was washed successively with water, a pH 6.8 buffer and an aqueous saturated sodium chloride solution, dried over magnesium sulfate, and then concentrated under reduced pressure. The resulting residue was washed with hexane to obtain 25 g of ethyl 3-{3-ethoxy-4-(benzyloxy)phenyl}acrylate. 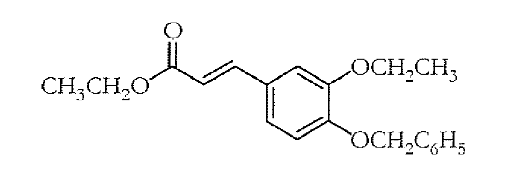 ¹H-NMR (CDCl₃, TMS) δ (ppm): 7.59 (1H, d, J = 16 Hz), 7.28-7.44 4 (5H, m), 7. 07 (1H, d, J = 2.0 Hz), 7.02 (1 H, dd, J = 8.3 Hz, 2.0 Hz), 6.86 (1H, d, J = 8.3 Hz), 6.28 (1H, d, J = 16 Hz), 5.18 (2H, s), 4.28 (2H, q, J = 7.1 Hz), 4.31 (2H, q, J = 6.8 Hz), 1.47 (3H, t, J = 6.8 Hz), 1.33 (3H, t, J = 7.1 Hz)

### Reference Example 7

23 g of ethyl 3-{3-ethoxy-4-(benzyloxy)phenyl}acrylate, 0.2 g of 5% palladium carbon, about 0.04 g of 36% hydrochloric acid, 120 ml of ethanol and 100 ml of tetrahydrofuran were mixed and stirred under a hydrogen atmosphere. After absorption of a hydrogen gas was stopped, the reaction mixture was filtered. The resulting filtrate was concentrated under reduced pressure, and the residue was washed with hexane to obtain 17 g of ethyl 3-(4-hydroxy-3-ethoxyphenyl)propionate. Ethyl 3-(4-hydroxy-3-ethoxyphenyl)propionate 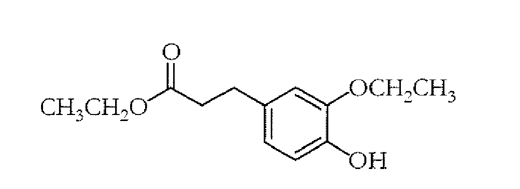 ¹H-NMR (CDCl₃, TMS) δ (ppm) : 6.83 (1H, d, J = 8.0 Hz), 6.66-6.70 (2H, m), 5.55 (1H, s), 4.01-4.15 (4H, m), 2.86 (2H, t, J = 7.8 Hz), 2.57 (2H, t, J = 7.8 Hz), 1.43 (3H, t, J = 6.9 Hz), 1.23 (3H, t, J = 7.2 Hz)

### Reference Example 8

16.2 g of ethyl 3-(4-hydroxy-3-ethoxyphenyl)propionate, 7.3 ml of propargyl bromide, 13.2 g of potassium carbonate and 160 ml of acetonitrile were mixed and stirred at 80°C for 2 hours. After the reaction mixture was allowed to cool to around room temperature, ethyl acetate was added and the mixture was filtered. The resulting filtrate was concentrated under reduced pressure to obtain 18.8 g of ethyl 3-{3-ethoxy-4-(2-propynyloxy)phenyl}propionate. 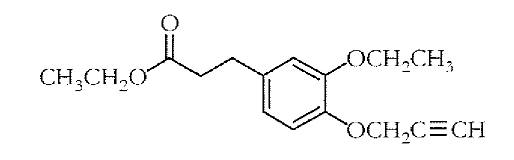 ¹H-NMR (CDCl₃, TMS) δ (ppm): 6.96 (1H, d, J = 8.0 Hz), 6.74 (1H, d, J = 1.9 Hz), 6.72 (1H, dd, J = 8.0 Hz, 1.9 Hz), 4.73 (2H, d, J = 2.4 Hz), 4.12 (2H, q, J = 7.0 Hz) , 4.07 (2H, q, J = 7.0 Hz), 2.89 (2H, t, J = 7.5 Hz), 2.59 (2H, t, J = 7.5 Hz), 2.48 (1H, t, J = 2.4 Hz), 1.44 (3H, t, J = 7.0 Hz), 1.23 (3H, t, J = 7.0 Hz)

### Reference Example 9

28.7 g of ethyl 3-{3-ethoxy-4-(2-propynyloxy)phenyl}propionate, 2.3 g of lithium hydroxide, 180 ml of tetrahydrofuran and 60 ml of water were mixed and stirred at 65°C for 3 hours. After the reaction mixture was allowed to cool to around room temperature, water was added and the mixture was concentrated under reduced pressure. The mixture was washed with tert-butyl methyl ether. To the resulting aqueous layer was added 5% hydrochloric acid, followed by extraction with ethyl acetate. The organic layer was washed with an aqueous saturated sodium chloride solution, dried over magnesium sulfate, and then concentrated under reduced pressure. The residue was washed with hexane to obtain 15.5 g of 3-{3-ethoxy-4-(2-propynyloxy)phenyl}propionic acid. 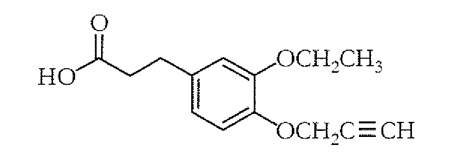 ¹H-NMR (CDCl₃, TMS) δ (ppm) : 6.97 (1H, d, J = 8.2 Hz), 6.74 (1H, d, J = 1.9 Hz), 6.73 (1H, dd, J = 8.2 Hz, 1.9 Hz), 4.73 (2H, d, J = 2.4 Hz), 4.08 (2H, q, J = 7.0 Hz), 2.90 (2H, t, J = 7.7 Hz), 2.66 (2H, t, J = 7.7 Hz), 2.48 (1H, t, J = 2.4 Hz), 1.44 (3H, t, J = 7.0 Hz)

### Reference Example 10

6.7 g of 3-{3-ethoxy-4-(2-propynyloxy)phenyl}propionic acid, 3.2 ml of thionyl chloride, 100 ml of toluene and about 0.03 g of N,N-dimethylformamide were mixed, and stirred at 80°C for 30 minutes and then at 100°C for 30 minutes. The reaction mixture was allowed to cool to around room temperature and then concentrated under reduced pressure to obtain 17.5 g of 3-{3-ethoxy-4-(2-propynxyloxy)phenyl}propionyl chloride. 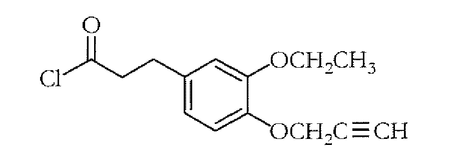 ¹H-NMR (CDCl₃,TMS) δ (ppm): 6.98 (1H, d, J = 8.3 Hz), 6.71-6.73 (2H, m), 4.74 (2H, d, J = 2.4 Hz), 4.08 (2H, q, J = 7.1 Hz), 3.18 (2H, t, J = 7.6 Hz), 2.95 (2H, t, J = 7.6 Hz), 2.49 (1H, t, J = 2.4 Hz), 1.45 (3H, t, J = 7.1 Hz)

### Reference Example 11

30 g of 4-acetyl-2-methoxyphenol, 32 g of benzyl bromide, 28 g of potassium carbonate and 300 ml of acetonitrile were mixed and stirred under reflux for 4 hours. After the reaction mixture was allowed to cool to around room temperature, ethyl acetate was added and the mixture was filtered. The resulting filtrate was concentrated under reduced pressure. The residue was washed with hexane to obtain 46 g of 4-benzyloxy-3-methoxyacetophenone. 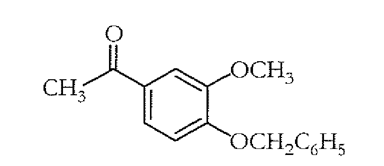 ¹H-NMR (CDCl₃, TMS) δ (ppm): 7.28-7.57 (7H, m), 6.89 (1H, d, J = 8.3 Hz), 5.23 (2H, s), 3.94 (3H, s), 2.54 (3H, s)

### Reference Example 12

18 g of ethyl diethylphosphonoacetate and 400 ml of tetrahydrofuran were mixed, and thereto 3.2 g of sodium hydride (content 60%) was added under ice-cooling. The mixture was stirred for 10 minutes. Then, a mixture of 20 g of 4-benzyloxy-3-methoxyacetophenone and 50 ml of tetrahydrofuran was added gradually under ice-cooling, and the mixture was stirred at room temperature for 2 hours. Water was added to the resulting mixture. The mixture was concentrated under reduced pressure and then extracted with ethyl acetate. The resulting organic layer was washed with water and then an aqueous saturated sodium chloride solution, dried over magnesium sulfate, and then concentrated under reduced pressure. The resulting residue was purified by a silica gel column to obtain 17 g of ethyl 3-(3-methoxy-4-benzyloxyphenyl)butenoate. 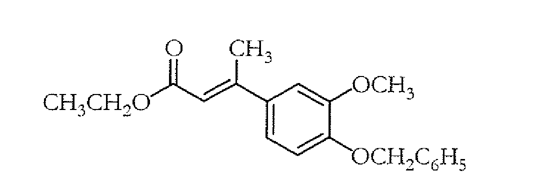 ¹H-NMR (CDCl₃, TMS) δ (ppm): 7.22-7.44 (5H, m), 7.00-7.02 (2H, m), 6.86 (1H, d, J = 8.7 Hz), 6.09 (1H, q, J = 1.2 Hz), 5.17 (2H, s), 4.21 (2H, q, J = 7.0 Hz), 3.92 (3H, s), 2.55 (3H, d, J = 1.2 Hz), 1.31 (3H, t, J = 7.0 Hz)

### Reference Example 13

10.0 g of ethyl 3-(3-methoxy-4-benzyloxyphenyl)butenoate, 1.0 g of 10% palladium carbon, 15 ml of concentrated hydrochloric acid, 0.5 g of 10% platinum carbon and 100 ml of ethanol were mixed and stirred under a hydrogen atmosphere. After absorption of a hydrogen gas was stopped, the reaction mixture was filtered, and the filtrate was concentrated under reduced pressure. The residue was washed with ethyl acetate to obtain 7.24 g of ethyl 3-(4-hydroxy-3-methoxyphenyl)butyrate. 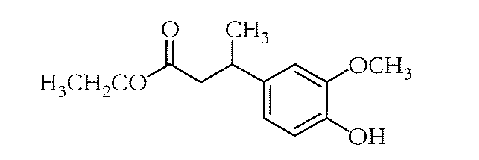 ¹H-NMR (CDCl₃, TMS) δ (ppm) : 6.83 (1H, d, J = 8.7 Hz), 6.70-6.75 (2H, m), 5.50 (1H, br.s), 4.07 (2H, q, J = 7.0 Hz), 3.87 (3H, s), 3.15-3.26 (1H, m), 2.46-2.63 (2H, m), 1.27 (3H, d, J = 6.7 Hz), 1.19 (3H, t, J = 7.0 Hz)

### Reference Example 14

7.14 g of ethyl 3-(4-hydroxy-3-methoxyphenyl)butyrate, 3.93 g of benzyl bromide, 4.98 g of potassium carbonate and 50 ml of acetonitrile were mixed and stirred at 80°C for 4 hours. After the reaction mixture was allowed to cool to around room temperature, water was added and the mixture was extracted with ethyl acetate. The resulting organic layer was washed successively with water and an aqueous saturated sodium chloride solution, dried over magnesium sulfate, and then concentrated under reduced pressure to obtain 8.3 g of ethyl 3-{3-methoxy-4-(2-propynyloxy)phenyl}butyrate. 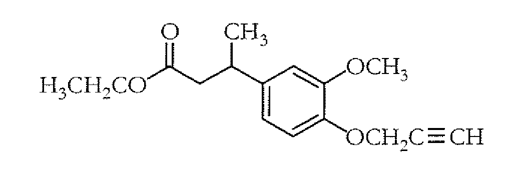 ¹H-NMR (CDCl₃, TMS) δ (ppm): 6.96 (1H, d, J = 8.9 Hz), 6.73-6.78 (2H, m), 4.72 (2H, d, J = 2.4 Hz), 4.08 (2H, q, J = 7.0 Hz), 3.86 (3H, s), 3.18-3.29 (1H, m), 2.47-2.63 (3H, m), 1.28 (3H, d, J = 7.0 Hz), 1.19 (3H, t, J = 7.1 Hz)

### Reference Example 15

8.0 g of ethyl 3-{3-methoxy-4-(2-propynyloxy)phenyl}butyrate, 1.04 g of lithium hydroxide, 40 ml of tetrahydrofuran and 15 ml of water were mixed and stirred at 65°C for 4 hours. After the reaction mixture was allowed to cool to around room temperature, water was added and the mixture was concentrated under reduced pressure. To the residue was added 5% hydrochloric acid. The mixture was extracted with chloroform thrice. The organic layer was successively washed with water and an aqueous saturated sodium chloride solution, dried over magnesium sulfate and then concentrated under reduced pressure. The residue was washed with hexane to obtained 7.0 g of 3-{3-methoxy-4-(2-propynyloxy)phenyl}butyric acid. 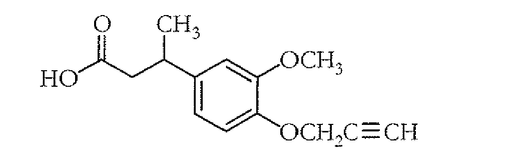 ¹H-NMR (CDCl₃ , TMS) δ (ppm): 6.97 (1H, d, J = 8.8 Hz), 6.74-6.78 (2H, m), 4.73 (2H, d, J = 2.4 Hz), 3.86 (3H, s), 3.17-3.30 (1H, m), 2.52-2.70 (2H, m), 2.49 (1H, t, J = 2.4 Hz), 1.31 (3H, d, J = 7.1 Hz)

### Reference Example 16

7.0 g of 3-{3-methoxy-4-(2-propynyloxy)phenyl}butyric acid, 5.0 g of thionyl chloride and 100 ml of toluene were mixed, and stirred at 50°C for 30 minutes and then at 80°C for 3 hours. The reaction mixture was allowed to cool to room temperature and then concentrated under reduced pressure. The residue was washed with hexane to obtain 6.3 g of 3-{3-methoxy-4-(2-propynyloxy)phenyl}butyryl chloride. 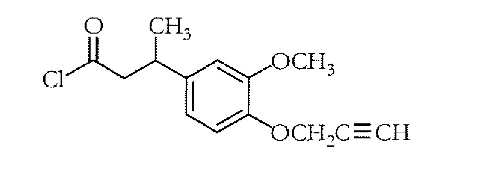 ¹H-NMR (CDCl₃, TMS) δ (ppm): 6.97 (1H, d, J = 7.7 Hz), 6.72-6.78 (2H, m), 4.73 (2H, d, J = 2.4 Hz), 3.87 (3H, s), 3.25-3.35 (1H, m), 3.04-3.21 (2H, m), 2.49 (1H, t, J = 2.4 Hz), 1.34 (3H, d, J = 7.0 Hz)

### Reference Example 17

30 g of 4-benzyloxy-3-methoxybenzaidehyde, 12 g of sodium propionate and 24 g of propionic anhydride were mixed and stirred at 150°C for 6 hours. After the reaction mixture was allowed to cool to room temperature, water was added, and the mixture was extracted with ethyl acetate. The resulting organic layer was washed with 5% hydrochloric acid and then with an aqueous saturated sodium chloride solution twice, dried over magnesium sulfate, and then concentrated under reduced pressure. The resulting residue was washed with toluene and hexane to obtain 28 g of 3-(3-methoxy-4-benzyloxyphenyl)-2-methylacrylic acid. 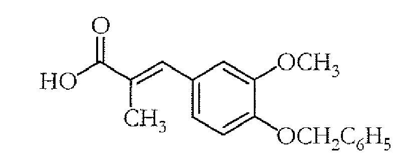 ¹H-NMR (CDCl₃, TMS) δ (ppm): 7.74 (1H, br.s), 7. 29-7. 45 (5H, m), 6.99-7.04 (2H, m), 6.91 (1H, d, J = 8.9 Hz), 5.20 (2H, s), 3.91 (3H, s), 2.16 (3H, d, J = 1.3 Hz)

### Reference Example 18

75 ml of ethanol and 9 ml of acetyl chloride were mixed and stirred at room temperature for 10 minutes. To the mixture was added 8.3 g of 3-(3-methoxy-4-benzyloxyphenyl)-2-methylacrylic acid, and the mixture was stirred under reflux for 2 hours. The reaction mixture was allowed to cool to around room temperature and then filtered. The resulting filtrate was concentrated under reduced pressure. To the resulting residue, 1.0 g of 10% palladium carbon, 0.5 g of 10% platinum carbon, 15 ml of 36% hydrochloric acid and 80 ml of ethanol were added, and stirred under a hydrogen atmosphere. After absorption of a hydrogen gas was stopped, the reaction mixture was filtered, and the resulting filtrate was concentrated under reduced pressure. The residue was washed with hexane to obtain 5.4 g of ethyl 3-(4-hydroxy-3-methoxyphenyl)-2-methylpropionate. 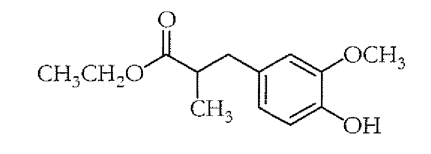 ¹H-NMR (CDCl₃, TMS) δ (ppm): 6.81 (1H, d, J = 8.5 Hz), 6.64-6.66 (2H, m), 5.46 (1H, br.s), 4.09 (3H, q, J = 7.1 Hz), 3.86 (3H, s), 2.94 (1H, dd, J = 13.3 Hz, 6.8 Hz), 2.56-2.72 (2H, m), 1.20 (3H, t, J = 7.1 Hz), 1.14 (3H, d, J = 7.0 Hz)

### Reference Example 19

In the same way as Reference Example 14, 6.8 g of ethyl 3-{3-methoxy-4-(2-propynyloxy)phenyl}-2-methylpropionate was obtained from 5.4 g of ethyl 3-(3-hydroxy-4-methoxyphenyl)-2-methylpropionate. 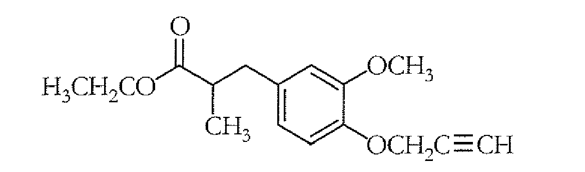 ¹H-NMR (CDCl₃, TMS) δ (ppm): 6.94 (1H, d, J = 8.8 Hz), 6.68-6.74 (2H, m), 4.73 (2H, d, J = 2.4 Hz), 4.09 (2H, q, J = 7.0 Hz), 3.85 (3H, s), 2.92-3.01 (1H, m), 2.58-2.74 (2H, m), 2.48 (1H, t, J = 2.4 Hz), 1.19 (3H, t, J = 7.2 Hz), 1.15 (3H, d, J = 6.8 Hz)

### Reference Example 20

In the same way as Reference Example 15, 5.33 g of 3-{3-methoxy-4-(2-propynyloxy)phenyl}-2-methylpropionic acid was obtained from 6.50 g of ethyl 3-{3-methoxy-4-(2-propynyloxy)phenyl}-2-methylpropionate. 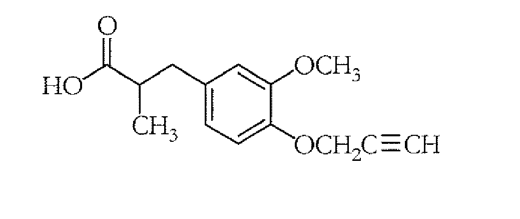 ¹H-NMR (CDCl₃ , TMS) δ (ppm): 6.95 (1H, d, J = 8.7 Hz), 6.70-6.75 (2H, m), 4.73 (2H, d, J = 2.2 Hz), 3.84 (3H, s), 2.97-3.06 (1H, m), 2.58-2.80 (2H, m), 2.49 (1H, t, J = 2.4 Hz), 1.19 (3H, d, J = 6.7 Hz)

### Reference Example 21

In the same way as Reference Example 16, 5.09 g of 3-{3-methoxy-4-(2-propynyloxy)phenyl}-2-methylpropionyl chloride was obtained from 5.03 g of 3-{3-methoxy-4-(2-propynyloxy)phenyl}-2-methylpropionic acid. 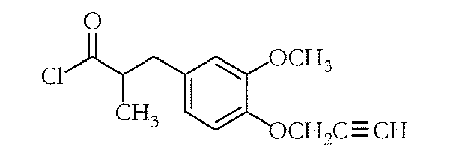 ¹H-NMR (CDCl₃, TMS) δ (ppm): 6.96 (1H, d, J = 8.2 Hz), 6.72-6.75 (2H, m), 4.73 (2H, d, J = 2.4 Hz), 3.85 (3H, s), 3.07-3.18 (2H, m), 2.67-2.77 (1H, m), 2.49 (1H, t, J = 2.4 Hz), 1.28 (3H, d, J = 6.8 Hz)

### Reference Example 22

20 g of 4-hydroxy-3-methoxybenzaldehyde, 21 g of sodium acetate and 40 g of acetic anhydride were mixed and stirred at 150°C (the temperature of a bath) for 6 hours. After the reaction mixture was allowed to cool to room temperature, water was added and the mixture was extracted with ethyl acetate. The resulting organic layer was washed with water, dried over magnesium sulfate, and then concentrated under reduced pressure. Toluene was added to the resulting residue and the mixture was filtered. The resulting solid was washed with toluene and hexane, and then dried to obtain 14 g of 3-(3-methoxy-4-acetoxyphenyl)acrylic acid. 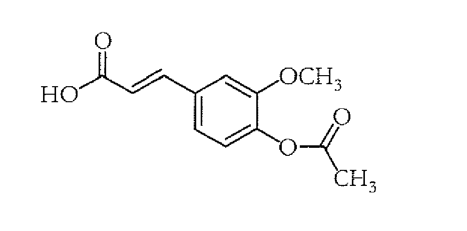 ¹H-NMR (CD₃SOCD₃, TMS) δ (ppm): 12.4 (1H, br.s), 7.60 (1H, d, J = 15 Hz), 7.50 (1H, s), 7.25 (1H, d, J = 8.0 Hz), 7.11 (1H, d, J = 8.0 Hz), 6.58 (1H, d, J = 15 Hz), 3.82 (3H, s), 2.26 (3H, s)

### Reference Example 23

In the same way as Reference Example 18, 9.7 g of 3-(3-methoxy-4-acetoxyphenyl)propionic acid was obtained from 10 g of 3-(3-methoxy-4-acetoxyphenyl)acrylic acid. 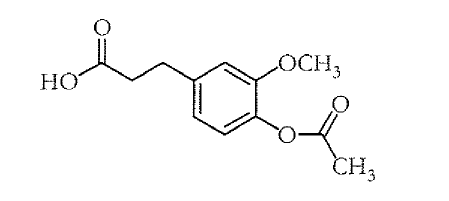 ¹H-NMR (CDCl₃, TMS) δ (ppm) : 6.94 (1H, d, J = 8.0 Hz), 6.76-6.81 (2H, m), 3.81 (3H, s), 2.95 (2H, t, J = 7.5 Hz), 2.69 (2H, t, J = 7.5 Hz), 2.30 (3H, s)

### Reference Example 24

100 g of 4-benzyloxy-3-methoxybenzaldehyde, 120 g of ethyl diethylphosphonoacetate, 570 g of potassium carbonate and 570 ml of water were mixed and stirred under reflux for 20 hours. After the reaction mixture was allowed to cool to room temperature, water was added and the mixture was extracted with ethyl acetate twice. The resulting organic layer was washed with water and then an aqueous saturated sodium chloride solution, dried over magnesium sulfate, and then concentrated under reduced pressure. The resulting residue was recrystallized from ethanol to obtain 58.6 g of ethyl 3-{3-methoxy-4-(benzyloxy)phenyl}acrylate. 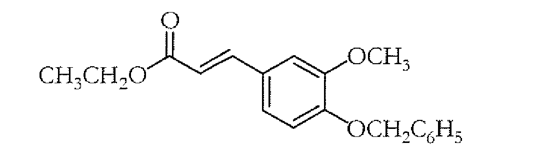 ¹H-NMR (CDCl₃, TMS) δ (ppm): 7.60 (1H, d, J = 15 Hz), 7.29-7.44 (5H, m), 7.06 (1H, d, J = 1.9 Hz), 7.02 (1H, dd, J = 8.2 Hz, 1.9 Hz), 6.86 (1H, d, J = 8.2 Hz), 6.29 (1H, d, J = 15 Hz), 5.18 (2H, s), 4.25 (2H, q, J = 7.3 Hz), 3.91 (3H, s), 1.33 (3H, t, J = 7.3 Hz)

### Reference Example 25

33 g of ethyl 3-{3-methoxy-4-(benzyloxy)phenyl}acrylate, 0.3 g of 5% palladium carbon, about 0.05 g of 36% hydrochloric acid and 200 ml of ethanol were mixed and stirred under a hydrogen atmosphere. After absorption of a hydrogen gas was stopped, the reaction mixture as filtered and the filtrate was concentrated under reduced pressure. To the residue, ethyl acetate and water were added and layers were separated. After the resulting organic layer was dried over magnesium sulfate, about 5 g of activated carbon and about 5 g of caustic terra alba were added. The mixture was filtered, and the filtrate was concentrated under reduced pressure. The residue was washed with hexane to obtain 23 g of ethyl 3-(4-hydroxy-3-methoxyphenyl)propionate. 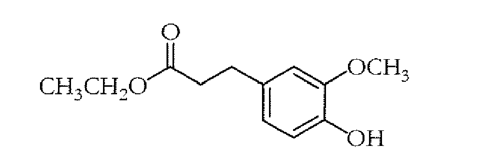 ¹H-NMR (CnCl₃ , TMS) δ (ppm): 6.82 (1H, d, J = 7.7 Hz), 6.67-6.70 (2H, m), 5.47 (1H, s), 4.19 (2H, q, J = 7.2 Hz), 3.87 (3H, s), 2.88 (2H, t, J = 7.5 Hz), 2.58 (2H, t, J = 7.5 Hz), 1.24 (3H, t, J = 7.2 Hz)

### Reference Example 26

15.2 g of 3-hydroxy-4-methoxybenzaldehyde, 18.0 g of benzyl bromide, 15.2 g of potassium carbonate and 200 ml of acetonitrile were mixed and stirred at 80°C for 3 hours. After the reaction mixture was allowed to cool to around room temperature, ethyl acetate was added and the mixture was filtered. The filtrate was concentrated under reduced pressure, and the residue was purified by a silica gel column to obtain 23.3 g of (3-benzyloxy-4-methoxy)benzaldehyde. 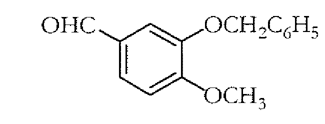 ¹H-NMR (CDCl₃, TMS) δ (ppm): 9.80 (1H, s), _{*r*} 7.30-7.48 (7H, m), 6. 98 (1H, d, J = 8.7 Hz), 5.118 (2H, s), 3.95 (3H, s)

### Reference Example 27

2.1 g of sodium hydride and 150 ml of tetrahydrofuran were mixed at 0 to 5°C, and thereto 11.2 g of ethyl diethylphosphonoacetate was added dropwise. After addition, the mixture was stirred at room temperature for 30 minutes. Then, a mixed solution of 12.1 g of (3-benzyloxy-4-methoxy)benzaldehyde in tetrahydrofuran was added dropwise, and stirred at 0 to 5°C for 15 minutes and then at room temperature for 30 minutes. After water was added thereto, layers were separated with ethyl acetate. The organic layer was dried over magnesium sulfate and then concentrated under reduced pressure. The residue was washed with hexane to obtain 14.1 g of ethyl 3-(3-benzyl-oxy-4-methoxyphenyl)acrylate. 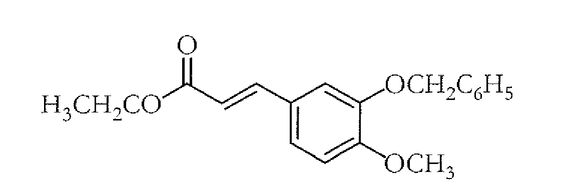 ¹H-NMR (CDCl₃, TMS) δ (ppm): 7.56 (1H, d, J = 15.7 Hz) 7.42-7.47 (2H, m), 7.34-7.41 (2H, m), 7.27-7.34 (1H, m), 7.11 (1H, dd, J = 8.2 Hz, 1.9 Hz), 7.07 (1H, d, J = 1.9 Hz), 6.87 1H, d, J = 8.2 Hz), 6.22 (1H, d, J = 15.6 Hz), 5.15 (2H, s), 4.24 (2H, q, J = 7.0 Hz), 3.91 (3H, s), 1.32 (3H, t, J = 7.0 Hz)

### Reference Example 28

8.0 g of ethyl 3-(3-benzyloxy-4-methoxyphenyl)acrylate, 0.8 g of 10% palladium carbon and 80 ml of ethanol were mixed and stirred under a hydrogen atmosphere. After absorption of a hydrogen gas was stopped, the reaction mixture was filtered, and the filtrate was concentrated under reduced pressure. The residue was washed with hexane to obtain 5.53 g of ethyl 3-(3-hydroxy-4-methoxyphenyl)propionate. 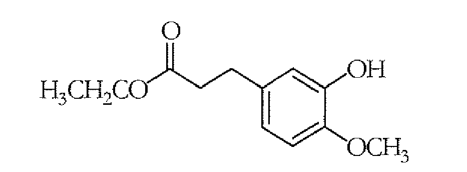 ¹H-NMR (CDCl₃, TMS) δ (ppm): 6.74-6.78 (2H, m), 6.67 (1H, dd, J = 8.1 Hz, 2.0 Hz), 5.55 (1H, br.s), 4.12 (2H, q, J = 7.0 Hz), 3.86 (3H, s), 2.85 (2H, t, J = 7.9 Hz), 2.57 (2H, t, J = 7.9 Hz), 1.23 (3H, t, J = 7.1 Hz)

### Reference Example 29

In the same way as Reference Example 14, 2.53 g of ethyl 3-{4-methoxy-3-(2-propynyloxy)phenyl}propionate was obtained from 2.24 g of ethyl 3-(3-hydroxy-4-methoxyphenyl)propionate. 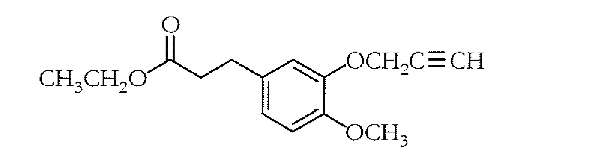 ¹H-NMR (CDCl₃, TMS) δ (ppm): 6.79-6.89 (3H, m), 4.74 (2H, d, J = 2.4 Hz), 4.12 (2H, q, J = 7.1 Hz), 3.84 (3H, s), 2.89 (2H, t, J = 7.6 Hz), 2.59 (2H, t, J = 7.6 Hz), 2.49 (1H, t, J = 2.4 Hz), 1.24 (3H, t, J = 7.1 Hz)

### Reference Example 30

In the same way as Reference Example 15, 1.93 g of 3-{4-methoxy-3-(2-propynyloxy)phenyl}propionic acid was obtained from 2.53 g of ethyl 3-{4-methoxy-3-(2-propynyloxy)phenyl}propionate. 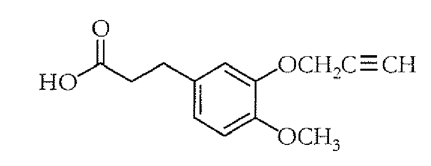 ¹H-NMR (CDCl₃, TMS) δ (ppm): 6.80-6.90 (3H, m), 4.75 (2H, d, J = 2.4 Hz), 3.84 (3H, s), 2.91 (2H, t, J = 7.6 Hz), 2.66 (2H, t, J = 7.6 Hz), 2.49 (1H, t, J = 2.4 Hz)

### Reference Example 31

10.36 g of 3,4-dihydroxybenzaldehyde, 25.65 g of benzyl bromide, 22.8 g of potassium carbonate and 200 ml of N,N-dimethylformaldehyde were mixed and stirred at room temperature for 3 hours. Water was then added to the reaction mixture, and the mixture was extracted with ethyl acetate. The organic layer was washed successively with 5% hydrochloric acid, water and an aqueous saturated sodium chloride solution, dried over magnesium sulfate, and then concentrated under reduced pressure. The residue was washed with hexane to obtain 23.27 g of 3,4-bis(benzyloxy)benzaldehyde. 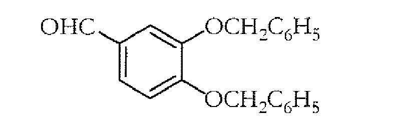 ¹H-NMR (CDCl₃, TMS) δ (ppm): 9.80 (1H, s), 7.27-7.49 (12H, m), 7.01 (1H, d, J = 8.1 Hz), 5.25 (2H, s), 5.21 (2H, s)

### Reference Example 32

1.88 g of sodium hydride and 150 ml of tetrahydrofuran were mixed at 0 to 5°C. Thereto 10.0 g of ethyl diethylphosphonoacetate was added dropwise. After addition, the mixture was stirred at room temperature for 30 minutes. Then, a mixed solution of 114.3 g of 3,4-bis(benzyloxy)benzaldehyde in tetrahydrofuran was added dropwise. The mixture was stirred at 0 to 5°C for 15 minutes and then at room temperature for 30 minutes. After water was added to the mixture, layers were separated with ethyl acetate. The organic layer was washed successively with 5% hydrochloric acid, water and an aqueous saturated sodium chloride solution, dried over magnesium sulfate, and then concentrated under reduced pressure. The residue was washed with hexane to obtain 16.54 g of ethyl 3-{3,4-bis(benzyloxy)phenyl}acrylate. 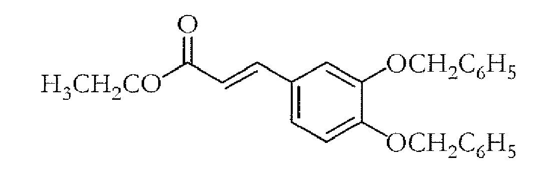 ¹H-NMR (CDCl₃, TMS) δ (ppm): 7.55 (1H, d, J = 15.6 Hz) 7.26-7.47 (10H, m), 7.11 (1H, d, J = 1.9 Hz), 7.05 (1H, dd, J = 8.3 Hz, 2.0 Hz), 6.90 (1H, d, J = 8.4 Hz), 6.23 (1H, d, J=15.9Hz), 5.18 (2H, s), 5.16 (2H, s), 4.23 (2H, q, J = 7.0 Hz), 1.33 (3H, t, J = 7.1 Hz)

### Reference Example 33

10.0 g of ethyl 3-{3,4-bis(benzyloxy)phenyl}acrylate, 0.5 g of 10% palladium carbon and 150 ml of ethanol were mixed and stirred under a hydrogen atmosphere. After absorption of a hydrogen gas was stopped, the reaction mixture was filtered and the filtrate was concentrated under reduced pressure. The residue was washed with hexane to obtain 5.17 g of ethyl 3-(3,4-dihydroxyphenyl)propionate. 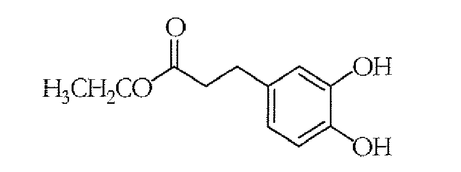 ¹H-NMR (CDCl₃, TMS) δ (ppm): 6.76 (1H, d, J = 8.0 Hz), 6.70 (1H, d, J = 1.9 Hz), 6.61 (1H, dd, J = 8.0 Hz, 1.7 Hz), 5.31 (2H, br.s), 4.13 (2H, q, J = 7.0 Hz), 2.83 (2H, t, J = 7.7 Hz), 2.58 (2H, t, J = 7.7 Hz), 1.23 (3H, t, J = 7.0 Hz)

### Reference Example 34

5.17 g of ethyl 3-(3,4-dihydroxyphenyl)propionate, 5.85 g of propargyl bromide, 7.48 g of potassium carbonate and 60 ml of acetonitrile were mixed and stirred at 80°C for 3 hours. After the reaction mixture was allowed to cool to around room temperature, water was added and the mixture was extracted with ethyl acetate. The organic layer was washed successively with 5% hydrochloric acid, water and an aqueous saturated sodium chloride solution, dried over magnesium sulfate, and then concentrated under reduced pressure to obtain 6.49 g of ethyl 3-{3,4-bis(2-propynyloxy)phenyl}propionate. 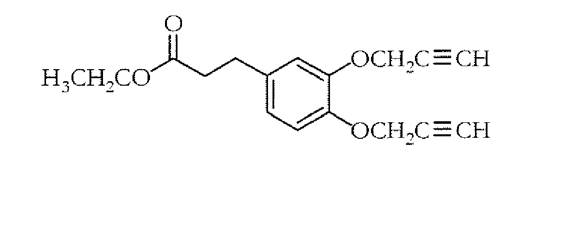 ¹H-NMR (CDCl₃, TMS) δ (ppm): 6.97 (1H, d, J = 8.2 Hz), 6.90 (1H, d, J = 2.1 Hz), 6.80 (1H, dd, J = 8.0 Hz, 1.9 Hz), 4.73 (2H, d, J = 2.4 Hz), 4.72 (2H, d, J = 2.4 Hz), 4.12 (2H, q, J = 7.0 Hz), 2.90 (2H, t, J = 7.8 Hz), 2.59 (2H, t, J = 7.8 Hz), 2.50 (1H, t, J = 2.4 Hz), 2.49 (1H, t, J = 2.4 Hz), 1.23 (3H, t, J = 7.1 Hz)

### Reference Example 35

6.17 g of ethyl 3-{3,4-bis(2-propynyloxy)phenyl}propionate, 0.77 g of lithium hydroxide, 30 ml of tetrahydrofuran and 15 ml of water were mixed and stirred at 65°C for 3 hours. The reaction mixture was allowed to cool to around room temperature and then concentrated under reduced pressure. To the residue was added 5% hydrochloric acid, and the mixture was extracted with chloroform thrice. The organic layer was dried over magnesium sulfate and then concentrated under reduced pressure. The residue was washed with hexane to obtain 4.59 g of 3-{3,4-bis(2-propynyloxy)phenyl}propionic acid. 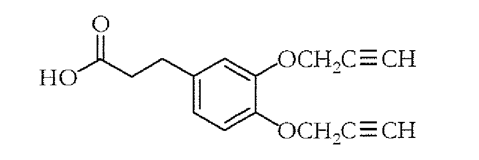 ¹H-NMR (CDCl₃, TMS) δ (ppm): 6.98 (1H, d, J = 8.2 Hz), 6.92 (1H, d, J = 1.9 Hz), 6.81 (1H, dd, J = 8.2 Hz, 1.9 Hz), 4.74 (2H, d, J = 2.4 Hz), 4.73 (2H, d, J = 2.4 Hz), 2.92 (2H, t, J = 7. 6 Hz), 2.67 (2H, t, J = 7.6 Hz), 2.50 (1H, t, J = 2.4 Hz), 2.49 (1H, t, J = 2.4 Hz)

### Reference Example 36

2.24 g of ethyl 3-(4-hydroxy-3-methoxyphenyl)propionate, 1.72 g of ethyl iodide, 1.66 g of potassium carbonate and 50 ml of N,N-dimethylformaldehyde were mixed and stirred at room temperature for 4 hours. Water was then added to the reaction mixture, and the mixture was extracted with tert-butyl methyl ether. The organic layer was washed successively with 5% hydrochloric acid, water and an aqueous saturated sodium chloride solution, dried over magnesium sulfate, and then concentrated under reduced pressure to obtain 2.52 g of ethyl 3-{4-ethoxy-3-methoxyphenyl}propionate. 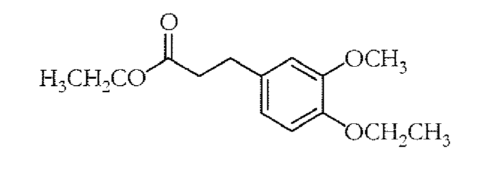 ¹H-NMR (CDCl₃, TMS) δ (ppm): 6.79 (1H, d, J = 7.7 Hz), 6.69-6.74 (2H, m), 4.12 (2H, q, J = 7.0 Hz), 4.07 (2H, q, J = 7.0 Hz), 3.85 (3H, s), 2.89 (2H, t, J = 7.5 Hz), 2.59 (2H, t, J = 7.5 Hz), 1.44 (3H, t, J = 7.0 Hz), 1.23 (3H, t, J = 7.0 Hz)

### Reference Example 37

2.52 g of ethyl 3-{4-ethoxy-3-methoxyphenyl}propionate and 3.2 ml of a 25% aqueous sodium hydroxide solution were mixed. Then, ethanol was added to the mixture until a homogeneous solution was obtained, and the mixture was stirred at 78°C for 2 hours. The reaction mixture was then concentrated under reduced pressure. To the residue was added 5% hydrochloric acid, and the mixture was extracted with chloroform thrice. The organic layer was dried over magnesium sulfate and then concentrated under reduced pressure. The residue was washed with hexane to obtain 1.80 g of 3-{4-ethoxy-3-methoxyphenyl}propionic acid. 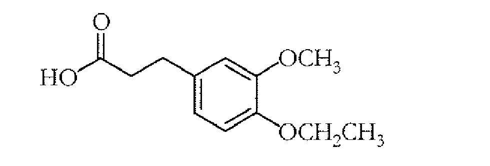 ¹H-NMR (CDCl₃, TMS) δ (ppm): 6.79 (1H, d, J = 8.0 Hz), 6.70-6.75 (2H, m), 4.07 (2H, q, J = 7.0 Hz), 3.85 (3H, s), 2.90 (2H, t, J = 7.8 Hz), 2.66 (2H, t, J = 7.7 Hz), 1.44 (3H, t, J = 7.0 Hz)

### Reference Example 38

2.24 g of ethyl 3-(4-hydroxy-3-methoxyphenyl)propionate, 1.87 g of propyl iodide, 1.66 g of potassium carbonate and 50 ml of N,N-dimethylformaldehyde were mixed, and stirred at room temperature for 2 hours and then at 80°C for 1 hour. Water was then added to the reaction mixture, and the mixture was extracted with ethyl acetate. The organic layer was washed successively with 5% hydrochloric acid, water and an aqueous saturated sodium chloride solution, dried over magnesium sulfate, and then concentrated under reduced pressure to obtain 2.33 g of ethyl 3-(3-methoxy-4-propoxyphenyl)propionate. 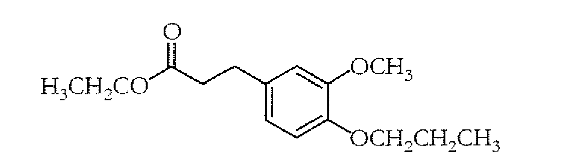 ¹H-NMR (CDCl₃, TMS) δ (ppm): 6.79 (1H, d, J = 8.0 Hz), 6.69-6.73 (2H, m), 4.12 (2H, q, J = 7.1 Hz), 3.94 (2H, t, J = 6.8 Hz), 3.84 (3H, s), 2.85-2.92 (2H, m), 2.59 (2H, t, J = 7.8 Hz), 1.78-1.90 (2H, m), 1.23 (3H, t, J = 7.1 Hz), 1.02 (3H, t, J = 7.3 Hz)

### Reference Example 39

2.33 g of ethyl 3-(3-methoxy-4-propoxyphenyl)propionate and 2.8 ml of a 25% aqueous sodium hydroxide solution were mixed. Then, ethanol was added to the mixture until an almost homogeneous solution was obtained, and the mixture was stirred at 78°C for 2 hours. The reaction mixture was allowed to cool to around room temperature and then concentrated under reduced pressure. To the residue was added 5% hydrochloric acid, and the mixture was extracted with chloroform thrice. The organic layer was dried over magnesium sulfate and then concentrated under reduced pressure. The residue was washed with hexane to obtain 1.72 g of 3-(3-methoxy-4-propoxyphenyl)propionic acid. 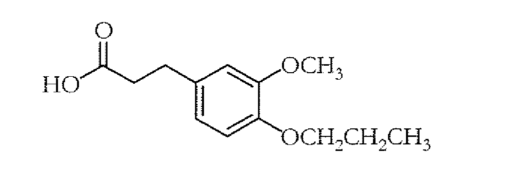 ¹H-NMR (CDCl₃, TMS) δ (ppm): 6.80 (1H, d, J = 8.0 Hz), 6.70-6.74 (2H, m), 3.95 (2H, t, J = 6.7 Hz), 3.83 (3H, s), 2.90 (2H, t, J = 7.8 Hz), 2.66 (2H, t, J = 7.7 Hz), 1.79-1.91 (2H, m), 1.02 (3H, t, J = 7. 3 Hz)

### Reference Example 40

2.24 g of ethyl 3-(4-hydroxy-3-methoxyphenyl)propionate, 1.33 g of allyl bromide, 1.66 g of potassium carbonate and 50 ml of N,N-dimethylformaldehyde were mixed, and stirred at room temperature for 2 hours and then at 80°C for 4 hours. After the reaction mixture was allowed to cool to around room temperature, water was added thereto, followed by extraction with ethyl acetate. The organic layer was washed successively with 5% hydrochloric acid, water and an aqueous saturated sodium chloride solution, dried over magnesium sulfate, 1 and then concentrated under reduced pressure to obtain 2.40 g of ethyl 3-{3-methoxy-4-(2-propenyloxy)phenyl}propionate. 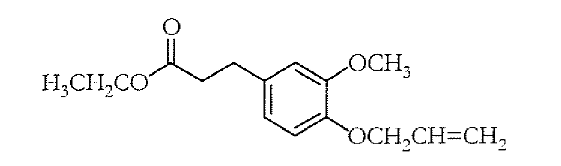 ¹H-NMR (CDCl₃, TMS) δ (ppm): 6.77-6.85 (1H, m), 6.67-6.75 (2H, m), 6.01-6.15 (1H, m), 5.33-5.43 (1H, m), 5.23-5.29 (1H, m), 4.55-4.59 (2H, m), 4.08-4.17 (2H, m), 3.85 (3H, s), 2.84-2.92 (2H, m), 2.59 (2H, t, J = 7.7 Hz), 1.23 (3H, t, J = 7.3 Hz)

### Reference Example 41

2.40 g of ethyl 3-{3-methoxy-4-(2-propenyloxy)phenyl}propionate and 2.9 ml of a 25% aqueous sodium hydroxide were mixed. Then, ethanol was added to the mixture until an almost homogeneous solution was obtained, and the mixture was stirred under reflux for 2 hours. The reaction mixture was allowed to cool to around room temperature and then concentrated under reduced pressure. To the residue was added 5% hydrochloric acid, and the mixture was extracted with chloroform thrice. The organic layer was dried over magnesium sulfate and then concentrated under reduced pressure. The residue was washed with hexane to obtain 1.60 g of 3-{3-methoxy-4-(2-propenyloxy)phenyl}propionic acid. 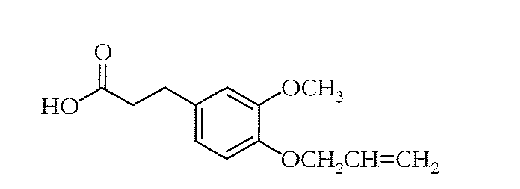 ¹H-NMR (CDCl₃, TMS) δ (ppm): 6.80 (1H, d, J = 8.0 Hz), 6.70-6.75 (2H, m), 6.01-6.13 (1H, m), 5.25-5.42 (2H, m), 4.57-4.60 (2H, m), 3.85 (3H, s), 2.90 (2H, t, J = 7.6 Hz), 2.66 (2H, t, J = 7.6 Hz)

### Reference Example 42

2.24 g of ethyl 3-(4-hydroxy-3-methoxyphenyl) propionate, 1.35 g of isopropyl bromide, 1.66 g of potassium carbonate and 50 ml of N,N-dimethylformaldehyde were mixed, and stirred at room temperature for 2 hours and then at 80°C for 4 hours. After the reaction mixture was allowed to cool to around room temperature, water was added and the mixture was extracted with ethyl acetate. The organic layer was washed successively with 5% hydrochloric acid, water and an aqueous saturated sodium chloride solution, dried over magnesium sulfate, and then concentrated under reduced pressure to obtain 1.19 g of ethyl 3-(4-isopropoxy-3-methoxyphenyl)propionate. 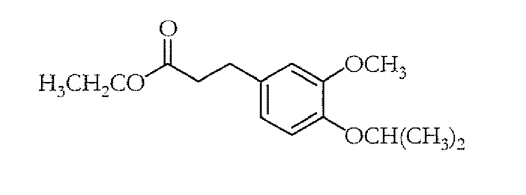 ¹H-NMR (CDCl₃, TMS) δ (ppm): 6.81 (1H, d, J = 8.0 Hz), 6. 73 (1H, d, J = 1.9 Hz), 6.70 (1H, dd, J = 8.0 Hz, 1.9 Hz), 4.41-4.52 (1H, m)4.12 (2H, q, J = 7.0 Hz), 3.83 (3H, s)2.88 (2H, t, J = 7.8 Hz), 2.59 (2H, t, J = 7.8 Hz), 1.34 (6H, d, J = 6.1 Hz), 1.23 (3H, t, J = 7.2 Hz)

### Reference Example 43

1.19 g of ethyl 3-(4-isopropoxy-3-methoxyphenyl)propionate and 1.1 ml of a 25% aqueous sodium hydroxide solution were mixed. Then, ethanol was added to the mixture until an almost homogeneous solution was obtained, and the mixture was stirred at 78°C for 2 hours. The reaction mixture was then concentrated under reduced pressure. To the residue was added 5% hydrochloric acid, and the mixture was extracted with chloroform thrice. The organic layer was dried over magnesium sulfate and then concentrated under reduced pressure. The residue was washed with hexane to obtain 0.98 g of 3-(4-isopropoxy-3-methoxyphenyl)-propionic acid. 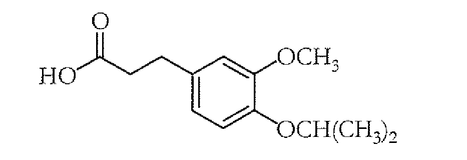 ¹H-NMR (CDCl₃, TMS) δ (ppm): 6.82 (1H, d, J = 8.0 Hz), 6.73 (1H, d, J = 1.7 Hz), 6.71 (1H, dd, J = 8.0 Hz, 1.9 Hz), 4.43-4.51 (1H, m), 3.83 (3H, s), 2.90 (2H, t, J = 7.8 Hz), 2.66 (2H, t, J = 7.8 Hz), 1.35 (6H, d, J = 6.0 Hz)

### Reference Example 44

3.2 g of 2-fluoroethanol, 6.56 g of triethylamine and 50 ml of tetrahydrofuran were mixed, and thereto 6.3 g of methanesulfonyl chloride was added dropwise at 0°C. The mixture was stirred at 0°C for 30 minutes and then at room temperature for 2 hours. The reaction mixture was then concentrated under reduced pressure. Water was added to the residue, followed by extraction with ethyl acetate. The organic layer was washed successively with 5% hydrochloric acid, water and an aqueous saturated sodium chloride solution, dried over magnesium sulfate, and then concentrated under reduced pressure to obtain 4.50 g of (2-fluoroethyl) methanesulfonate. 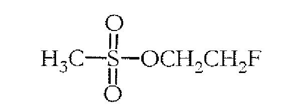 ¹H-NMR (CDCl₃ , TMS) δ (ppm): 4.67 (2H, ddd, J = 47 Hz, 4.0 Hz, 4.0 Hz), 4.47 (2H, ddd, J = 27 Hz, 4.1 Hz, 4.1 Hz), 3.19 (3H, s)

### Reference Example 45

1.12 g of ethyl 3-(4-hydroxy-3-methoxyphenyl)propionate, 0.85 g of (2-fluoroethyl) methanesulfonate, 1.03 g of potassium carbonate and 25 ml of acetonitrile were mixed and stirred at 80°C for 4 hours. After the reaction mixture was allowed to cool to around room temperature, water was added thereto, followed by extraction with ethyl acetate. The organic layer was washed successively with 5% hydrochloric acid, water and an aqueous saturated sodium chloride solution, dried over magnesium sulfate, and then concentrated under reduced pressure to obtain 1.08 g of ethyl 3-{4-(2-fluoroethoxy)-3-methoxy-phenyl}propionate. 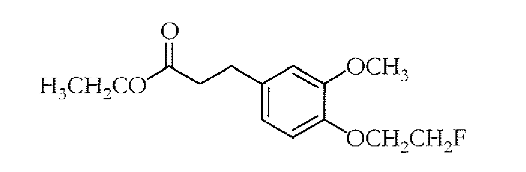 ¹H-NMR (CDCl₃, TMS) δ (ppm): 6.83 (1H, d, J = 8.0 Hz), 6 . 70- 6. 77 (2H, m), 4.75 (2H, ddd, J = 4.2 Hz, 4.2 Hz, 47 Hz), 4.24 (2H, ddd, J = 4.2 Hz, 4.2 Hz, 27 Hz), 4.12 (2H, q, J = 7.2 Hz), 3.85 (3H, s), 3.85 (2H, t, J = 7.6 Hz), 2.59 (2H, t, J = 7.6 Hz), 1.23 (3H, t, J = 7.2 Hz)

### Reference Example 46

1.00 g of ethyl 3-{4-(2-fluoroethoxy)-3-methoxhphenyl}propionate and 0.9 ml of a 25% aqueous sodium hydroxide solution were mixed. Then, ethanol was added to the mixture until an almost homogeneous solution was obtained, and the mixture was stirred at 78°C for 2 hours. Thereafter, the reaction mixture was concentrated under reduced pressure. To the residue was added 5% hydrochloric acid, and the mixture was extracted with chloroform thrice. The organic layer was dried with magnesium sulfate and then concentrated under reduced pressure. The residue was washed with hexane to obtain 0.79 g of 3-{4-(2-fluoroethoxy)-3-methoxyphenyl}propionic acid. 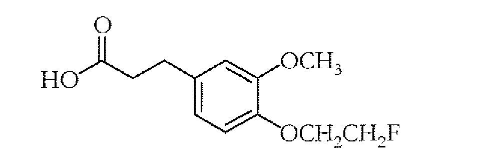 ¹H-NMR (CDCl₃, TMS) δ (ppm): 6.85 (1H, d, J = 8.3 Hz), 6.71-6.77 (2H, m), 4.87 (2H, ddd, J = 4.3 Hz, 4.32 Hz, 47 Hz), 4.24 (2H, ddd, J = 4.3 Hz, 4.3 Hz, 27 Hz), 3.85 (3H, s), 2.91 (2H, t, J = 7.7 Hz), 2.67 (2H, t, J = 7.7 Hz)

### Reference Example 47

1.12 g of ethyl 3-(4-hydroxy-3-methoxyphenyl)propionate, 0.80 g of 1-bromo-2-butyne, 1.03 g of potassium carbonate and 25 ml of acetonitrile were mixed and stirred at 80°C for 4 hours. After the reaction mixture was allowed to cool to around room temperature, water was added, followed by extraction with ethyl acetate. The organic layer was washed successively with 5% hydrochloric acid, water and an aqueous saturated sodium chloride solution, dried over magnesium sulfate, and then concentrated under reduced pressure to obtain 1.10 g of ethyl 3-{4-(2-butynyloxy)-3-methoxyphenyl}propionate. 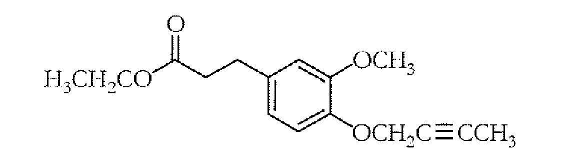 ¹H-NMR (CDCl₃, TMS) δ (ppm): 6.92 (1H, d, J = 8.8 Hz), 6.70-6.75 (2H, m), 4.68 (2H, d, J = 2.4 Hz), 4.13 (2H, q, J = 7.0 Hz), 3.85 (3H, s), 2.90 (2H, t, J = 7.8 Hz), 2.60 (2H, t, J = 7.8 Hz), 1.83 (3H, t, J = 2.4 Hz), 1.24 (3H, t, J = 7.2 Hz)

### Reference Example 48

1.10 g of ethyl 3-{4-(2-butynyloxy)-3-methoxyphenyl}propionate and 1.0 ml of a 25% aqueous sodium hydroxide solution were mixed. Then, ethanol was added to the mixture until an almost homogeneous solution was obtained, and the mixture was stirred at 78°C for 2 hours. Thereafter, the reaction mixture was concentrated under reduced pressure. To the residue was added 5% hydrochloric acid, and the mixture was extracted with chloroform thrice. The organic layer was dried over magnesium sulfate and then concentrated under reduced pressure. The residue was washed with hexane to obtain 0.84 g of 3-{4-(2-butynyloxy)-3-methoxyphenyl}propionic acid. 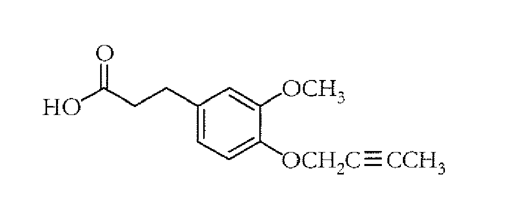 ¹H-NMR (CDCl₃, TMS) δ (ppm): 6.94 (1H, d, J = 8.7 Hz), 6.72-6.76 (2H, m), 4.66-4.70 (2H, m), 3.85 (3H, s), 2.91 (2H, t, J = 7.7 Hz), 2.67 (2H, t, J = 7.7 Hz), 1.83 (3H, t, J = 2.3 Hz)

### Reference Example 49

3.5 g of 3-butyn-2-ol, 6.56 g of triethylamine and 50 ml of tetrahydrofuran were mixed, and thereto 6.3 g of methanesulfonyl chloride was added dropwise at 0°C. The mixture was stirred at 0°C for 30 minutes and then at room temperature for 4 hours. The reaction mixture was then concentrated under reduced pressure. Water was added to the residue, followed by extraction with ethyl acetate. The organic layer was washed successively with 5% hydrochloric acid, water and an aqueous saturated sodium chloride solution, dried over magnesium sulfate, and then concentrated under reduced pressure to obtain 4.8 g of (1-methyl-propynyl) methanesulfonate. 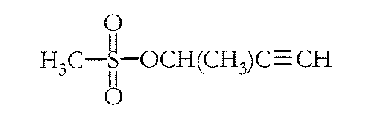 ¹H-NMR (CDCl₃, TMS) δ (ppm): 5.29 (1H, dq, J = 6.8 Hz, 2.2 Hz), 3.12 (3H, s), 2.71 (1H, d, J = 1.9 Hz), 1.66 (3H, d, J = 6.6 Hz)

### Reference Example 50

1.12 g of ethyl 3-(4-hydroxy-3-methoxyphenyl)propionate, 0.89 g of (1-methyl-2-propynyl) methanesulfonate, 1.03 g of potassium carbonate and 25 ml of acetonitrile were mixed and stirred at 80°C for 4 hours. After the reaction mixture was allowed to cool to around room temperature, water was added and the mixture was extracted with ethyl acetate. The organic layer was washed successively with 5% hydrochloric acid, water and an aqueous saturated sodium chloride solution, dried over magnesium sulfate, and then concentrated under reduced pressure. The residue was subjected to silica gel column purification to obtain 0.53 g of ethyl 3-{3-methoxy-4-(1-methyl-2-propynyloxy)phenyl}propionate. 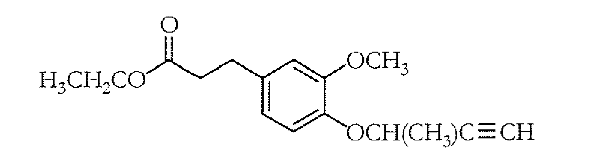 ¹H-NMR (CDCl₃, TMS) δ (ppm): 7.00 (1H, d, J = 8.0 Hz), 6.70-6.76 (2H, m), 4.82-4.90 (1H, m) 4.12 (2H, q, J = 7.2 Hz), 3.84 (3H, s), 2.90 (2H, t, J = 7.8 Hz), 2.60 (2H, t, J = 7.8 Hz), 2.44 (1H, d, J = 2.4 Hz), 1.69 (3H, d, J = 6.4 Hz), 1.25 (3H, t, J = 7.2 Hz)

### Reference Example 51

0.37 g of ethyl 3-{3-methoxy-4-(1-methyl-2-propynyloxy)phenyl}propionate and 0.3 ml of a 25% aqueous sodium hydroxide solution were mixed. Then, ethanol was added to the mixture until an almost homogeneous solution was obtained, and the mixture was stirred at 78°C for 2 hours. The reaction mixture was allowed to cool to around room temperature and then concentrated under reduced pressure. To the residue was added 5% hydrochloric acid, and the mixture was extracted with chloroform thrice. The organic layer was dried over magnesium sulfate and then concentrated under reduced pressure. The residue was washed with hexane to obtain 0.3 g of 3-{3-mehoxy-4-(1-methyl-2-propynyloxy)phenyl}propionic acid. 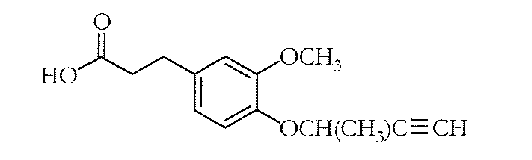 ¹H-NMR (CDCl₃ , TMS) δ (ppm): 7.01 (1H, d, J = 7.5 Hz), 6.71-6.78 (2H, m), 4.83-4.91 (1H, m), 3.84 (3H, s), 2.91 (2H, t, J = 7.7 Hz), 2.67 (2H, t, J = 7.7 Hz), 2.43-2.46 (1H, m), 1.68 (3H, d, J = 6.3 Hz)

### Reference Example 52

30 g of 4-chlorobenzaldehyde, 34 ml of trimethylsilyl cyanide and 1.4 g of zinc iodide were mixed and stirred at room temperature for 15 minutes. Thereto 30 ml of a 10% solution of ammonia in methanol was added, and the mixture was stirred at 40°C for 2 hours. The reaction mixture was allowed to cool to around room temperature and then concentrated under reduced pressure. To the resulting residue was added ethyl acetate. The mixture was dried over magnesium sulfate and then filtered. The filtrate was concentrated under reduced pressure. The resulting residue was dissolved in 300 ml of acetonitrile. Then, 25 ml of 36% hydrochloric acid was gradually added to the solution at 0°C. The mixture was filtered to obtain a solid. The solid was washed with acetonitrile and hexane, and then dried under reduced pressure to obtain 22 g of 2-amino-2-(4-chlorophenyl)acetonitrile hydrochloride. 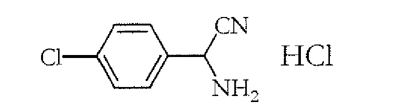 ¹H-NMR (CD₃SOCD₃, TMS) δ (ppm): 9.54 (3H, br.s), 7.68-7.72 (2H, m), 7.61-7.64 (2H, m), 5.98 (1H, s)

### Reference Example 53

In the same way as Reference Example 52, 1.5 g of 2-amino-2-(3-chlorophenyl)acetonitrile hydrochloride was obtained from 5.0 g of 3-chlorobenzaldehyde. 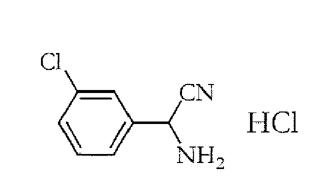 ¹H-NMR (CD₃SOCD₃, TMS) δ (ppm): 9.44 4 (3H, br.s), 7.76 (1H, d, J = 1.7 Hz), 7.55-7.66 (3H, m), 5.97 (1H, s)

### Reference Example 54

In the same way as Reference Example 52, 2.2 g of 2-amino-2-(4-methyl phenyl) acetonitrile hydrochloride was obtained from 5.0 g of 4-methylbenzaldehyde. 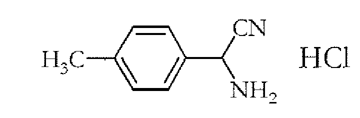 ¹H-NMR (CD₃SOCD₃, TMS) δ (ppm) : 9.51 (3H, br.s), 7. 54 (2 H, d, J = 8.2 Hz), 7.35 (2H, d, J = 8.2 Hz), 5.30 (1H, s), 2.35 (3H, s)

### Reference Example 55

In the same way as Reference Example 52, 1.5 g of 2-amino-2-(4-bromophenyl)acetonitrile hydrochloride was obtained from 5.0 g of 4-bromobenzaldehyde. 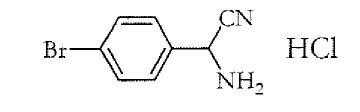 ¹H-NMR (CD₃SOCD₃, TMS) δ (ppm) : 9.49 (3H, br.s), 7.75-7.77 (2H, m), 7.61-7.64 (2H, m), 5.96 (1H, s)

### Reference Example 56

In the same way as Reference Example 52, 2.2 g of 2-amino-2-(3,4-dichlorophenyl)acetonitrile hydrochloride was obtained from 5.0 g of 3,4-dichlorobenzaldehyde. 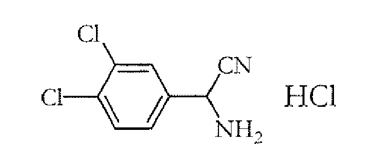 ¹H-NMR (CD₃SOCD₃, TMS) δ (ppm): 9.31 (3H, br.s), 7.95 (1H, d, J = 2.1 Hz), 7.85 (1H, d, J = 8.2 Hz), 7.66 (1H, dd, J = 8.2 Hz, 2.1 Hz), 5.96 (1H, s)

### Reference Example 57

5.2 g of ammonium chloride, 4.0 g of sodium cyanide and 100 ml of a 28% aqueous ammonia solution were mixed, and thereto 10 g of 4-chloroacetophenone was added gradually at 0°C. After the reaction mixture was stirred at room temperature for 24 hours, water was added and the mixture was extracted with chloroform. The organic layer was washed with water, dried over magnesium sulfate, and then concentrated under reduced pressure. The resulting residue was dissolved in 100 ml of acetonitrile, and then 36% hydrochloric acid was gradually added to the solution at 0°C. The mixed solution was concentrated under reduced pressure. The solid thus produced was washed with acetonitrile, tert-butyl methyl ether and hexane, and then dried under reduced pressure to obtain 5.0 g of 2-amino-2-(4-chlorophenyl)propionitrile hydrochloride. 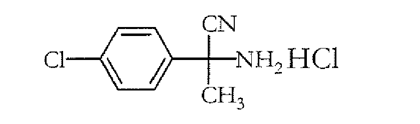 ¹H-NMR (CD₃SOCD₃, TMS) δ (ppm): 9.77 (3H, br.s), 7.75 (2H, d, J = 9.1 Hz), 7.64 (2H, d, J = 9.1 Hz), 2.05 (3H, s)

### Reference Example 58

22 g of ammonium chloride, 12 g of sodium cyanide and 300 ml of a 28% aqueous ammonia solution were mixed, and thereto 30 g of 4-chlorobenzaldehyde was gradually added at 0°C. The reaction mixture was stirred at 0°C for 1 hour and then at room temperature for 8 hours. Then water was added to the reaction mixture, followed by extraction with chloroform. The organic layer was washed with water, dried over magnesium sulfate, and then concentrated under reduced pressure. The resulting residue was dissolved in 300 ml of acetonitrile, and then 25 ml of 36% hydrochloric acid was added to the solution at 0°C. The solid thus produced was filtered, washed with acetonitrile, tert-butyl methyl ether and hexane, and then dried under reduced pressure to obtain 23 g of 2-amino-2-(4-chlorophenyl)acetonitrile hydrochloride. 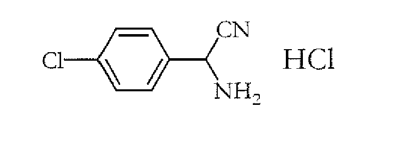 ¹H-NMR (CD₃ SOCD₃ , TMS) δ (ppm) : 9.54 (3H, br.s), 7.68-7.72 (2H, m), 7.61-7.64 (2H, m), 5.98 (1H, s)

### Reference Example 59

A mixture of 31 g of aluminum chloride and 150 ml of methylene chloride was cooled with an ice. Then 30 g of ethyl oxalyl chloride was mixed with the mixture. The mixture was stirred for 30 minutes under ice-cooling. The resulting mixture was gradually added to a mixture of 22 g of indane and 200 ml of methylene chloride under ice-cooling, followed by stirring at room temperature for 1 hour. The reaction mixture was gradually mixed into ice water, and the organic layer was separated. The organic layer was washed with water, dried over magnesium sulfate, and then concentrated under reduced pressure to obtain 37 g of ethyl indan-5-yl-oxoacetate as a crude product. 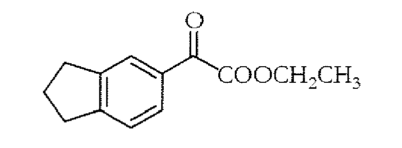 ¹H-NMR (CDCl₃, TMS) δ (ppm): 7.84 (1H, s), 7.78 (1H, d, J = 7.8 Hz), 7.34 (1H, d, J = 7.8 Hz), 4.44 (2H, q, J = 7.1 Hz), 2.95-2.99 (4H, m), 2.09-2.17 (2H, m), 1.42 (3H, t, J = 7.1 Hz)

### Reference Example 60

A mixture of 25 g of crude ethyl indan-5-yloxoacetate, 7.0 g of sodium borohydride and 250 ml of ethanol was stirred at room temperature for 1 hour and then at 60°C for 2 hours. Water was added to the reaction mixture. After the organic solvent was distilled off under reduced pressure, the residue was adjusted to pH 2 with 36% hydrochloric acid and extracted with chloroform. The organic layer was washed with water, dried over magnesium sulfate, and then distilled under reduced pressure to remove the organic solvent. The residue was washed with hexane to obtain 11 g of indan-5-ylethane-1,2-diol. 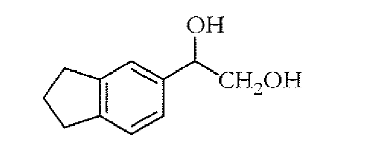 ¹H-NMR (CDCl₃, TMS) δ (ppm): 7.22 (1H, s), 7.20 (1H, d, J = 7.7 Hz), 7.11 (1H, d, J = 7.7 Hz), 4.78 (1H, dd, J = 8.2 Hz, 3.6 Hz), 3.62-3.75 (2H, m), 2.87-2.91 (4H, m), 2.5 (1H, br.s), 2.3 (1H, br.s), 2.03-2.10 (2H, m)

### Reference Example 61

A mixture of 11 g of crude indan-5-ylethane-1,2-diol, 18 g of periodic acid, 100 ml of water and 100 ml of ethanol was stirred at room temperature for 12 hours. Water was added to the reaction mixture. The mixture was extracted with ethyl acetate, washed with water twice, and then distilled under reduced pressure to remove the organic solvent. The residue was purified by a silica gel column to obtain 8.1 g of indane-5-carbaldehyde. 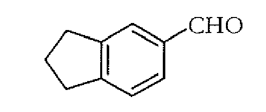 ¹H-NMR (CDCl₃, TMS) δ (ppm): 9.95 (1H, s), 7.73 (1H, s), 7.65 (1H, dd, J = 7.7 Hz, 1.2 Hz), 7.36 (1H, d, J = 7.7 Hz), 2.97 (4H, t, J = 7.5 Hz), 2.08-2.17 (2H, m)

### Reference Example 62

In the same way as Reference Example 59, 55 g of ethyl 5,6,7,8-tetrahydronaphthalen-2-yloxoacetate as a crude product was obtained from 58 g of tetralin. 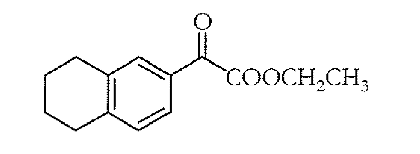 ¹H-NMR (CDCl₃ TMS) δ (ppm): 7.69-7.72 (2H, m), 7.17 (1H, d, J = 7.8 Hz), 4.44 (2H, q, J = 7.2 Hz), 2.75-2.83 (4H, m), 1.17-1.85 (4H, m), 1.42 (3H, t, J = 7.2 Hz)

### Reference Example 63

In the same way as Reference Example 60, 17 g of 5,6,7,8-tetrahydronaphthalen-2-ylethane-1,2-diol was obtained from 30 g of crude ethyl 5,6,7,8-tetrahydronaphthalen-2-yloxoacetate. 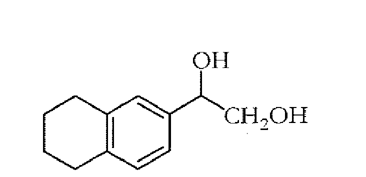 ¹H-NMR (CDCl₃, TMS) δ (ppm): 7.01-7.04 (3H, m), 4.76 (1H, dd, J = 8.1 Hz, 3.7), 3.63-3.77 (2H, m), 2.75-2.76 (4H, m), 2.4 (1H, br.s), 2.0 (1H, br.s), 1.17-1.18 (4H, m)

### Reference Example 64

In the same way as Reference Example 61, 13 g of 5,6,7,8-tetrahydronaphthalene-2-carbaldehyde was obtained from 16 g of 5,6,7,8-tetrahydronaphthalen-2-ylethane-1,2-diol. 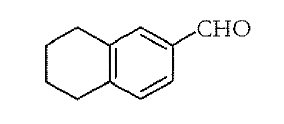 ¹H-NMR (CDCl₃, TMS) δ (ppm): 9.92 (1H, s), 7.57-7.59 (2H, m), 7.20 (1H, d, J = 7.5 Hz), 2.82-2.85 (4H, m), 1.81-1.84 (4H, m)

### Reference Example 65

2.2 g of ethyl 2-fluoro-2-diethylphosphonoacetate and 50 ml of tetrahydrofuran were mixed. Under ice-cooling, 040 g of sodium hydride (content 55%) was added and stirred for 10 minutes. Then, a mixture of 2.0 g of 4-benzyloxy-3-methoxybenzaldehyde and 5 ml of tetrahydrofuran was gradually added under ice-cooling, and the mixture was stirred at room temperature for 3 hours. Water was added to the resulting mixture. After the organic solvent was distilled off under reduced pressure, the mixture was extracted with ethyl acetate. The organic layer was washed successively with water, 5% hydrochloric acid, an aqueous saturated sodium hydrogen carbonate solution and an aqueous saturated sodium chloride solution, dried over magnesium sulfate, and then concentrated under reduced pressure. The resulting residue was purified by a silica gel column to obtain 2.8 g of ethyl 3-(3-methoxy-4-benzyloxyphenyl)-2-fluoroacrylate (a mixture of cis form and trans form) as a crude product. 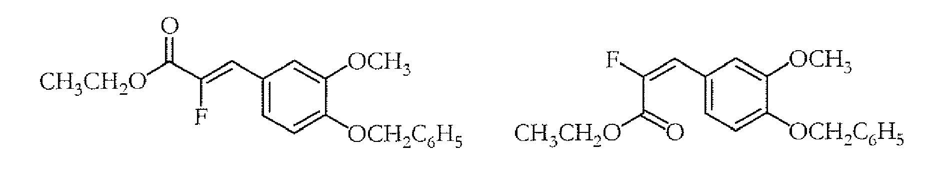 ¹H-NMR (CDCl₃, TMS) δ (ppm): 7.27-7.44 (6H, m), 7.13 (0.3H, dd, J = 8.5 Hz, 1.7 Hz), 7.01 (0.6H, dd, J = 8.3 Hz, 1.9 Hz), 6.79-6.90 (2H, m), 5.20 (0.6H, s), 5.18 (1.4H, s), 4.25-4.36 (2H, m), 3.91 (3H, s), 1.27-1.36 (3H, m)

### Reference Example 66

Using 2.6 g of crude ethyl 3-(3-methoxy-4-benzyloxyphenyl)-2-fluoroacrylate, 2.0 g of ethyl 3-(3-methoxy-4-hydroxyphenyl)-2-fluoropropionate was obtained as a crude product in the same way as Reference Example 11. 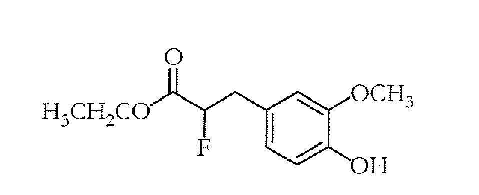 ¹H-NMR (CDCl₃, TMS) δ(ppm): 6.85 (1H, d, J = 8.0 Hz), 6.70-6.75 (2H, m), 5.52 (1H, br.s), 5.04 (1H, ddd, J = 49 Hz, 7.6 Hz, 4.2 Hz), 4.22 (2H, q, J = 7.1 Hz), 3.88 (3H, s), 3.06-3.18 (2H, m), 1.27 (3H, t, J = 7.1 Hz)

### Reference Example 67

Using 2.0 g of crude ethyl 3-(3-methoxy-4-hydroxyphenyl)-2-fluoropropionate and 0.86 ml of 3-bromopropyne, 2.3 g of ethyl 3-{3-methoxy-4-(2-propynyloxy)phenyl}-2-fluoropropionate was obtained as a crude product in the same way as Reference Example 12. 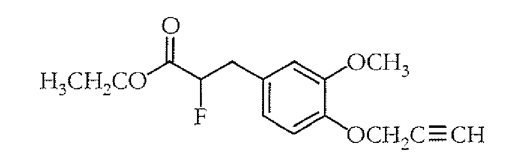 ¹H-NMR (CDCl₃, TMS) δ (ppm): 6.97 (1H, d, J = 8.9 Hz), 6.72-6.79 (2H, m), 5.06 (1H, ddd, J = 49 Hz, 7.5 Hz, 4.1 Hz), 4.74 (1H, d, J = 2.4 Hz), 4.23 (2H, q, J = 7.0 Hz), 3.86 (3H, s), 3.08-3.20 (2H, m), 2.49-2.50 (1H, m), 1.27 (3H, t, J = 7.0 Hz)

### Reference Example 68

Using 2.3 g of crude ethyl 3-{3-methoxy-4-(2-propynyloxy)phenyl}-2-fluoropropionate, 1.8 g of 3-{3-methoxy-4-(2-propynyloxy)phenyl}-2-fluoropropionic acid was obtained in the same way as Reference Example 13. 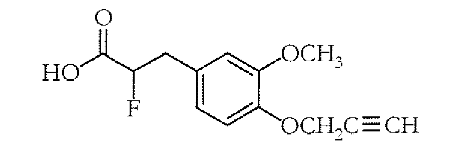 ¹H-NMR (CDCl₃, TMS) δ (ppm): 6.98 (1H, d, J = 8.7 Hz), 6.74-6.82 (2H, m), 5.21 (1H, ddd, J = 49 Hz, 7.5 Hz, 3.6 Hz), 4.75 (1H, d, J = 2.4 Hz), 3.86 (3H, s), 3.08-3.31 (2H, m), 2.49 (1H, t, J = 2.4 Hz)

### Reference Example 69

In the same way as Reference Example 4, 5.0 g of 3-{3-methoxy-4-(2-propynyloxy)phenyl}-2-fluoroproplonyl chloride was obtained as a crude product from about 4.7 g of 3-{3-methoxy-4-(2-propynyloxy)phenyl}-2-fluoropropionic acid. 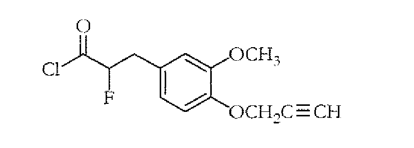 ¹H-NMR (CDCl₃ , TMS) δ (ppm): 6. 99 (1H, d, J = 8.2 Hz), 6.79-6.82 (2H, m), 5.20 (1H, ddd, J = 49 Hz, 7.3 Hz, 3.9 Hz), 4.75 (1H, d, J = 2.4 Hz), 3.87 (3H, s), 3. 17-3. 37 (2H, m), 2.50 (1H, t, J = 2.4 Hz)

### Reference Example 70

Into a mixture of 1.7 g of 4-bromobenzaldehyde, 1.0 g of cyclopropylboronic acid, 0.26 g of triphenylphosphine, 7.9 g of tripotassium phosphate n-hydrate, 20 ml of toluene and 1 ml of water; 0.10 g of palladium acetate was added, and the mixture was stirred at 100°C for 3 hours. After the reaction mixture was allowed to cool to around room temperature, water was added thereto and insoluble substances were filtered off. The filtrate was extracted with ethyl acetate. The organic layer was washed with an aqueous saturated sodium chloride solution, dried over magnesium sulfate, concentrated under reduced pressure, and subjected to silica gel column purification to obtain 1.1 g of 4-cyclopropylbenzaldehyde. 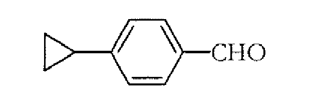 ¹H-NMR (CDCl₃, TMS) δ (ppm): 9.94 (1H, s), 7.76 (2H, d, J = 8.3 Hz), 7.18 (2H, d, J = 7.9 Hz), 1.93-2.00 (1H, m), 1.02-1.15 (2H, m), 0.75-0.86 (2H, m)

### Reference Example 71

1.1 g of 3-fluoro-4-hydroxy-5-methoxybenzaldehyde, 1.2 g of benzyl bromide, 1.3 g of potassium carbonate and 15 ml of acetonitrile were mixed and stirred under reflux for 4 hours. After the reaction mixture was cooled to room temperature, ethyl acetate was added and solids were filtered off. The resulting organic layer was concentrated under reduced pressure, and the residue was washed with hexane to obtain 1.8 g of 4-benzyloxy-3-fluoro-5-methoxybenzaldehyde. Then, 1.5 g of ethyl diethylphosphonoacetate and 20 ml of tetrahydrofuran were mixed, and thereto 0.29 g of sodium hydride (content 55%) was added under ice-cooling and mixed for 10 minutes. To the mixture was added gradually a mixture of 1.8 g of 4-benzyloxy-3-fluoro-5-methoxybenzaldehyde and 5 ml of tetrahydrofuran under ice-cooling, and the mixture was stirred at room temperature for 1 hour. After water was added to the resulting mixture, and the mixture was distilled under reduced pressure to remove the organic solvent and then extracted with ethyl acetate. The oily layer was separated, washed successively with 5% hydrochloric acid, an aqueous saturated sodium hydrogen carbonate solution and an aqueous saturated sodium chloride solution, dried over magnesium sulfate, and then concentrated under reduced pressure. The resulting residue was washed with hexane to obtain 3.6 g of ethyl 3-(4-benzyloxy-3-fluoro-5-methoxyphenyl)acrylate. 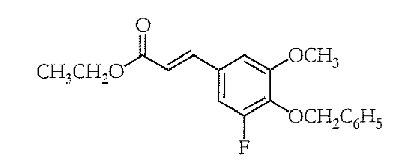 ¹H-NMR (CDCl₃, TMS) δ (ppm) : 7.54 (1H, d, J = 16 Hz), 7.44-7.46 (2H, m), 7.29-7.40 (3H, m), 6. 89 (1H, dd, J = 11 Hz, 1.9 Hz), 6.83 (1H, br.s), 6.32 (1H, d, J = 16 Hz), 5.14 (2H, s), 4.26 (2H, q, J = 7.2 Hz), 3.88 (3H, s), 1.33 (3H, t, J = 7.2 Hz)

### Reference Example 72

3.6 g of ethyl 3-(4-benzyloxy-3-fluoro-5-methoxyphenyl)acrylate, 0.1 g of 5% palladium carbon, about 0.01 g of 36% hydrochloric acid and 50 ml of ethanol were stirred under a hydrogen atmosphere. After absorption of a hydrogen gas was stopped, the mixture was filtered. The filtrated was concentrated under reduced pressure to obtain 2.2 g of ethyl 3-(3-fluoro-4-hydroxy-5-methoxyphenyl)propanoate. 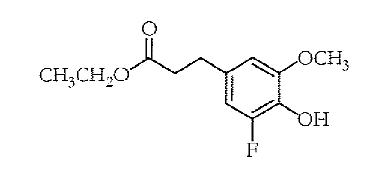 ¹H-NMR (CDCl₃, TMS) δ (ppm): 6.58 (1H, dd, J = 11 Hz, 2.0 Hz), 6.51-6.52 (1H, m), 4.13 (2H, q, J = 7.1 Hz), 3.89 (3H, s), 2.86 (2H, t, J = 7.7 Hz), 2.58 (2H, t, J = 7.7 Hz), 1.24 (3H, t, J = 7.1 Hz)

### Reference Example 73

2.3 g of ethyl 3-(3-fluoro-4-hydroxy-5-methoxyphenyl)propanoate, 0.63 ml of propargyl bromide, 1.23 g of potassium carbonate and 300 ml of acetonitrile were mixed and stirred under reflux for 1 hour. After the reaction mixture was cooled to room temperature, ethyl acetate was added and the mixture was filtered. The filtrate was concentrated under reduced pressure. The resulting residue, 0.54 g of lithium hydroxide, 40 ml of tetrahydrofuran and 20 ml of water were mixed and stirred under reflux for 3 hours. After water was added to the reaction mixture, the mixture was concentrated under reduced pressure. The mixture was washed with tert-butyl methyl ether. The aqueous layer was separated and thereto 5% hydrochloric acid was added. The mixture was extracted with chloroform thrice. The organic layer was dried over magnesium sulfate and then concentrated under reduced pressure. The residue was washed with hexane to obtain 1.5 g of 3-{3-fluoro-5-methoxy-4-(2-propynyloxy)phenyl}propanoic acid. 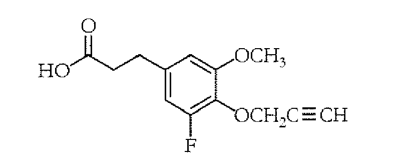 ¹H-NMR (CDCl₃, TMS) δ (ppm) : 6.59 (1H, dd, J = 11 Hz, 2.2 Hz), 6.55-6.56 (1H, m), 4.73 (2H, d, J = 2.4 Hz), 3.56 (3H, s), 2.90 (2H, t, J = 7.7 Hz), 2.67 (2H, t, J = 7.7 Hz), 2.27 (1H, t, J = 7.1 Hz)

### Reference Example 74

20 g of 3-(4-hydroxy-3-methoxyphenyl)propionic acid, 55 g of benzyl bromide, 45 g of potassium carbonate and 300 ml of acetonitrile were mixed and stirred at 80°C for 6 hours. After the reaction mixture was allowed to cool to around room temperature, ethyl acetate was added and the mixture was filtered. The filtrate was concentrated under reduced pressure. The resulting residue was mixed with 46 g of a 20% aqueous sodium hydroxide solution. Then, ethanol was added to the mixture until a homogeneous solution was obtained, and the mixture was stirred under reflux for 1 hour. After the reaction mixture was allowed to cool to around room temperature, water was added and the mixture was concentrated under reduced pressure, and washed with tert-butyl methyl ether. To the residue was added 5% hydrochloric acid to adjust it to pH 2, followed by extraction with ethyl acetate. The organic layer was washed with water and an aqueous saturated sodium chloride solution, dried over magnesium sulfate, and then concentrated under reduced pressure. The residue was washed with hexane and then dried to obtain 62 g of 3-(3-methoxy-4-benzyloxyphenyl)propionic acid. 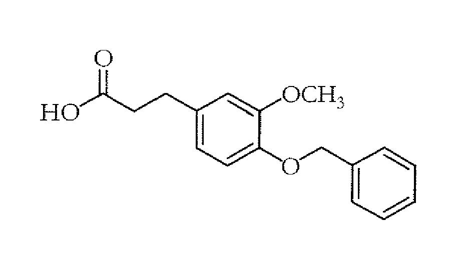 ¹H -NMR (CDCl₃, TMS) δ (ppm): 7.42-7.44 (1H, m), 7.26-7.37 (4H, m), 6.80 (1H, d, J = 8.4 Hz) , 6.75 (1H, d, J = 2.0 Hz), 6.67 (1H, dd, J = 8.4 Hz, 2.0 Hz), 5.12 (2H, s), 3.87 (3H, s), 2.89 (2H, t, J = 7.2 Hz), 2. 55 (2H, t, J = 7.2 Hz)

### Reference Example 75

100 ml of methanol and 10 ml of acetyl chloride were mixed at room temperature for 15 minutes. To the mixture, 4.0 g of 3-(3-methoxy-4-benzyloxyphenyl)propionic acid was added and stirred under reflux for 6 hours. The reaction mixture was allowed to cool to around room temperature and then concentrated under reduced pressure. To the residue, tert-butyl methyl ether was added, and the mixture was washed with water, an aqueous saturated sodium hydrogen carbonate solution and an aqueous saturated sodium chloride solution, dried over magnesium sulfate, and then concentrated under reduced pressure. 100 ml of monochlorobenzene and 3.0 g of the resulting residue were mixed, and thereto 1.7 ml of sulfuryl chloride was gradually added. The mixture was stirred at room temperature for 1 hour. The reaction mixture was washed with water, an aqueous saturated sodium hydrogen carbonate solution and an aqueous saturated sodium chloride solution, dried over magnesium sulfate, and then concentrated under reduced pressure. The residue was washed with hexane and dried to obtain 2.3 g of methyl 3-(6-chloro-3-methoxy-4-benzyloxyphenyl)propionate. 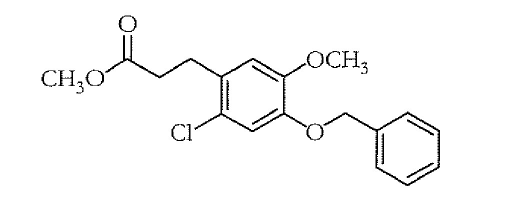 ¹H-NMR (CDCl₃, TMS) δ (ppm): 7.29-7.43 (5H, m), 6.88 (1H, s), 6.77 (1H, s), 5.09 (2H, s), 3.85 (3H, s), 3.68 (3H, s), 2.98 (2H, t, J = 7.6 Hz), 2.61 (2H, t, J = 7.6 Hz)

### Reference Example 76

2.2 g of methyl 3-(6-chloro-3-methoxy-4-benzyloxyphenyl)propionate, 1.4 g of a 47% aqueous hydrobromic acid solution and 30 ml of acetic acid were mixed and stirred at 80°C for 3 hours. After the reaction mixture was allowed to cool to around room temperature, toluene was added and the solvent was distilled off under reduced pressure. The residue was washed with hexane to obtain 1.4 g of 3-(6-chloro-4-hydroxy-3-methoxyphenyl)propionic acid. 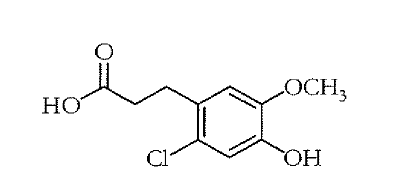 ¹H-NMR (CDCl₃, TMS) δ (ppm): 6.93 (1H, s), 6.74 (1H, s), 3.86 (3H, s), 2.98 (2H, t, J = 7.9 Hz), 2.68 (2H, t, J = 7.9 Hz)

### Reference Example 77

1.3 g of 3-(6-chloro-4-hydroxy-3-methoxyphenyl)propionic acid, 1.6 ml of propargyl bromide, 4.5 g of potassium carbonate and 30 ml of acetonitrile were mixed and stirred under reflux for hour. After the reaction mixture was allowed to cool to around room temperature, ethyl acetate was added and the mixture was filtered. The resulting filtrate was concentrated under reduced pressure. To the resulting residue, 0.55 g of lithium hydroxide monohydrate, 40 ml of tetrahydrofuran and 20 ml of water were added and stirred under reflux for 3 hours. After the reaction mixture was allowed to cool to around room temperature, water was added thereto. The mixture was concentrated under reduced pressure and then washed with tert-butyl methyl ether. To the resulting aqueous layer was added 5% hydrochloric acid, and the mixture was extracted with chloroform twice. The organic layer was washed with water, dried over magnesium sulfate, and then concentrated under reduced pressure. The residue was washed with hexane to obtain 1.2 g of 3-{6-chloro-3-methoxy-4-(2-propynyloxy)phenyl}propionic acid. 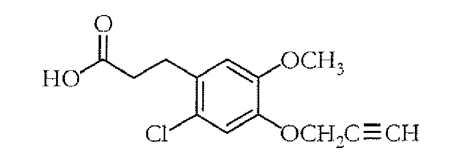 ¹H-NMR (CDCl₃, TMS) δ (ppm): 7.03 (1H, s), 6.78 (1H, s), 4.73 (2H, d, J = 2.4 Hz), 3.84 (3H, s), 3.00 (2H, t, J = 7.9 Hz), 2.69 (2H, t, J = 7.9 Hz), 2.54 (1H, t, J = 2.4 Hz)

### Reference Example 79

According to the method descried in Synlett, 2000, No.12, p.1801-1803, 10 g of 4-bromobenzonitrile, trisdibenzylideneacetone palladium(0), 0.98 g of 2-(di-tert-butylphosphino)biphenyl and 200 ml of dimethylimidazolinone were mixed at room temperature for 5 minutes hours. Then, 16.9 ml of hexamethyldisilane and 2.0 g of water were added thereto, and the mixture was washed with water five times, dried over magnesium sulfate, and then concentrated under reduced pressure. The residue was purified by a silica gel column to obtain 8.0 g of 4-trimethylsilylbenzonitrile. 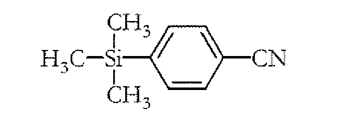 ¹H-NMR (CDCl3, TMS) δ (ppm): 7.60 (4H, s), 0.29 (9H, s) Reference Example 80

To a mixture of 1.5 g of 4-trimethylsilylbenzonitrile and 30 ml of toluene, 8.6 ml of a 1M solution of diisobutylaluminum hydride in toluene was gradually added and mixed at 45°C for 2 hours. The reaction mixture was allowed to cool to room temperature and then gradually added to an aqueous saturated ammonium chloride solution. The mixture was stirred at room temperature for 1 hour and then filtered with Celite to remove insoluble substances. The resulting organic layer was washed successively with 5% hydrochloric acid, an aqueous saturated sodium hydrogen carbonate solution and an aqueous saturated sodium chloride solution, dried over magnesium sulfate, and then concentrated under reduced pressure. The resulting residue was washed with hexane to obtain 1.2 g of 4-trimethylsilylbenzaldehyde. 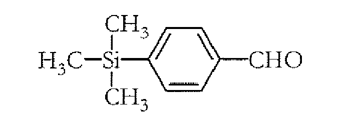 ¹H-NMR (CDCl₃, TMS) δ (ppm): 10.0 (1H, s), 7.84 (2H, d, J = 8.0 Hz), 7.69 (2H, d, J = 7.9 Hz), 0.31 (9H, s)

Next, Formulation Examples will be shown. Part represents part by weight.

### Formulation Example 1

Fifty parts of each of the compounds of the compounds 1 to 46 of the present invention, 3 parts of calcium ligninsulfonate, 2 parts of magnesium laurylsulfate and 45 parts of synthetic hydrated silica are pulverized and mixed well to give a wettable powder of each compound.

### Formulation Example 2

Twenty parts of each of the compounds 1 to 46 of the present invention and 1.5 parts of sorbitan trioleate are mixed with 28.5 parts of an aqueous solution containing 2 parts of polyvinyl alcohol, and wet-pulverized finely. To the obtained mixture, 40 parts of an aqueous solution containing 0.05 part of xanthan gum and 0.1 part of aluminium magnesium silicate is added and further 10 parts of propylene glycol is added to give a flowable of each compound.

### Formulation Example 3

Two parts of each of the compounds 1 to 46 of the present invention, 88 parts of kaolin clay and 10 parts of talc are pulverized and mixed well to give a dust of each compound.

### Formulation Example 4

Five parts of each of the compounds 1 to 46 of the present invention, 14 parts of polyoxyethylenestyryl phenyl ether, 6 parts of calcium dodecylbenzenesulfonate and 75 parts of xylene are mixed well to give an emulsifiable concentrate of each compound.

### Formulation Example 5

Two parts of each of the compounds 1 to 46 of the present invention, 1 part of synthetic hydrated silica, 2 parts of calcium ligninsulfonate, 30 parts of bentonite and 65 parts of kaolin clay are pulverized and mixed well, and water is added thereto and kneeded, granulated and dried to give a granule of each compound.

### Formulation Example 6

Ten parts of each of the compounds 1 to 46 of the present invention, 35 parts of white carbon containing 50% by weight of ammonium polyoxyethylenealkyl ether sulfate and 55 parts of water are mixed and wet pulverized finely to give a formulation of each compound.

Next, it is shown that the compounds of the present invention are useful for controlling plant diseases in Test Examples.

Additionally, the control effect was evaluated by visually observing the area of a lesion on a sample plant in investigation and comparing the area of a lesion on a non-treatment plant and the area of a lesion on a treated plant with the compound of the present invention.

In addition, the compound A described in Journal of Chemical and Engineering Data, 10(2), pp. 188(1965) 1965, vol.10, No.2, pp.188 was subjected to a test.

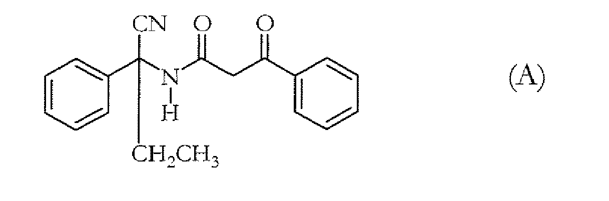

### Test Example 1

Sand loam was compacted in a plastic pot, and a tomato (variety: Ponterosa) was seeded and grown in a green house for 20 days. Each of the compounds 1, 3 to 6, 8 to 23, 26 to 28, 30 to 34, 36, 38, 39, 41 to 50, 52 to 57, 59 and 61 of the present invention was formulated according to Formulation Example 6, and then diluted with water to the prescribed concentration (500 ppm). The diluted solution was sprayed onto the stems and leaves of the tomato so as to adhere to the surface of the leaves sufficiently. After spraying, the stems and leaves were air-dried to such an extent that the diluted solution on the leaf surface was dried, and a suspension of zoosporangia of Phytophthora infestans (about 10,000 zoosporangia were contained in 1 ml of the suspension) was inoculated by spraying (the amount of the sprayed suspension was about 2 ml for one plant). After inoculation, the plant was grown for one day at 23°C under 90% or more humidity, and further grown 24°C in the daytime and 20°C in the nighttime for 4 days in a green house. Then, the controlling effect was examined.

As a result, the lesion areas on plants treated with the compounds of the present invention were not more than 10% of the lesion area on a non-treatment plant. The lesion area on a plant treated with the compound (A) was 76 to 100% of the lesion area on a non-treatment plant.

### Test Example 2

Sand loam was compacted in a plastic pot, and a grape (variety: Berry A) was seeded and grown in a green house for 40 days. Each of the compounds 1 to 5, 8 to 10, 12 to 15, 17, 19, 20, 22, 23, 25, 27 to 34, 37, 38, 39, 41 to 46, 50 to 54, 59, 61 to 64 and 65 of the present invention was formulated according to Formulation Example 6, and then diluted with water to the prescribed concentration (200 ppm). The diluted solution was sprayed onto the stems and leaves of the grape so as to adhere to the surface of the leaves. After spraying, the stems and leaves were air-dried to such an extent that the diluted solution on the leaf surface was dried, and a suspension of zoosporangia of Plasmopara viticola (about 10,000 zoosporangia were contained in 1 ml of the suspension) was inoculated by spraying (the amount of the sprayed suspension was about 2 ml for one plant). After inoculation, the plant was grown for one day at 23°C under 90% or more humidity, and further grown 24_°C in the daytime and 20°C in the nighttime for 6 days in a green house. Then, the controlling effect was examined.

As a result, the lesion areas on plants treated with the compounds of the present invention were not more than 10% of the lesion area on a non-treatment plant.

### Industrial Applicability

Since the compounds of the present invention have excellent plant disease-controlling activity, they are useful as an active ingredient of a plant disease-controlling composition.

## Claims

1. An N-(α-cyanobenzyl)amide compound represented by the formula (1): 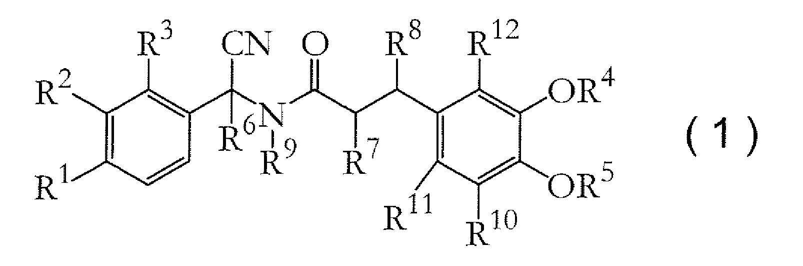 wherein R¹ represents a hydrogen atom; a halogen atom; a nitro group; a cyano group; a C1-C6 alkyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C2-C6 alkenyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C2-C6 alkynyl group optionally substituted with a substituent selected from the group consisting of a halogen atom, a cyano group, a hydroxyl group, 1 a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C3-C6 cycloalkyl group optionally substituted with a substituent selected from the group consisting of a halogen atom, a cyano group, a hydroxyl group, a C3-C6 cycloalkyl group, a C1-C6 alkoxy group, a C3-C6 cycloalkoxy group, a C1-C6 alkylthio group, a C3-C6 cycloalkylthio group and a tri(C1-C3 alkyl)silyl group; a C1-C6 alkoxy group optionally substituted with a substituent selected from the group consisting of a halogen atom, a C1-C6 alkoxy group and a C1-C6 alkylthio group; a C3-C6 cycloalkoxy group optionally substituted with a substituent selected from the group consisting of a halogen atom, a C1-C6 alkoxy group and a C1-C6 alkylthio group; a C1-C6 alkylthio group optionally substituted with a substituent selected from the group consisting of a halogen atom, a C1-C6 alkoxy group and a C1-C6 alkylthio group; a C3-C6 cycloalkylthio group optionally substituted with a substituent selected from the group consisting of a halogen atom, C1-C6 alkoxy group and a C1-C6 alkylthio group; a di(C1-C6 alkyl)amino group optionally substituted with a substituent selected from the group consisting of a halogen atom, a C1-C6 alkoxy group and a C1-C6 alkylthio group; a phenyl group optionally substituted with a substituent selected from the group consisting of a halogen atom, a C1-C6 alkyl group, a C1-C6 haloalkyl group and a C1-C6 alkoxy group; a phenoxy group optionally substituted with a substituent selected from the group consisting of a halogen atom, a C1-C6 alkyl group, a C1-C6 haloalkyl group and a C1-C6 alkoxy group; a phenylthio group optionally substituted with a substituent selected from the group consisting of a halogen atom, a C1-C6 alkyl group, a C1-C6 haloalkyl group and a C1-C6 alkoxy group; a benzoyl group optionally substituted with a substituted selected from the group consisting of a halogen atom, a C1-C6 alkyl group, a C1-C6 haloalkyl group and a C1-C6 alkoxy group; a benzoyloxy group optionally substituted with a substituent selected from the group consisting of a halogen atom, a C1-C6 alkyl group, a C1-C6 haloalkyl group and a C1-C6 alkoxy group; a formyl group; a (C1-C6 alkyl)carbonyl group; a (C2-C6 alkenyl)carbonyl group; a (C2-C6 alkynyl)carbonyl group; a (C3-C6 cycloalkyl)carbonyl group; a (C1-C6 haloalkyl)carbonyl group; a (C1-C6 alkaxy)oarbonyl group; or a tri(C1-C6 alkyl)silyl group,
R² represents a hydrogen atom, a halogen atom, a C1-C6 alkyl group, C3-C6 cycloalkyl group, a C2-C6 alkenyl group, a C2-C6 alkynyl group, a C1-C6 haloalkyl group, a C1-C6 alkoxy group, a C1-C6 haloalkoxy group, a C1-C6 alkylthio group, a C1-C6 haloalkylthio group, a di(C1-C6 alkyl)amino group, a nitro group or a cyano group, or
R¹ and R² are taken together to represent a C3-C6 polymethylene group optionally substituted with a substituent selected from the group consisting of a halogen atom, a C1-C3 alkyl group and a C1-C3 haloalkyl group; or a 1,3-butadiene-1,4-diyl group optionally substituted with a substituent selected from the group consisting of a halogen atom, a C1-C3 alkyl group and a C1-C3 haloalkyl group,
R³ represents a hydrogen atom, a halogen atom or a C1-C3 alkyl group,
R⁴ represents a C1-C4 alkyl group, a C1-C4 haloalkyl group, a C3-C4 alkenyl group or a C3-C4 alkynyl group,
R⁵ represents a C1-C4 alkyl group, a C3-C4 alkenyl group, a C3-C6 alkynyl group, a C1-C4 haloalkyl group, a cyano C1-C6 alkyl group, or a (C1-C3 alkyl)carbonyl group,
R⁶ represents a hydrogen atom, a C1-C4 alkyl group, a C3-C4 alkenyl group, or a C3-C4 alkynyl group,
R⁷ represents a hydrogen atom, a halogen atom or a Cl-C3 alkyl group,
R⁸ represents a hydrogen atom, a halogen atom or a Cl-C3 alkyl group,
R⁹ represents a hydrogen atom or a Cl-C3 alkyl group,
R¹⁰ represents a hydrogen atom, a halogen atom or a C1-C3 alkyl group,
R¹¹ represents a hydrogen atom, a halogen atom or a C1-C3 alkyl group, and
R¹² represents a hydrogen atom, a halogen atom or a C1-C3 alkyl group.

2. The N-(α-cyanobenzyl)amide compound according to claim 1, wherein R¹ is a hydrogen atom, a halogen atom, a C1-C6 alkyl group, a C2-C6 alkenyl group, a C2-C6 alkynyl group, a C1-C6 haloalkyl group, a C1-C6 alkoxy group, a C1-C6 haloalkoxy group, a C1-C6 alkylthio group, a C1-C6 haloalkylthio group, a di(C1-C6 alkyl)amino group, a phenyl group, 1 a phenoxy group or a cyano group, R² is a hydrogen atom, a halogen atom, a C1-C6 alkyl group, a C1-C6 alkoxy group or a C1-C6 haloalkyl group, or R¹ and R² are taken together to form a C3-C5 alkylene group or a CH=CH-CH=CH group, R³ is a hydrogen atom, a halogen atom or a C1-C3 alkyl group, R⁴ is a C1-C4 alkyl group, a C3-C4 alkenyl group or a C3-C4 alkynyl group, R⁵ is a C1-C4 alkyl group, a C3-C4 alkenyl group, a C3-C6 alkynyl group, a C1-C4 haloalkyl group or a (C1-C3 alkyl)carbonyl group, 1 R⁶ is a hydrogen atom, a C1-C4 alkyl group, a C3-C4 alkenyl group, or a C3-C4 alkynyl group, R⁷ is a hydrogen atom, a halogen atom or a C1-C3 alkyl group, R⁸ is a hydrogen atom, a halogen atom or a C1-C3 alkyl group, R⁹ is a hydrogen atom or a C1-C3 alkyl group, and R¹⁰, R¹¹ and R¹² are hydrogen atoms.

3. The N-(α-cyanobenzyl)amide compound according to claim 1, wherein R¹ is a hydrogen atom, a halogen atom, a C1-C4 alkyl group, a C1-C4 alkoxy group or a C1-C4 haloalkyl group, R² is a hydrogen atom, a halogen atom, a C1-C4 alkyl group, a C1-C4 alkoxy group or a C1-C4 haloalkyl group, or R¹ and R² are taken together to form a C3-C5 alkylene group or a CH=CH-CH=CH group, R⁴ is a C1-C4 alkyl group, a C3-C4 alkenyl group or a C3-C4 alkynyl group, R⁵ is a C1-C4 alkyl group, a C3-C4 alkenyl group or a C3-C4 alkynyl group, R⁶ is a hydrogen atom or a C3-C4 alkyl group, and R ³ R⁷, R⁸, R⁹ R¹⁰, R¹¹ and R¹² are hydrogen atoms.

4. A plant disease-controlling composition comprising the N-(α-cyanobenzyl)amide compound according to any one of claims 1 to 3 and an inert carrier.

5. A method for controlling a plant disease, which comprises treating a plant or soil where a plant is cultivated with an effective amount of the N-(α-cyanobenzyl)amide compound according to any one of claims 1 to 3.

6. Use of the N-(α-cyanobenzyl)amide compound according to any one of claims 1 to 3 as an active ingredient of a plant disease-controlling composition.
